# EUROPEAN PATENT APPLICATION

(11) **EP 4 782 435 A1**
(43) Date of publication of application: **29.07.2026**
(21) Application number: 25154108.2
(22) Date of filing: 27.01.2025
(51) Int. Cl.: C07D 213/82, C07D 239/28, C07D 231/14, C07D 307/68, C07D 333/38, A01N 43/08, A01N 43/10, A01N 43/40, A01N 43/54, A01N 43/56

(54) **HERBICIDAL ALANINE ANALOGUES**

(71) Applicant: BASF SE, 67056 Ludwigshafen am Rhein (DE)
(72) Inventor: KORDES, Markus, 67056 Ludwigshafen am Rhein (DE); PAES LACERDA, Maira, 67117 Limburgerhof (DE); SEISER, Tobias, 67056 Ludwigshafen am Rhein (DE); HEINRICH, Marc, 67056 Ludwigshafen am Rhein (DE)
(74) Representative: BASF IP Association

(57) **Abstract**

The present invention relates to specific alanine analogues, and to the use of said alanine analogues for controlling unwanted vegetation. Furthermore, the invention relates to agrochemical formulations comprising these alanine analogues and to methods of applying the alanine analogues or their agrochemical formulations for controlling unwanted vegetation.

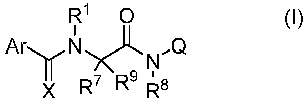

## Description

The present invention relates to specific alanine analogues of formula (I) and to the use of said alanine analogues for controlling unwanted vegetation. Furthermore, the invention relates to agrochemical formulations comprising these alanine analogues and to methods of applying the alanine analogues or their agrochemical formulations for controlling unwanted vegetation.

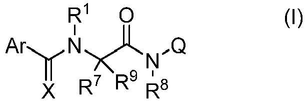

For controlling unwanted vegetation, especially in crop plants (also referred to as cultivated plants), there is an ongoing need for new herbicides that have high activity and selectivity together with a substantial lack of toxicity for humans and animals.

PCT/EP2024/070822 describes substituted alanine analogues and their use as herbicides.

The prior art compounds often suffer from insufficient herbicidal activity at low application rates and/or unsatisfactory selectivity, resulting in a low compatibility with crop plants. Accordingly, it is an object of the present invention to provide further herbicidally active compounds with strong herbicidal activity, even at low application rates, a sufficiently low toxicity for humans and animals and/or a high compatibility with crop plants. The compounds should also show a broad activity spectrum against many different unwanted plants. These and further objectives are achieved by the compounds of formula (I) defined below.

Accordingly, the present invention relates to compounds of formula (I) 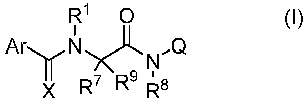 wherein the substituents have the following meanings:
- Ar: is a 5- or 6-membered heteroaromatic ring; or a bicyclic or tricyclic saturated, partially unsaturated, partially aromatic condensed, or aromatic ring system; wherein the 5- or 6-membered heteroaromatic ring contains 1, 2, 3, or 4 heteroatoms selected from the group consisting of N, O, and S as ring members; wherein the bicyclic or tricyclic ring system contains 0, 1, 2, 3, or 4 heteroatoms selected from the group consisting of N, O, and S as ring members; and with the provision that the 5- or 6-membered heteroaromatic ring cannot contain 2 or more atoms selected from O and S, and the bicyclic or tricyclic ring system cannot contain 2 contiguous atoms selected from O and S; and wherein the heteroaromatic ring, or the bicyclic, or tricyclic ring systems are substituted with k substituents R^{A1} and q substituents R^{A2};
- R^{A1}: independently of each other are halogen, nitro, hydroxyl, oxo, cyano, (C₁-C₃)-alkyl, (C₁-C₃)-haloalkyl, hydroxy-(C₁-C₃)-alkyl, (C₃-C₅)-cycloalkyl, (C₁-C₃)-alkoxy, (C₁-C₃)-haloalkoxy, (C₂-C₃)-alkenyl, (C₂-C₃)-haloalkenyl, (C₂-C₃)-alkynyl, or (C₂-C₃)-haloalkynyl; and whereas the sulfur ring atoms bear 0, 1, or 2 oxo groups;
- R^{A2}: independently of each other are phenyl-(C₁-C₃)-alkyl, (C₁-C₄)-alkylcarbonyl, aminocarbonyl, (C₁-C₄)-alkylaminocarbonyl, di-(C₁-C₄-alkyl)aminocarbonyl, (C₁-C₄)-alkoxycarbonyl, benzyloxycarbonyl, fluorenyloxycarbonyl, allyloxycarbonyl, (C₁-C₃)-alkoxy-(C₁-C₃)-alkyl, (C₁-C₃)-alkylthio, (C₁-C₃)-alkylsulfinyl, (C₁-C₃)-alkylsulfonyl, or phenylsulfonyl, where the aliphatic or aromatic moieties in the 14 last-mentioned radicals are unsubstituted or substituted by m radicals selected from the group consisting of fluorine, chlorine, bromine, cyano and (C₁-C₂)-alkoxy;
- k: is 0, 1, 2, 3, 4, or 5;
- q: is 0, 1, 2, 3, 4, or 5;
with the provision that the total number of substituents R^{A1} and R^{A2} on each ring or ring system does not exceed the number of substituents that are theoretically possible; and excluding heteroaromatic rings that bear an electronic charge;
- R¹, R⁸: independently of each other are hydrogen, (C₁-C₃)-alkyl, (C₁-C₃)-haloalkyl, (C₃-C₄)-cycloalkyl, (C₂-C₃)-alkenyl, (C₂-C₃)-haloalkenyl, (C₃-C₄)-alkynyl, (C₃-C₄)-haloalkynyl, (C₁-C₃)-alkoxy-(C₁-C₃)-alkyl, aryl, aryl-(C₁-C₃)-alkyl, (C₁-C₃)-alkoxy, (C₁-C₃)-haloalkoxy, (C₁-C₃)-alkoxy-(C₁-C₃)-alkoxy, (C₁-C₃)-alkylcarbonyl, arylcarbonyl, (C₁-C₃)-alkoxycarbonyl, or aryloxycarbonyl;
- R⁷: is methyl or ethyl;
- R⁹: is hydrogen or methyl; or
- R⁷ and R⁹: together with the carbon atom to which they are attached form a 3- or 4-membered, saturated carbocyclic ring;
- X: is oxygen or sulfur;
- Q: represents the radical of formula (Z-Y), 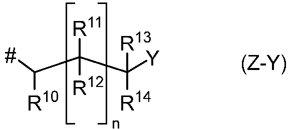
wherein # indicates the bond to the adjacent nitrogen atom, and wherein the substituents have the following meanings
- R¹⁰: is (C₁-C₄)-alkyl;
- R¹¹, R¹², R¹³, R¹⁴: independently of each other are hydrogen or (C₁-C₄)-alkyl; or
- R¹⁰ and R¹⁴: together with the carbon atoms to which they are bound form a three- to six-membered saturated or partially unsaturated carbocycle or a three- to six-membered saturated or partially unsaturated heterocycle containing 1 or 2 oxygen, nitrogen or sulfur atoms as ring members; with the provision that the heterocycle cannot contain 2 contiguous atoms selected from O and S;
- Y: is CO₂R^{e}, CONR^{b}R^{h}, CON(R^{x})-OR^{y}, C(=N-OR^{y})-OR^{x}, CONR^{e}S(=O)R^{a}, CONR^{e}SO₂R^{a}, or CONR^{b1}SO₂NR^{b2}R^{b3};
- R^{a}: is each independently (C₁-C₆)-alkyl, (C₁-C₆)-haloalkyl, (C₃-C₄)-alkynyl, or (C₃-C₆)-cycloalkyl, each of which is unsubstituted or substituted with m radicals selected from the group consisting of fluorine, chlorine, bromine, iodine, cyano, hydroxyl, and (C₁-C₃)-alkoxy;
- R^{b}: is hydrogen, (C₁-C₆)-alkyl, (C₂-C₄)-alkenyl, (C₃-C₄)-alkynyl, (C₃-C₆)-cycloalkyl, (C₃-C₆)-cycloalkyl-(C₁-C₃)-alkyl, phenyl-(C₁-C₃)-alkyl, or furanyl-(C₁-C₃)-alkyl, where each of the seven last-mentioned radicals is unsubstituted or substituted with m radicals selected from the group consisting of fluorine, chlorine, bromine, cyano, CO₂R^{a}, (C₁-C₂)-alkoxy, (C₁-C₃)-alkylthio, (C₁-C₃)-alkylsulfinyl, (C₁-C₃)-alkylsulfonyl, phenylthio, phenylsulfinyl, and phenylsulfonyl;
- R^{b1} and R^{b2}: independently of each other and independently of each occurrence, are hydrogen or have independently of each other one of the meanings given for R^{a};
- R^{b3}: is hydrogen, (C₁-C₆)-alkyl, (C₂-C₄)-alkenyl, (C₃-C₄)-alkynyl, (C₃-C₆)-cycloalkyl, (C₃-C₆)-cycloalkyl-(C₁-C₃)-alkyl, phenyl-(C₁-C₃)-alkyl, or furanyl-(C₁-C₃)-alkyl, where each of the seven last-mentioned radicals is unsubstituted or substituted with m radicals selected from the group consisting of fluorine, chlorine, bromine, cyano, CO₂R^{a}, , (C₁-C₂)-alkoxy, (C₁-C₃)-alkylthio, (C₁-C₃)-alkylsulfinyl, (C₁-C₃)-alkylsulfonyl, phenylthio, phenylsulfinyl, and phenylsulfonyl; or
- R^{b2} and R^{b3}: form, together with the nitrogen atom to which they are bound, a saturated 3-, 4-, 5-, 6-, or 7-membered heterocycle which may contain one further heteroatom or heteroatom group selected from the group consisting of N, O, S, S(=O) and S(=O)₂ as ring member;
- R^{e}: is hydrogen, (C₁-C₆)-alkyl, (C₂-C₄)-alkenyl, (C₃-C₄)-alkynyl, (C₃-C₆)-cycloalkyl, (C₃-C₆)-cycloalkyl-(C₁-C₃)-alkyl, phenyl-(C₁-C₃)-alkyl, or furanyl-(C₁-C₃)-alkyl, where each of the seven last-mentioned radicals is unsubstituted or substituted with m radicals selected from the group consisting of fluorine, chlorine, bromine, cyano, CO₂R^{a}, (C₁-C₂)-alkoxy, (C₁-C₂)-haloalkoxy, (C₁-C₃)-alkylthio, (C₁-C₃)-haloalkylthio, (C₁-C₃)-alkylsulfinyl, (C₁-C₃)-haloalkylsulfinyl, (C₁-C₃)-alkylsulfonyl, (C₁-C₃)-haloalkylsulfonyl, phenylthio, phenylsulfinyl, and phenylsulfonyl;
- R^{h}: is hydrogen, hydroxyl, (C₁-C₆)-alkyl, (C₁-C₂)-alkoxy, (C₃-C₆)-cycloalkyl, (C₂-C₄)-alkenyl, (C₁-C₆)-alkoxycarbonyl-(C₁-C₆)-alkyl, or (C₃-C₄)-alkynyl, where each of the six last-mentioned radicals is unsubstituted or substituted with m radicals selected from the group consisting of fluorine, chlorine, bromine, cyano, CO₂R^{a}, (C₁-C₂)-alkyl, (C₁-C₂)-alkoxy and (C₁-C₂)-alkoxy-(C₁-C₂)-alkoxy; or
- R^{b} and R^{h}: form, together with the nitrogen atom to which they are bound, a saturated 3-, 4-, 5-, 6-, or 7-membered heterocycle which may contain one further heteroatom or heteroatom group selected from the group consisting of N, O, S, S(=O), and S(=O)₂ as ring member;
- R^{x} and R^{y}: together with the atoms to which they are each bound, form a saturated 5- or 6-membered heterocycle;
- m: is each independently 1, 2, or 3;
- n: is 0, 1, or 2;
including their agriculturally acceptable salts, stereoisomers and tautomers.

The invention also relates to an agrochemical formulation comprising at least one compound of formula (I) and one or more auxiliaries customary for formulating crop protection compounds.

The invention relates moreover to the use of a compound of formula (I) for controlling unwanted vegetation, and to a method for controlling unwanted vegetation which comprises allowing a herbicidally effective amount of at least one compound of formula (I) to act on plants, their seed and/or their habitat.

Depending on the kind of substituents, the compounds of formula (I) may have one or more centers of chirality, in which case they may be present as mixtures of enantiomers or diastereomers but also in the form of the pure enantiomers or pure diastereomers. The invention provides both the pure enantiomers or pure diastereomers of the compounds of formula (I) and their mixtures and the use according to the invention of the pure enantiomers or pure diastereomers of the compound of formula (I) or their mixtures. Suitable compounds of formula (I) also include all possible geometrical stereoisomers (cis/trans isomers) as a specific form of diastereomers and mixtures thereof. Cis/trans isomers may be present with respect to an alkene, carbon-nitrogen double-bond, nitrogen-sulfur double bond, amide group or a cyclic, non-aromatic moiety. The term "stereoisomer(s)" encompasses both optical isomers, such as enantiomers or diastereomers existing due to more than one stereogenic center in the molecule, as well as geometrical isomers (cis/trans isomers). Just by way of example, a stereogenic center is the carbon atom carrying R⁷ and R⁹. The stereo configuration of a particular compound is explicitly specified using the rules of the Cahn-Ingold-Prelog convention.

If the compounds of formula (I) as described herein have ionizable functional groups, they can also be employed in the form of their agriculturally acceptable salts. Suitable are, in general, the salts of those cations and the acid addition salts of those acids whose cations and anions, respectively, have no adverse effect on the activity of the active compounds.

Preferred cations are the ions of the alkali metals, preferably of lithium, sodium and potassium, of the alkaline earth metals, preferably of calcium and magnesium, and of the transition metals, preferably of manganese, copper, zinc and iron, further ammonium and substituted ammonium in which one to four hydrogen atoms are replaced by C₁-C₄-alkyl, hydroxy-C₁-C₄-alkyl, C₁-C₄-alkoxy-C₁-C₄-alkyl, hydroxy-C₁-C₄-alkoxy-C₁-C₄-alkyl, phenyl or benzyl, preferably ammonium, methylammonium, isopropylammonium, dimethylammonium, diethylammonium, diisopropylammonium, trimethylammonium, triethylammonium, tris(isopropyl)ammonium, heptylammonium, dodecylammonium, tetradecylammonium, tetramethylammonium, tetraethylammonium, tetrabutylammonium, 2-hydroxyethylammonium (olamine salt), 2-(2-hydroxyeth-1-oxy)eth-1-ylammonium (diglycolamine salt), di(2-hydroxyeth-1-yl)ammonium (diolamine salt), tris(2-hydroxyethyl)ammonium (trolamine salt), tris(2-hydroxypropyl)ammonium, benzyltrimethylammonium, benzyltriethylammonium, N,N,N-trimethylethanolammonium (choline salt), furthermore phosphonium ions, sulfonium ions, preferably tri(C₁-C₄-alkyl)sulfonium, such as trimethylsulfonium, and sulfoxonium ions, preferably tri(C₁-C₄-alkyl)sulfoxonium, and finally the salts of polybasic amines such as N,N-bis-(3-aminopropyl)methylamine and diethylenetriamine.

Anions of useful acid addition salts are primarily chloride, bromide, fluoride, iodide, hydrogensulfate, methylsulfate, sulfate, dihydrogenphosphate, hydrogenphosphate, nitrate, bicarbonate, carbonate, hexafluorosilicate, hexafluorophosphate, benzoate and also the anions of C₁-C₄-alkanoic acids, preferably formate, acetate, propionate and butyrate.

If the compounds of formula (I) can form tautomers due to intramolecular proton transfer, these tautomers are also encompassed by the definition and comprised by the scope of general formula (I) according to the invention. Common examples of such interconversions i.a. are keto-enol- (H-O-C=C 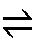 O=C-C-H) and amide-imidic acid-(H-N-C=O 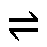 N=C-O-H) tautomerism. The amount in which the one or other tautomeric form is present depends on the complete molecular structure and even stronger on the surrounding conditions (presence or absence of solvent, type of solvent, pH, temperature etc.).

The term "undesired vegetation" ("weeds") is understood to include any vegetation growing in non-crop-areas or at a crop plant site or locus of seeded and otherwise desired crop, where the vegetation is any plant species, including their germinant seeds, emerging seedlings and established vegetation, other than the seeded or desired crop (if any). Weeds, in the broadest sense, are plants considered undesirable in a particular location.

The organic moieties mentioned in the above definitions of the variables are - like the term halogen - collective terms for individual listings of the individual group members. The prefix Cₙ-Cₘ indicates in each case the possible number of carbon atoms in the group.

The term "halogen" denotes in each case fluorine, bromine, chlorine or iodine, in particular fluorine, chlorine or bromine.

The term "partially or completely halogenated" means that 1 or more, e.g. 1, 2, 3, 4, or 5 or all of the hydrogen atoms of a given radical have been replaced by a halogen atom, in particular by fluorine or chlorine. A partially or completely halogenated radical is termed below also "halo-radical". For example, partially or completely halogenated alkyl is also termed haloalkyl.

The term "alkyl" as used herein (and in the alkyl moieties of other groups comprising an alkyl group, e.g. alkoxy, alkylamino, dialkylamino, alkylcarbonyl, alkoxycarbonyl, alkylthio, alkylsulfonyl and alkoxyalkyl) denotes in each case a straight-chain or branched alkyl group having usually from 1 to 12 carbon atoms (= C₁-C₁₂-alkyl), frequently from 1 to 6 carbon atoms (= C₁-C₆-alkyl), in particular 1 to 4 carbon atoms (= C₁-C₄-alkyl) and especially from 1 to 3 carbon atoms (= C₁-C₃-alkyl) or 1 or 2 carbon atoms (= C₁-C₂-alkyl). C₁-C₂-Alkyl is methyl or ethyl. C₁-C₃-Alkyl is methyl, ethyl, n-propyl or iso-propyl. Examples of C₁-C₄-alkyl are methyl, ethyl, n-propyl, iso-propyl, n-butyl, 2-butyl (= sec-butyl), isobutyl and tert-butyl. Examples for C₁-C₆-alkyl are, in addition to those mentioned for C₁-C₄-alkyl, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl. Examples for C₁-C₈-alkyl are, in addition to those mentioned for C₁-C₆-alkyl, n-heptyl, 1-methylhexyl, 2-methylhexyl, 3-methylhexyl, 4-methylhexyl, 5-methylhexyl, 1-ethylpentyl, 2-ethylpentyl, 3-ethylpentyl, n-octyl, 1-methylheptyl, 2-methylheptyl, 1-ethylhexyl, 2-ethylhexyl, 1,2-dimethylhexyl, 1-propylpentyl and 2-propylpentyl. Examples for C₁-C₁₂-alkyl are, apart those mentioned for C₁-C₈-alkyl, nonyl, decyl, 2-propylheptyl, 3-propylheptyl, undecyl, dodecyl and positional isomers thereof.

The term "haloalkyl" as used herein (and in the haloalkyl moieties of other groups comprising a haloalkyl group, e.g. haloalkoxy, haloalkylthio, haloalkylcarbonyl, haloalkylsulfonyl and haloalkylsulfinyl), which is also expressed as "alkyl which is partially or fully halogenated", denotes in each case a straight-chain or branched alkyl group having usually from 1 to 6 carbon atoms (= C₁-C₆-haloalkyl), more frequently 1 to 3 carbon atoms (= C₁-C₃-haloalkyl), as defined above, wherein the hydrogen atoms of this group are partially or totally replaced with halogen atoms. Preferred haloalkyl moieties are selected from C₁-C₃-haloalkyl, specifically from C₁-C₂-haloalkyl, in particular from fluorinated C₁-C₂-alkyl. Examples for C₁-C₂-haloalkyl are fluoromethyl, difluoromethyl, trifluoromethyl, chloromethyl, dichloromethyl, trichloromethyl, chlorofluoromethyl, dichlorofluoromethyl, chlorodifluoromethyl, bromomethyl,1-fluoroethyl, 2-fluoroethyl, 2,2-difluoroethyl, 2,2,2-trifluoroethyl, pentafluoroethyl, 1-chloroethyl, 2-chloroethyl, 2,2,-dichloroethyl, 2,2,2-trichloroethyl, 2-chloro-2-fluoroethyl, 2-chloro-2,2-difluoroethyl, 2,2-dichloro-2-fluoroethyl, 1-bromoethyl, and the like. Examples for C₁-C₃-haloalkyl are, in addition to those mentioned for C₁-C₂-haloalkyl, 1-fluoropropyl, 2-fluoropropyl, 3-fluoropropyl, 3,3-difluoropropyl, 3,3,3-trifluoropropyl, heptafluoropropyl, 1,1,1-trifluoroprop-2-yl, 3-chloropropyl, and the like.

The term "hydroxyalkyl" denotes in each case a straight-chain or branched alkyl group having usually from 1 to 6 carbon atoms (= C₁-C₆-hydroxyalkyl), more frequently 1 to 3 carbon atoms (= C₁-C₃-hydroxyalkyl), as defined above, wherein one hydrogen atom of this group is replaced with a hydroxyl group. Examples are hydroxymethyl, 1-hydroxyethyl, 2-hydroxyethyl, 1-hydroxypropyl, 2-hydroxypropyl, 3-hydroxypropyl, 1-hydroxy-2-propyl and the like. The term "alkenyl" as used herein denotes in each case a monounsaturated straight-chain or branched hydrocarbon radical having usually 2 to 12 (= C₂-C₁₂-alkenyl), preferably 2 to 6 carbon atoms (= C₂-C₆-alkenyl), e.g. 3 to 6 carbon atoms (= C₃-C₆-alkenyl), in particular 2 to 4 carbon atoms (= C₂-C₄-alkenyl) or 2 or 3 carbon atoms (= C₂-C₃-alkenyl), and a double bond in any position, for example C₂-C₃-alkenyl, such as ethenyl, 1-propenyl, 2-propenyl or 1-methylethenyl; C₂-C₄-alkenyl, such as ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl or 2-methyl-2-propenyl; C₂-C₆-alkenyl, such as ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl, 1-ethyl-2-methyl-2-propenyl and the like, or C₂-C₁₂-alkenyl, such as the radicals mentioned for C₂-C₆-alkenyl and additionally 1-heptenyl, 2-heptenyl, 3-heptenyl, 1-octenyl, 2-octenyl, 3-octenyl, 4-octenyl, the nonenyls, decenyls, undecenyls, dodecenyls and the positional isomers thereof.

Examples for C₃-C₆-alkenyl are those mentioned above for C₂-C₆-alkenyl, except for ethenyl.

The term "haloalkenyl" as used herein, which may also be expressed as "alkenyl which is substituted with halogen", and the haloalkenyl moieties in haloalkenyloxy and the like refers to unsaturated straight-chain or branched hydrocarbon radicals having 2 to 6 (= C₂-C₆-haloalkenyl) or 2 to 4 (= C₂-C₄-haloalkenyl) or 2 to 3 (= C₂-C₃-haloalkenyl) carbon atoms and a double bond in any position, where some or all of the hydrogen atoms in these groups are replaced by halogen atoms as mentioned above, in particular fluorine, chlorine and bromine, for example chlorovinyl, chloroallyl and the like.

The term "alkynyl" as used herein denotes unsaturated straight-chain or branched hydrocarbon radicals having usually 2 to 12 (= C₂-C₁₂-alkynyl), frequently 2 to 6 (= C₂-C₆-alkynyl), preferably 2 to 4 carbon atoms (= C₂-C₄-alkynyl) or 2 to 3 carbon atoms (= C₂-C₃-alkynyl) and a triple bond in any position, for example C₂-C₃-alkynyl, such as ethynyl, 1-propynyl or 2-propynyl; C₂-C₄-alkynyl, such as ethynyl, 1-propynyl or 2-propynyl, 1-butynyl, 2-butynyl, 3-butynyl, 1-methyl-2-propynyl and the like; C₂-C₆-alkynyl, such as ethynyl, 1-propynyl, 2-propynyl, 1-butynyl, 2-butynyl, 3-butynyl, 1-methyl-2-propynyl, 1-pentynyl, 2-pentynyl, 3-pentynyl, 4-pentynyl, 1-methyl-2-butynyl, 1-methyl-3-butynyl, 2-methyl-3-butynyl, 3-methyl-1-butynyl, 1,1-dimethyl-2-propynyl, 1-ethyl-2-propynyl, 1-hexynyl, 2-hexynyl, 3-hexynyl, 4-hexynyl, 5-hexynyl, 1-methyl-2-pentynyl, 1-methyl-3-pentynyl, 1-methyl-4-pentynyl, 2-methyl-3-pentynyl, 2-methyl-4-pentynyl, 3-methyl-1-pentynyl, 3-methyl-4-pentynyl, 4-methyl-1-pentynyl, 4-methyl-2-pentynyl, 1,1-dimethyl-2-butynyl, 1,1-dimethyl-3-butynyl, 1,2-dimethyl-3-butynyl, 2,2-dimethyl-3-butynyl, 3,3-dimethyl-1-butynyl, 1-ethyl-2-butynyl, 1-ethyl-3-butynyl, 2-ethyl-3-butynyl, 1-ethyl-1-methyl-2-propynyl and the like.

The term "haloalkynyl" as used herein, which is also expressed as "alkynyl which is substituted with halogen", refers to unsaturated straight-chain or branched hydrocarbon radicals having usually 2 to 6 carbon atoms (= C₂-C₆-haloalkynyl), preferabyl 2 to 4 carbon atoms (= C₂-C₄-haloalkynyl) or 2 or 3 carbon atoms (= C₂-C₃-haloalkynyl), and a triple bond in any position (as mentioned above), where some or all of the hydrogen atoms in these groups are replaced by halogen atoms as mentioned above, in particular fluorine, chlorine and bromine.

The term "cycloalkyl" as used herein (and in the cycloalkyl moieties of other groups comprising a cycloalkyl group, e.g. cycloalkoxy and cycloalkylalkyl) denotes in each case a mono- or bicyclic, saturated cycloaliphatic radical having usually from 3 to 8 carbon atoms (= C₃-C₈-cycloalkyl), preferably 3 to 6 carbon atoms (= C₃-C₆-cycloalkyl), 3 to 5 carbon atoms (= C₃-C₅-cycloalkyl) or 3 to 4 carbon atoms (= C₃-C₄-cycloalkyl) as (only) ring members. Examples of monocyclic saturated cycloaliphatic radicals having 3 or 4 carbon atoms comprise cyclopropyl and cyclobutyl. Examples of monocyclic saturated cycloaliphatic radicals having 3 to 5 carbon atoms comprise cyclopropyl, cyclobutyl and cyclopentyl. Examples of monocyclic saturated cycloaliphatic radicals having 3 to 6 carbon atoms comprise cyclopropyl, cyclobutyl, cyclopentyl and cyclohexyl. Examples of monocyclic saturated cycloaliphatic radicals having 3 to 8 carbon atoms comprise cyclopropyl, cyclobutyl, cyclopentyl, cyclohexyl, cycloheptyl and cyclooctyl. C₅-C₆-Cycloalkyl is cyclopentyl or cyclohexyl. Examples of bicyclic radicals having 6 to 8 carbon atoms comprise bicyclo[2.1.1]hexyl, bicyclo[2.2.1]heptyl, bicyclo[3.1.1]heptyl, bicyclo[2.2.1]heptyl, bicyclo[2.2.2]octyl and bicyclo[3.2.1]octyl.

The term "halocycloalkyl" as used herein (and in the halocycloalkyl moieties of other groups comprising an halocycloalkyl group) denotes in each case a mono- or bicyclic cycloaliphatic radical having usually from 3 to 8 carbon atoms ("C₃-C₈-halocycloalkyl"), preferably 3 to 5 carbon atoms ("C₃-C₅-halocycloalkyl"), wherein at least one, e.g. 1, 2, 3, 4 or 5 of the hydrogen atoms are replaced by halogen, in particular by fluorine or chlorine. Examples are 1- and 2- fluorocyclopropyl, 1,2-, 2,2- and 2,3-difluorocyclopropyl, 1,2,2-trifluorocyclopropyl, 2,2,3,3-tetrafluorocyclpropyl, 1- and 2-chlorocyclopropyl, 1,2-, 2,2- and 2,3-dichlorocyclopropyl, 1,2,2-trichlorocyclopropyl, 2,2,3,3-tetrachlorocyclpropyl, 1-,2- and 3-fluorocyclopentyl, 1,2-, 2,2-, 2,3-, 3,3-, 3,4-, 2,5-difluorocyclopentyl, 1-,2-and 3-chlorocyclopentyl, 1,2-, 2,2-, 2,3-, 3,3-, 3,4-, 2,5-dichlorocyclopentyl and the like.

The term "hydroxycycloalkyl" denotes in each case a mono- or bicyclic cycloaliphatic radical having usually from 3 to 6 carbon atoms ("hydroxy-(C₃-C₆)-cycloalkyl"), preferably 3 to 5 carbon atoms ("hydroxy-(C₃-C₅)-cycloalkyl"), wherein at least one, e.g. 1, 2, 3, 4 or 5 of the hydrogen atoms are replaced by a hydroxyl group. Examples are 1-hydroxycyclopropyl, 2-hydroxycyclopropyl, 1,2-dihydroxycyclopropyl, 2,3-dihydroxycyclopropyl, 1-hydroxycyclobutyl, 2-hydroxycyclobutyl, 3-hydroxycyclobutyl, 1,2-dihydroxycyclobutyl, 1,3-dihydroxycyclobutyl, 2,3-dihydroxycyclobutyl, 1-hydroxycyclopentyl, 2-hydroxycyclopentyl, 3-hydroxycyclopentyl, 1,2-dihydroxycyclopentyl, 1,3-dihydroxycyclopentyl, 2,3-dihydroxycyclopentyl and the like.

The term "alkoxy" as used herein denotes in each case a straight-chain or branched alkyl group usually having from 1 to 6 carbon atoms (= C₁-C₆-alkoxy), preferably 1 to 3 carbon atoms (= C₁-C₃-alkoxy), in particular 1 or 2 carbon atoms (= C₁-C₂-alkoxy), which is bound to the remainder of the molecule via an oxygen atom. C₁-C₂-Alkoxy is methoxy or ethoxy. C₁-C₃-Alkoxy is additionally, for example, n-propoxy or 1-methylethoxy (isopropoxy). C₁-C₆-alkoxy is additionally, for example, butoxy, 1-methylpropoxy (sec-butoxy), 2-methylpropoxy (isobutoxy), or 1,1-dimethylethoxy (tert-butoxy), pentoxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy, or 1-ethyl-2-methylpropoxy.

The term "haloalkoxy" as used herein denotes in each case a straight-chain or branched alkoxy group, as defined above, having from 1 to 6 carbon atoms (= C₁-C₆-haloalkoxy), preferably 1 to 3 carbon atoms (= C₁-C₃-haloalkoxy), in particular 1 or 2 carbon atoms (= C₁-C₂-haloalkoxy), wherein the hydrogen atoms of this group are partially or totally replaced with halogen atoms, in particular fluorine atoms (in this case, the radical is also termed fluorinated alkoxy). C₁-C₂-Haloalkoxy is, for example, OCH₂F, OCHF₂, OCF₃, OCH₂Cl, OCHCl₂, OCCl₃, chlorofluoromethoxy, dichlorofluoromethoxy, chlorodifluoromethoxy, 2-fluoroethoxy, 2-chloroethoxy, 2-bromoethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy, or OC₂F₅.C₁-C₃-Haloalkoxy is additionally, for example, 2-fluoropropoxy, 3-fluoropropoxy, 2,2-difluoropropoxy, 2,3-difluoropropoxy, 2-chloropropoxy, 3-chloropropoxy, 2,3-dichloropropoxy, 2-bromopropoxy, 3-bromopropoxy, 3,3,3-trifluoropropoxy, 3,3,3-trichloropropoxy, OCH₂-C₂F₅, OCF₂-C₂F₅, 1-(CH₂F)-2-fluoroethoxy, 1-(CH₂Cl)-2-chloroethoxy, or 1-(CH₂Br)-2-bromoethoxy. C₁-C₆-Haloalkoxy is additionally, for example, 4-fluorobutoxy, 4-chlorobutoxy, 4-bromobutoxy, or nonafluorobutoxy , 5-fluoropentoxy, 5-chloropentoxy, 5-brompentoxy, 5-iodopentoxy, undecafluoropentoxy, 6-fluorohexoxy, 6-chlorohexoxy, 6-bromohexoxy, 6-iodohexoxy, or dodecafluorohexoxy.

The term "alkenyloxy" denotes an alkenyl group, as defined above, attached via an oxygen atom to the remainder of the molecule. C₂-C₆-Alkenyloxy is a C₂-C₆-alkenyl group, as defined above, attached via an oxygen atom to the remainder of the molecule. C₃-C₆-Alkenyloxy is a C₃-C₆-alkenyl group, as defined above, attached via an oxygen atom to the remainder of the molecule.

The term "haloalkenyloxy" denotes a haloalkenyl group, as defined above, attached via an oxygen atom to the remainder of the molecule. C₂-C₆-Haloalkenyloxy is a C₂-C₆-haloalkenyl group, as defined above, attached via an oxygen atom to the remainder of the molecule. C₃-C₆-Haloalkenyloxy is a C₃-C₆-haloalkenyl group, as defined above, attached via an oxygen atom to the remainder of the molecule.

The term "alkynyloxy" denotes an alkynyl group, as defined above, attached via an oxygen atom to the remainder of the molecule. C₂-C₆-Alkynyloxy is a C₂-C₆-alkynyl group, as defined above, attached via an oxygen atom to the remainder of the molecule. C₃-C₆-Alkynyloxy is a C₃-C₆-alkynyl group, as defined above, attached via an oxygen atom to the remainder of the molecule.

The term "haloalkynyloxy" denotes a haloalkynyl group, as defined above, attached via an oxygen atom to the remainder of the molecule. C₂-C₆-Haloalkynyloxy is a C₂-C₆-haloalkynyl group, as defined above, attached via an oxygen atom to the remainder of the molecule. C₃-C₆-Haloalkynyloxy is a C₃-C₆-haloalkynyl group, as defined above, attached via an oxygen atom to the remainder of the molecule.

The term "cycloalkoxy" denotes a cycloalkyl group, as defined above, attached via an oxygen atom to the remainder of the molecule. C₃-C₆-Cycloalkoxy is a C₃-C₆-cycloalkyl group, as defined above, attached via an oxygen atom to the remainder of the molecule. Examples of C₃-C₆-cycloalkoxy comprise cyclopropoxy, cyclobutoxy, cyclopentoxy and cyclohexoxy.

The term "alkoxy-alkoxy" as used herein, refers to an alkoxy group, as defined above, where one hydrogen atom is replaced by another alkoxy group, as defined above. The term "C₁-C₃-alkoxy-C₁-C₃-alkoxy" as used herein, refers to an alkoxy group having 1 to 3 carbon atoms, as defined above, where one hydrogen atom is replaced by a C₁-C₃-alkoxy group, as defined above. Examples are methoxymethoxy, ethoxymethoxy, propoxymethoxy, isopropoxymethoxy, 1-methoxyethoxy, 1-ethoxyethoxy, 1-propoxyethoxy, 1-isopropoxyethoxy, 2-methoxyethoxy, 2-ethoxyethoxy, 2-propoxyethoxy, 2-isopropoxyethoxy, 1-methoxypropoxy, 1-ethoxypropoxy, 1-propoxypropoxy, 1-isopropoxypropoxy, 2-methoxypropoxy, 2-ethoxypropoxy, 2-propoxypropoxy, 2-isopropoxypropoxy, 3-methoxypropoxy, 3-ethoxypropoxy, 3-propoxypropoxy, 3-isopropoxypropoxy, and the like.

The term "alkylthio" (also alkylsulfanyl, "alkyl-S" or "alkyl-S(=O)ₖ" (wherein k is 0) as used herein denotes in each case a straight-chain or branched saturated alkyl group as defined above, usually comprising 1 to 6 carbon atoms (= C₁-C₆-alkylthio), preferably 1 to 3 carbon atoms (= C₁-C₃-alkylthio), which is attached via a sulfur atom at any position in the alkyl group. C₁-C₂-Alkylthio is methylthio or ethylthio. C₁-C₃-Alkylthio is additionally, for example, n-propylthio or 1-methylethylthio (isopropylthio). C₁-C₆-Alkylthio is additionally, for example, butylthio, 1-methylpropylthio (secbutylthio), 2-methylpropylthio (isobutylthio), 1,1-dimethylethylthio (tert-butylthio), pentylthio, 1-methylbutylthio, 2-methylbutylthio, 3-methylbutylthio, 1,1-dimethylpropylthio, 1,2-dimethylpropylthio, 2,2-dimethylpropylthio, 1-ethylpropylthio, hexylthio, 1-methylpentylthio, 2-methylpentylthio, 3-methylpentylthio, 4-methylpentylthio, 1,1-dimethylbutylthio, 1,2-dimethylbutylthio, 1,3-dimethylbutylthio, 2,2-dimethylbutylthio, 2,3-dimethylbutylthio, 3,3-dimethylbutylthio, 1-ethylbutylthio, 2-ethylbutylthio, 1,1,2-trimethylpropylthio, 1,2,2-trimethylpropylthio, 1-ethyl-1-methylpropylthio, or 1-ethyl-2-methylpropylthio.

The term "haloalkylthio" as used herein refers to an alkylthio group as defined above wherein the hydrogen atoms are partially or completely substituted with fluorine, chlorine, bromine and/or iodine. C₁-C₂-Haloalkylthio is, for example, SCH₂F, SCHF₂, SCF₃, SCH₂Cl, SCHCl₂, SCCl₃, chlorofluoromethylthio, dichlorofluoromethylthio, chlorodifluoromethylthio, 2-fluoroethylthio, 2-chloroethylthio, 2-bromoethylthio, 2-iodoethylthio, 2,2-difluoroethylthio, 2,2,2-trifluoroethylthio, 2-chloro-2-fluoroethylthio, 2-chloro-2,2-difluoroethylthio, 2,2-dichloro-2-fluoroethylthio, 2,2,2-trichloroethylthio, or SC₂F₅. C₁-C₄-Haloalkylthio is additionally, for example, 2-fluoropropylthio, 3-fluoropropylthio, 2,2-difluoropropylthio, 2,3-difluoropropylthio, 2-chloropropylthio, 3-chloropropylthio, 2,3-dichloropropylthio, 2-bromopropylthio, 3-bromopropylthio, 3,3,3-trifluoropropylthio, 3,3,3-trichloropropylthio, SCH₂-C₂F₅, SCF₂-C₂F₅, 1-(CH₂F)-2-fluoroethylthio, 1-(CH₂Cl)-2-chloroethylthio, 1-(CH₂Br)-2-bromoethylthio, 4-fluorobutylthio, 4-chlorobutylthio, 4-bromobutylthio, or nonafluorobutylthio. C₁-C₆-Haloalkylthio is additionally, for example, 5-fluoropentylthio, 5-chloropentylthio, 5-brompentylthio, 5-iodopentylthio, undecafluoropentylthio, 6-fluorohexylthio, 6-chlorohexylthio, 6-bromohexylthio, 6-iodohexylthio or dodecafluorohexylthio.

The term "alkylsulfinyl" denotes an alkyl group, as defined above, attached via a sulfinyl [S(=O)] group. For example, the term "C₁-C₂-alkylsulfinyl" refers to a C₁-C₂-alkyl group, as defined above, attached via a sulfinyl [S(=O)] group. The term "C₁-C₃-alkylsulfinyl" refers to a C₁-C₃-alkyl group, as defined above, attached via a sulfinyl [S(=O)] group. The term "C₁-C₆-alkylsulfinyl" refers to a C₁-C₆-alkyl group, as defined above, attached via a sulfinyl [S(=O)] group. C₁-C₂-alkylsulfinyl is methylsulfinyl or ethylsulfinyl. C₁-C₃-alkylsulfinyl is additionally, for example, n-propylsulfinyl or 1-methylethylsulfinyl (isopropylsulfinyl). C₁-C₆-alkylsulfinyl is additionally, for example, butylsulfinyl, 1-methylpropylsulfinyl (sec-butylsulfinyl), 2-methylpropylsulfinyl (isobutylsulfinyl), 1,1-dimethylethylsulfinyl (tertbutylsulfinyl), pentylsulfinyl, 1-methylbutylsulfinyl, 2-methylbutylsulfinyl, 3-methylbutylsulfinyl, 1,1-dimethylpropylsulfinyl, 1,2-dimethylpropylsulfinyl, 2,2-dimethylpropylsulfinyl, 1-ethylpropylsulfinyl, hexylsulfinyl, 1-methylpentylsulfinyl, 2-methylpentylsulfinyl, 3-methylpentylsulfinyl, 4-methylpentylsulfinyl, 1,1-dimethylbutylsulfinyl, 1,2-dimethylbutylsulfinyl, 1,3-dimethylbutylsulfinyl, 2,2-dimethylbutylsulfinyl, 2,3-dimethylbutylsulfinyl, 3,3-dimethylbutylsulfinyl, 1-ethylbutylsulfinyl, 2-ethylbutylsulfinyl, 1,1,2-trimethylpropylsulfinyl, 1,2,2-trimethylpropylsulfinyl, 1-ethyl-1-methylpropylsulfinyl, or 1-ethyl-2-methylpropylsulfinyl.

The term "haloalkylsulfinyl" denotes a haloalkyl group, as defined above, attached via a sulfinyl [S(=O)] group to the remainder of the molecule. C₁-C₂-Haloalkylsulfinyl is, for example, S(=O)CH₂F, S(=O)CHF₂, S(=O)CF₃, S(=O)CH₂Cl, S(=O)CHCl₂, S(=O)CCl₃, chlorofluoromethylsulfinyl, dichlorofluoromethylsulfinyl, chlorodifluoromethylsulfinyl, 2-fluoroethylsulfinyl, 2-chloroethylsulfinyl, 2-bromoethylsulfinyl, 2-iodoethylsulfinyl, 2,2-difluoroethylsulfinyl, 2,2,2-trifluoroethylsulfinyl, 2-chloro-2-fluoroethylsulfinyl, 2-chloro-2,2-difluoroethylsulfinyl, 2,2-dichloro-2-fluoroethylsulfinyl, 2,2,2-trichloroethylsulfinyl, or S(=O)C₂F₅. C₁-C₃-Haloalkylsulfinyl is additionally, for example, 2-fluoropropylsulfinyl, 3-fluoropropylsulfinyl, 2,2-difluoropropylsulfinyl, 2,3-difluoropropylsulfinyl, 2-chloropropylsulfinyl, 3-chloropropylsulfinyl, 2,3-dichloropropylsulfinyl, 2-bromopropylsulfinyl, 3-bromopropylsulfinyl, 3,3,3-trifluoropropylsulfinyl, 3,3,3-trichloropropylsulfinyl, S(=O)CH₂-C₂F₅, S(=O)CF₂-C₂F₅, 1-(CH₂F)-2-fluoroethylsulfinyl, 1-(CH₂Cl)-2-chloroethylsulfinylor 1-(CH₂Br)-2-bromoethylsulfinyl. C₁-C₄-Haloalkylsulfinyl is additionally, for example, 4-fluorobutylsulfinyl, 4-chlorobutylsulfinyl, 4-bromobutylsulfinyl, or nonafluorobutylsulfinyl. C₁-C₆-Haloalkylsulfinyl is additionally, for example, 5-fluoropentylsulfinyl, 5-chloropentylsulfinyl, 5-brompentylsulfinyl, 5-iodopentylsulfinyl, undecafluoropentylsulfinyl, 6-fluorohexylsulfinyl, 6-chlorohexylsulfinyl, 6-bromohexylsulfinyl, 6-iodohexylsulfinyl, or dodecafluorohexylsulfinyl.

The term "alkylsulfonyl" denotes an alkyl group, as defined above, attached via a sulfonyl [S(=O)₂] group. The term "C₁-C₂-alkylsulfonyl" refers to a C₁-C₂-alkyl group, as defined above, attached via a sulfonyl [S(=O)₂] group. The term "C₁-C₃-alkylsulfonyl" refers to a C₁-C₃-alkyl group, as defined above, attached via a sulfonyl [S(=O)₂] group. The term "C₁-C₆-alkylsulfonyl" refers to a C₁-C₆-alkyl group, as defined above, attached via a sulfonyl [S(=O)₂] group. C₁-C₂-alkylsulfonyl is methylsulfonyl or ethylsulfonyl. C₁-C₃-alkylsulfonyl is additionally, for example, n-propylsulfonyl or 1-methylethylsulfonyl (isopropylsulfonyl). C₁-C₆-alkylsulfonyl is additionally, for example, butylsulfonyl, 1-methylpropylsulfonyl (sec-butylsulfonyl), 2-methylpropylsulfonyl (isobutylsulfonyl), 1,1-dimethylethylsulfonyl (tert-butylsulfonyl), pentylsulfonyl, 1-methylbutylsulfonyl, 2-methylbutylsulfonyl, 3-methylbutylsulfonyl, 1,1-dimethylpropylsulfonyl, 1,2-dimethylpropylsulfonyl, 2,2-dimethylpropylsulfonyl, 1-ethylpropylsulfonyl, hexylsulfonyl, 1-methylpentylsulfonyl, 2-methylpentylsulfonyl, 3-methylpentylsulfonyl, 4-methylpentylsulfonyl, 1,1-dimethylbutylsulfonyl, 1,2-dimethylbutylsulfonyl, 1,3-dimethylbutylsulfonyl, 2,2-dimethylbutylsulfonyl, 2,3-dimethylbutylsulfonyl, 3,3-dimethylbutylsulfonyl, 1-ethylbutylsulfonyl, 2-ethylbutylsulfonyl, 1,1,2-trimethylpropylsulfonyl, 1,2,2-trimethylpropylsulfonyl, 1-ethyl-1-methylpropylsulfonyl, or 1-ethyl-2-methylpropylsulfonyl.

The term "haloalkylsulfonyl" denotes a haloalkyl group, as defined above, attached via a sulfonyl [S(=O)₂] group to the remainder of the molecule. C₁-C₂-Haloalkylsulfonyl is, for example, S(=O)₂CH₂F, S(=O)₂CHF₂, S(=O)₂CF₃, S(=O)₂CH₂Cl, S(=O)₂CHCl₂, S(=O)₂CCl₃, chlorofluoromethylsulfonyl, dichlorofluoromethylsulfonyl, chlorodifluoromethylsulfonyl, 2-fluoroethylsulfonyl, 2-chloroethylsulfonyl, 2-bromoethylsulfonyl, 2-iodoethylsulfonyl, 2,2-difluoroethylsulfonyl, 2,2,2-trifluoroethylsulfonyl, 2-chloro-2-fluoroethylsulfonyl, 2-chloro-2,2-difluoroethylsulfonyl, 2,2-dichloro-2-fluoroethylsulfonyl, 2,2,2-trichloroethylsulfonyl, or S(=O)₂C₂F₅. C₁-C₃-Haloalkylsulfonyl is additionally, for example, 2-fluoropropylsulfonyl, 3-fluoropropylsulfonyl, 2,2-difluoropropylsulfonyl, 2,3-difluoropropylsulfonyl, 2-chloropropylsulfonyl, 3-chloropropylsulfonyl, 2,3-dichloropropylsulfonyl, 2-bromopropylsulfonyl, 3-bromopropylsulfonyl, 3,3,3-trifluoropropylsulfonyl, 3,3,3-trichloropropylsulfonyl, S(=O)₂CH₂-C₂F₅, S(=O)₂CF₂-C₂F₅, 1-(CH₂F)-2-fluoroethylsulfonyl, 1-(CH₂Cl)-2-chloroethylsulfonylor 1-(CH₂Br)-2-bromoethylsulfonyl. C₁-C₄-Haloalkylsulfonyl is additionally, for example, 4-fluorobutylsulfonyl, 4-chlorobutylsulfonyl, 4-bromobutylsulfonyl, or nonafluorobutylsulfonyl. C₁-C₆-Haloalkylsulfonyl is additionally, for example, 5-fluoropentylsulfonyl, 5-chloropentylsulfonyl, 5-brompentylsulfonyl, 5-iodopentylsulfonyl, undecafluoropentylsulfonyl, 6-fluorohexylsulfonyl, 6-chlorohexylsulfonyl, 6-bromohexylsulfonyl, 6-iodohexylsulfonyl, or dodecafluorohexylsulfonyl.

The substituent "oxo" replaces a CH₂ group by a C(=O) group.

The substituent "cyano" denotes a C≡N group bound to the remainder of the molecule via the carbon atom.

The suffix "-carbonyl" in a group denotes in each case that the group is bound to the remainder of the molecule via a carbonyl C(=O) group. This is the case e.g. in alkylcarbonyl, haloalkylcarbonyl, aminocarbonyl, alkylaminocarbonyl, dialkylaminocarbonyl, alkoxycarbonyl, haloalkoxycarbonyl, arylcarbonyl.

The term "alkoxycarbonyl" denotes an alkoxy group, as defined above, attached via a carbonyl [C(=O)] group to the remainder of the molecule. C₁-C₃-Alkoxycarbonyl is a C₁-C₃-alkoxy group, as defined above, attached via a carbonyl [C(=O)] group to the remainder of the molecule. Examples for C₁-C₃-alkoxycarbonyl are methoxycarbonyl, ethoxycarbonyl, propoxycarbonyl and isopropoxycarbonyl. C₁-C₆-Alkoxycarbonyl is a C₁-C₆-alkoxy group, as defined above, attached via a carbonyl [C(=O)] group to the remainder of the molecule. Examples for C₁-C₆-alkoxycarbonyl are, in addition to those listed for C₁-C₃-alkoxycarbonyl, n-butoxycarbonyl, sec-butoxycarbonyl, isobutoxycarbonyl, tert-butoxycarbonyl, pentoxycarbonyl and hexoxycarbonyl.

The term "haloalkoxycarbonyl" denotes a haloalkoxy group, as defined above, attached via a carbonyl [C(=O)] group to the remainder of the molecule. C₁-C₃-Halolkoxycarbonyl is a C₁-C₃-haloalkoxy group, as defined above, attached via a carbonyl [C(=O)] group to the remainder of the molecule. Examples for C₁-C₃-haloalkoxycarbonyl are - C(=O)OCH₂F, -C(=O)OCHF₂, -C(=O)OCF₃, -C(=O)OCH₂Cl, -C(=O)OCHCl₂, -C(=O)OCCl₃, chlorofluoromethoxycarbonyl, dichlorofluoromethoxycarbonyl, chlorodifluoromethoxycarbonyl, 2-fluoroethoxycarbonyl, 2-chloroethoxycarbonyl, 2-bromoethoxycarbonyl, 2-iodoethoxycarbonyl, 2,2-difluoroethoxycarbonyl, 2,2,2-trifluoroethoxycarbonyl, 2-chloro-2-fluoroethoxycarbonyl, 2-chloro-2,2-difluoroethoxycarbonyl, 2,2-dichloro-2-fluoroethoxycarbonyl, 2,2,2-trichloroethoxycarbonyl, -C(=O)OC₂F₅, 2-fluoropropoxycarbonyl, 3-fluoropropoxycarbonyl, 2,2-difluoropropoxycarbonyl, 2,3-difluoropropoxycarbonyl, 2-chloropropoxycarbonyl, 3-chloropropoxycarbonyl, 2,3-dichloropropoxycarbonyl, 2-bromopropoxycarbonyl, 3-bromopropoxycarbonyl, 3,3,3-trifluoropropoxycarbonyl, 3,3,3-trichloropropoxycarbonyl, -C(=O)OCH₂-C₂F₅, - C(=O)OCF₂-C₂F₅, 1-(CH₂F)-2-fluoroethoxycarbonyl, 1-(CH₂Cl)-2-chloroethoxycarbonyl, or 1-(CH₂Br)-2-bromoethoxycarbonyl.

The term "alkoxycarbonyl-alkyl" denotes an alkyl group, as defined above, in which one hydrogen atom is replaced by an alkoxycarbonyl group, as defined above. C₁-C₆-Alkoxycarbonyl-C₁-C₆-alkyl is a C₁-C₆-alkyl group, as defined above, in which one hydrogen atom is replaced by a C₁-C₆-alkoxycarbonyl group, as defined above. Phenyl-(C₁-C₂)-alkyl is a C₁-C₂-alkyl group, as defined above, in which one hydrogen atom is replaced by a phenyl ring (i.e. the attachment to the remainder of the molecule is via the alkyl group). Examples are benzyl, 1-phenylethyl and 2-phenylethyl. Phenyl-(C₁-C₃)-alkyl is a C₁-C₃-alkyl group, as defined above, in which one hydrogen atom is replaced by a phenyl ring (i.e. the attachment to the remainder of the molecule is via the alkyl group). Examples are benzyl, 1-phenylethyl, 2-phenylethyl, 1-phenylpropyl, 2-phenylpropyl, 3-phenylpropyl, or 2-phenyl-2-propyl. Furanyl-(C₁-C₃)-alkyl is a C₁-C₃-alkyl group, as defined above, in which one hydrogen atom is replaced by a 2-or 3-furanyl ring (i.e. the attachment to the remainder of the molecule is via the alkyl group). Examples are furan-2-yl-methyl, furan-3-yl-methyl, 1-(furan-2-yl)-ethyl, 1-(furan-3-yl)-ethyl, 2-(furan-2-yl)-ethyl, 2-(furan-3-yl)-ethyl and the like. Phenylthio is a phenyl ring attached via an S atom to the remainder of the molecule.

Phenylsulfinyl is a phenyl ring attached via a S(=O) group to the remainder of the molecule.

Phenylsulfonyl is a phenyl ring attached via a S(=O)₂ group to the remainder of the molecule.

The term "aryl" as used herein refers to a polyunsaturated, aromatic hydrocarbyl group having a single ring (i.e. phenyl) or multiple aromatic rings fused together (e.g. naphthyl), typically containing 5 to 12 atoms; preferably 6 to 10, wherein at least one ring is aromatic. Examples of aryl groups include but are not limited to phenyl, naphthyl, anthracyl. Preferred aryl group according to the invention is phenyl.

The term "arylalkyl" as used herein refers to a group -alkyl-aryl, wherein alkyl and aryl are as herein defined. Examples of arylalkyl groups include but are not limited to benzyl.

The term "aryloxycarbonyl" as used herein refers to a group -C(=O)-O-aryl, wherein aryl is as herein defined. Nonlimiting examples of aryloxycarbonyl include phenyloxycarbonyl.

An unsaturated carbocycle contains at least one C-C double bond(s).

An unsaturated heterocycle contains at least one C-C and/or C-N and/or N-N double bond(s).

Partially unsaturated carbocycles contain less than the maximum number of C-C double bond(s) allowed by the ring size.

Partially unsaturated heterocycles contain less than the maximum number of C-C and/or C-N and/or N-N double bond(s) allowed by the ring size.

Examples for three-, four-, five-, or six-membered saturated, partly unsaturated, fully unsaturated or aromatic carbocycles are cyclopropyl, cyclobutyl, cyclopentyl, cyclohexyl, cycloprop-1-enyl, cycloprop-2-enyl, cyclobut-1-enyl, cyclobut-2-enyl, cyclobutadienyl, cyclopent-1-enyl, cyclopent-2-enyl, cyclopent-3-enyl, cyclopenta-1,3-dienyl, cyclopenta-1,4-dienyl, cyclopenta-2,4-dienyl, cyclohex-1-enyl, cyclohex-2-enyl, cyclohex-3-enyl, cyclohexa-1,3-dienyl, cyclohexa-1,4-dienyl, cyclohexa-1,5-dienyl, cyclohexa-2,4-dienyl, cyclohexa-2,5-dienyl, phenol and the like. Examples for three-, four-, five-, six-, or or seven-membered saturated, partly unsaturated, fully unsaturated or aromatic heterocycles are:
3-, 4-, 5-, or 6-membered monocyclic saturated heterocycle: e.g. oxiran-2-yl, thiiran-2-yl, aziridin-1-yl, aziridin-2-yl, oxetan-2-yl, oxetan-3-yl, thietan-2-yl, thietan-3-yl, 1-oxothietan-2-yl, 1-oxothietan-3-yl, 1,1-dioxothietan-2-yl, 1,1-dioxothietan-3-yl, azetidin-1-yl, azetidin-2-yl, azetidin-3-yl, tetrahydrofuran-2-yl, tetrahydrofuran-3-yl, tetrahydrothien-2-yl, tetrahydrothien-3-yl, 1-oxotetrahydrothien-2-yl, 1,1-dioxotetrahydrothien-2-yl, 1-oxotetrahydrothien-3-yl, 1,1-dioxotetrahydrothien-3-yl, 1,3-dioxolan-2-yl, 1,3-dioxolan-4-yl, 1,3-ditholan-2-yl, 1,3-ditholan-4-yl, 1,3-oxathiolan-2-yl, 1,3-oxathiolan-4-yl, 1,3-oxathiolan-5-yl, pyrrolidin-1-yl, pyrrolidin-2-yl, pyrrolidin-3-yl, pyrazolidin-1-yl, pyrazolidin-3-yl, pyrazolidin-4-yl, pyrazolidin-5-yl, imidazolidin-1-yl, imidazolidin-2-yl, imidazolidin-4-yl, oxazolidin-2-yl, oxazolidin-3-yl, oxazolidin-4-yl, oxazolidin-5-yl, isoxazolidin-2-yl, isoxazolidin-3-yl, isoxazolidin-4-yl, isoxazolidin-5-yl, thiazolidin-2-yl, thiazolidin-3-yl, thiazolidin-4-yl, thiazolidin-5-yl, isothiazolidin-2-yl, isothiazolidin-3-yl, isothiazolidin-4-yl, isothiazolidin-5-yl, 1,2,4-oxadiazolidin-3-yl, 1,2,4-oxadiazolidin-5-yl, 1,2,4-thiadiazolidin-3-yl, 1,2,4-thiadiazolidin-5-yl, 1,3,4-oxadiazolidin-2-yl, 1,3,4-thiadiazolidin-2-yl, 2-tetrahydropyranyl, 3-tetrahydropyranyl, 4-tetrahydropyranyl, 1,3-dioxan-2-yl, 1,3-dioxan-4-yl, 1,3-dioxan-5-yl, 1,4-dioxan-2-yl, piperidin-1-yl, piperidin-2-yl, piperidin-3-yl, piperidin-4-yl, hexahydropyridazin-1-yl, hexahydropyridazin-3-yl, hexahydropyridazin-4-yl, hexahydropyrimidin-1-yl, hexahydropyrimidin-2-yl, hexahydropyrimidin-4-yl, hexahydropyrimidin-5-yl, piperazin-1-yl, piperazin-2-yl, morpholin-2-yl, morpholin-3-yl, morpholin-4-yl, thiomorpholin-2-yl, thiomorpholin-3-yl, thiomorpholin-4-yl, 1-oxothiomorpholin-2-yl, 1 oxothiomorpholin-3-yl, 1-oxothiomorpholin-4-yl, 1,1 dioxothiomorpholin-2-yl, 1,1 dioxothiomorpholin-3-yl, 1,1-dioxothiomorpholin-4-yl, and the like;
5- or 6-membered monocyclic partially unsaturated heterocycles: e.g. 2,3-dihydrofuran-2-yl, 2,3-dihydrofuran-3-yl, 2,5-dihydrofuran-2-yl, 2,5-dihydrofuran-3-yl, 2,3-dihydrothien-2-yl, 2,3-dihydrothien-3-yl, 2,5-dihydrothien-2-yl, 2,5-dihydrothien-3-yl, 2-pyrrolin-2-yl, 2-pyrrolin-3-yl, 3-pyrrolin-2-yl, 3-pyrrolin-3-yl, 2-isoxazolin-3-yl, 3-isoxazolin-3-yl, 4-isoxazolin-3-yl, 2-isoxazolin-4-yl, 3-isoxazolin-4-yl, 4-isoxazolin-4-yl, 2-isoxazolin-5-yl, 3-isoxazolin-5-yl, 4-isoxazolin-5-yl, 2-isothiazolin-3-yl, 3-isothiazolin-3-yl, 4-isothiazolin-3-yl, 2-isothiazolin-4-yl, 3-isothiazolin-4-yl, 4-isothiazolin-4-yl, 2-isothiazolin-5-yl, 3-isothiazolin-5-yl, 4-isothiazolin-5-yl, 2,3-dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1-yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 3,4-dihydrooxazol-2-yl, 3,4-dihydrooxazol-3-yl, 3,4-dihydrooxazol-4-yl, 3,6-dihydro-2H-pyran-2-, -3-, -4-, -5-, or 6-yl, 3,4-dihydro-2H-pyran-2-, -3-, -4-, -5- or 6-yl, 3,6-dihydro-2H-thiopyran-2-, -3-, -4-, -5- or 6-yl, 3,4-dihydro-2H-thiopyran-2-, -3-, -4-, -5-, or 6-yl, 2-, 3-, 4-, 5-, or 6-di- or tetrahydropyridinyl, 3-di- or tetrahydropyridazinyl, 4-di- or tetrahydropyridazinyl, 2-di- or tetrahydropyrimidinyl, 4-di- or tetrahydropyrimidinyl, 5-di- or tetrahydropyrimidinyl, di- or tetrahydropyrazinyl;

If two radicals bound on the same nitrogen atom (for example R^{b} and R^{h}, R^{b2} and R^{b3}), together with the nitrogen atom to which they are bound, form a 3-, 4-, 5-, 6-, or 7-membered, saturated heterocyclic ring which may contain as a ring member a further heteroatom or heteroatom group selected from the group consisting of N, O, S, S(=O) and S(=O)₂, this is for example aziridn-1-yl, azetidin-1-yl, pyrrolidin-1-yl, pyrazolidin-1-yl, imidazolidin-1-yl, oxazolidin-3-yl, thiazolidin-3-yl, isoxazolidin-2-yl, isothiazolin-2-yl, piperidin-1-yl, piperazin-1-yl, morpholin-1-yl, thiomorpholin-1-yl, 1-oxothiomorpholin-1-yl, 1,1-dioxothiomorpholin-1-yl, azepan-1-yl, or 1,4-diazepan-1-yl.

Bicyclic ring systems in terms of the present invention contain two rings which have at least one ring atom in common. The term comprises condensed (fused) ring systems, in which the two rings have two neighboring ring atoms in common, as well as spiro systems, in which the rings have only one ring atom in common, and bridged systems with at least three ring atoms in common. If not specified otherwise, the bicyclic rings can be carbocyclic, containing only carbon atoms as ring members, as well as heterocyclic, containing at least one heteroatom or heteroatom group generally selected from N, O S, S(=O), and S(=O)₂ as ring member(s). Further details are given below.

Tricyclic rings contain three rings, each of which having at least one ring atom in common with at least one of the other rings of the polycyclic system. The rings can be condensed, spiro-bound or bridged; mixed systems (e.g. one ring is spiro-bound to a condensed system, or a bridged system is condensed with another ring) are also possible. If not specified otherwise, the polycyclic rings can be carbocyclic, containing only carbon atoms as ring members, as well as heterocyclic, containing at least one heteroatom or heteroatom group generally selected from N, O, S, S(=O), and S(=O)₂ as ring member(s). Further details are given below.

Examples for 3-, 4-, 5-, 6-, 7-, or 8-membered partly unsaturated or fully unsaturated monocyclic carbocyclic rings are cycloprop-1-enyl, cycloprop-2-enyl, cyclobut-1-enyl, cyclobut-2-enyl, cyclobutadienyl, cyclopent-1-enyl, cyclopent-2-enyl, cyclopent-3-enyl, cyclopenta-1,3-dienyl, cyclopenta-1,4-dienyl, cyclopenta-2,4-dienyl, cyclohex-1-enyl, cyclohex-2-enyl, cyclohex-3-enyl, cyclohexa-1,3-dienyl, cyclohexa-1,4-dienyl, cyclohexa-1,5-dienyl, cyclohexa-2,4-dienyl, cyclohexa-2,5-dienyl, cyclohept-1-enyl, cyclohept-2-enyl, cyclohept-3-enyl, cyclohept-4-enyl, cyclohepta-1,3-dienyl, cyclohepta-1,4-dienyl, cyclohepta-1,5-dienyl, cyclohepta-1,6-dienyl, cyclohepta-2,4-dienyl, cyclohepta-2,5-dienyl, cyclohepta-2,6-dienyl, cyclohepta-3,5-dienyl, cyclohepta-1,3,5-trienyl, cyclooct-1-enyl, cyclooct-2-enyl, cyclooct-3-enyl, cyclooct-4-enyl, cyclooct-5-enyl, cyclooct-6-enyl, cyclooct-7-enyl, cycloocta-1,3-dienyl, cycloocta-1,4-dienyl, cycloocta-1,5-dienyl, cycloocta-1,6-dienyl, cycloocta-1,7-dienyl, cycloocta-2,4-dienyl, cycloocta-2,5-dienyl, cycloocta-2,6-dienyl, cycloocta-2,7-dienyl, cycloocta-3,5-dienyl, cycloocta-3,6-dienyl, cycloocta-1,3,5-trienyl, cycloocta-1,3,7-trienyl, cycloocta-2,4,6-trienyl, cyclooctatetraenyl.

Examples for 3-, 4-, 5-, 6-, 7-, or 8-membered saturated, partly unsaturated, fully unsaturated or aromatic heterocyclic rings are:
3-, 4-, 5-, 6-, 7-, or 8-membered monocyclic saturated heterocycles: e.g. oxiran-2-yl, thiiran-2-yl, aziridin-1-yl, aziridin-2-yl, oxetan-2-yl, oxetan-3-yl, thietan-2-yl, thietan-3-yl, 1-oxothietan-2-yl, 1-oxothietan-3-yl, 1,1-dioxothietan-2-yl, 1,1-dioxothietan-3-yl, azetidin-1-yl, azetidin-2-yl, azetidin-3-yl, tetrahydrofuran-2-yl, tetrahydrofuran-3-yl, tetrahydrothien-2-yl, tetrahydrothien-3-yl, 1-oxotetrahydrothien-2-yl, 1,1-dioxotetrahydrothien-2-yl, 1-oxotetrahydrothien-3-yl, 1,1-dioxotetrahydrothien-3-yl, 1,3-dioxolan-2-yl, 1,3-dioxolan-4-yl, 1,3-dithiolan-2-yl, 1,3-dithiolan-4-yl, 1,3-oxathiolan-2-yl, 1,3-oxathiolan-4-yl, 1,3-oxathiolan-5-yl, pyrrolidin-1-yl, pyrrolidin-2-yl, pyrrolidin-3-yl, pyrazolidin-1-yl, pyrazolidin-3-yl, pyrazolidin-4-yl, pyrazolidin-5-yl, imidazolidin-1-yl, imidazolidin-2-yl, imidazolidin-4-yl, oxazolidin-2-yl, oxazolidin-3-yl, oxazolidin-4-yl, oxazolidin-5-yl, isoxazolidin-2-yl, isoxazolidin-3-yl, isoxazolidin-4-yl, isoxazolidin-5-yl, thiazolidin-2-yl, thiazolidin-3-yl, thiazolidin-4-yl, thiazolidin-5-yl, isothiazolidin-2-yl, isothiazolidin-3-yl, isothiazolidin-4-yl, isothiazolidin-5-yl, 1,2,4-oxadiazolidin-3-yl, 1,2,4-oxadiazolidin-5-yl, 1,2,4-thiadiazolidin-3-yl, 1,2,4-thiadiazolidin-5-yl, 1,3,4-oxadiazolidin-2-yl, 1,3,4-thiadiazolidin-2-yl, 1,2,4-triazolidin-1-yl, 1,2,4-triazolidin-3-yl, 1,2,4-triazolidin-4-yl, 2-tetrahydropyranyl, 3-tetrahydropyranyl, 4-tetrahydropyranyl, 1,3-dioxan-2-yl, 1,3-dioxan-5-yl, 1,4-dioxan-2-yl, piperidin-1-yl, piperidin-2-yl, piperidin-3-yl, piperidin-4-yl, hexahydropyridazin-1-yl, hexahydropyridazin-3-yl, hexahydropyridazin-4-yl, hexahydropyrimidin-1-yl, hexahydropyrimidin-2-yl, hexahydropyrimidin-4-yl, hexahydropyrimidin-5-yl, piperazin-1-yl, piperazin-2-yl, 1,3,5-hexahydrotriazin-1-yl, 1,3,5-hexahydrotriazin-2-yl, 1,2,4-hexahydrotriazin-1-yl, 1,2,4-hexahydrotriazin-2-yl, 1,2,4-hexahydrotriazin-3-yl, 1,2,4-hexahydrotriazin-4-yl, 1,2,4-hexahydrotriazin-5-yl, 1,2,4-hexahydrotriazin-6-yl, morpholin-2-yl, morpholin-3-yl, morpholin-4-yl, thiomorpholin-2-yl, thiomorpholin-3-yl, thiomorpholin-4-yl, 1-oxothiomorpholin-2-yl, 1-oxothiomorpholin-3-yl, 1-oxothiomorpholin-4-yl, 1,1-dioxothiomorpholin-2-yl, 1,1-dioxothiomorpholin-3-yl, 1,1-dioxothiomorpholin-4-yl, azepan-1-, -2-, -3-, or-4-yl, oxepan-2-, -3-, -4-, or -5-yl, hexahydro-1,3-diazepinyl, hexahydro-1,4-diazepinyl, hexahydro-1,3-oxazepinyl, hexahydro-1,4-oxazepinyl, hexahydro-1,3-dioxepinyl, hexahydro-1,4-dioxepinyl, oxocane, thiocane, azocanyl, [1,3]diazocanyl, [1,4]diazocanyl, [1,5]diazocanyl, [1,5]oxazocanyl and the like;
3-, 4-, 5-, 6-, 7-, or 8-membered partially unsaturated heteromonocyclic rings: 2,3-dihydrofuran-2-yl, 2,3-dihydrofuran-3-yl, 2,4-dihydrofuran-2-yl, 2,4-dihydrofuran-3-yl, 2,3-dihydrothien-2-yl, 2,3-dihydrothien-3-yl, 2,4-dihydrothien-2-yl, 2,4-dihydrothien-3-yl, 2-pyrrolin-2-yl, 2-pyrrolin-3-yl, 3-pyrrolin-2-yl, 3-pyrrolin-3-yl, 2-isoxazolin-3-yl, 3-isoxazolin-3-yl, 4-isoxazolin-3-yl, 2-isoxazolin-4-yl, 3-isoxazolin-4-yl, 4-isoxazolin-4-yl, 2-isoxazolin-5-yl, 3-isoxazolin-5-yl, 4-isoxazolin-5-yl, 2-isothiazolin-3-yl, 3-isothiazolin-3-yl, 4-isothiazolin-3-yl, 2-isothiazolin-4-yl, 3-isothiazolin-4-yl, 4-isothiazolin-4-yl, 2-isothiazolin-5-yl, 3-isothiazolin-5-yl, 4-isothiazolin-5-yl, 2,3-dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1-yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 3,4-dihydrooxazol-2-yl, 3,4-dihydrooxazol-3-yl, 3,4-dihydrooxazol-4-yl, 2-, 3-, 4-, 5-, or 6-di- or tetrahydropyridinyl, 3-di- or tetrahydropyridazinyl, 4-di- or tetrahydropyridazinyl, 2-di- or tetrahydropyrimidinyl, 4-di- or tetrahydropyrimidinyl, 5-di- or tetrahydropyrimidinyl, di- or tetrahydropyrazinyl, 1,3,5-di- or tetrahydrotriazin-2-yl, 1,2,4-di- or tetrahydrotriazin-3-yl, 2,3,4,5-tetrahydro[1H]azepin-1-, -2-, -3-, -4-, -5-, -6-, or -7-yl, 3,4,5,6-tetrahydro[2H]azepin-2-, -3-, -4-, -5-, -6-, or -7-yl, 2,3,4,7-tetrahydro[1H]azepin-1-, -2-, -3-, -4-, -5-, -6-, or -7-yl, 2,3,6,7-tetrahydro[1H]azepin-1-, -2-, -3-, -4-, -5-, -6-, or -7-yl, tetrahydrooxepinyl, such as 2,3,4,5-tetrahydro[1H]oxepin-2-, -3-, -4-, -5-, -6-, or -7-yl, 2,3,4,7-tetrahydro[1H]oxepin-2-, -3-, -4-, -5-, -6-, or -7-yl, 2,3,6,7-tetrahydro[1H]oxepin-2-, -3-, -4-, -5-, -6-, or -7-yl, tetrahydro-1,3-diazepinyl, tetrahydro-1,4-diazepinyl, tetrahydro-1,3-oxazepinyl, tetrahydro-1,4-oxazepinyl, tetrahydro-1,3-dioxepinyl, tetrahydro-1,4-dioxepinyl, 1,2,3,4,5,6-hexahydroazocine, 2,3,4,5,6,7-hexahydroazocine, 1,2,3,4,5,8-hexahydroazocine, 1,2,3,4,7,8-hexahydroazocine, 1,2,3,4,5,6-hexahydro-[1,5]diazocine, 1,2,3,4,7,8-hexahydro-[1,5]diazocine;
3-, 4-, 5-, 6-, 7-, or 8-membered maximally unsaturated (but not aromatic) heteromonocyclic rings: pyran-2-yl, pyran-3-yl, pyran-4-yl, thiopryran-2-yl, thiopryran-3-yl, thiopryran-4-yl, 1-oxothiopryran-2-yl, 1-oxothiopryran-3-yl, 1-oxothiopryran-4-yl, 1,1-dioxothiopryran-2-yl, 1,1-dioxothiopryran-3-yl, 1,1-dioxothiopryran-4-yl, 2H-oxazin-2-yl, 2H-oxazin-3-yl, 2H-oxazin-4-yl, 2H-oxazin-5-yl, 2H-oxazin-6-yl, 4H-oxazin-3-yl, 4H-oxazin-4-yl, 4H-oxazin-5-yl, 4H-oxazin-6-yl, 6H-oxazin-3-yl, 6H-oxazin-4-yl, 7H-oxazin-5-yl, 8H-oxazin-6-yl, 2H-1,3-oxazin-2-yl, 2H-1,3-oxazin-4-yl, 2H-1,3-oxazin-5-yl, 2H-1,3-oxazin-6-yl, 4H-1,3-oxazin-2-yl, 4H-1,3-oxazin-4-yl, 4H-1,3-oxazin-5-yl, 4H-1,3-oxazin-6-yl, 6H-1,3-oxazin-2-yl, 6H-1,3-oxazin-4-yl, 6H-1,3-oxazin-5-yl, 6H-1,3-oxazin-6-yl, 2H-1,4-oxazin-2-yl, 2H-1,4-oxazin-3-yl, 2H-1,4-oxazin-5-yl, 2H-1,4-oxazin-6-yl, 4H-1,4-oxazin-2-yl, 4H-1,4-oxazin-3-yl, 4H-1,4-oxazin-4-yl, 4H-1,4-oxazin-5-yl, 4H-1,4-oxazin-6-yl, 6H-1,4-oxazin-2-yl, 6H-1,4-oxazin-3-yl, 6H-1,4-oxazin-5-yl, 6H-1,4-oxazin-6-yl, 1,4-dioxine-2-yl, 1,4-oxathiin-2-yl, 1H-azepine, 1H-[1,3]-diazepine, 1H-[1,4]-diazepine, [1,3]diazocine, [1,5]diazocine, [1,5]diazocine;
5- or 6-membered monocyclic aromatic heterocyclic rings: e.g. 2-furyl, 3-furyl, 2 thienyl, 3-thienyl, 1-pyrrolyl, 2-pyrrolyl, 3-pyrrolyl, 1¬-pyrazolyl, 3¬-pyrazolyl, 4 pyrazolyl, 5-pyrazolyl, 1 imidazolyl, 2-imidazolyl, 4-imidazolyl, 5-imidazolyl, 2-oxazolyl, 4-oxazolyl, 5 oxazolyl, 3-isoxazolyl, 4-isoxazolyl, 5-isoxazolyl, 2-thiazolyl, 4-thiazolyl, 5 thiazolyl, 3-isothiazolyl, 4-isothiazolyl, 5-isothiazolyl, 1,3,4-triazol-1-yl, 1,3,4-triazol-2-yl, 1,3,4-triazol-3-yl, 1,2,3-triazol-1-yl, 1,2,3-triazol-2-yl, 1,2,3-triazol-4-yl, 1,2,5-oxadiazol-3-yl, 1,2,3-oxadiazol-4-yl, 1,2,3-oxadiazol-5-yl, 1,3,4-oxadiazol-2-yl, 1,2,5-thiadiazol-3-yl, 1,2,3-thiadiazol-4-yl, 1,2,3-thiadiazol-5-yl, 1,3,4-thiadiazol-2-yl, 1,2,3,4-tetrazol-1-yl, 1,2,3,4-tetrazol-2-yl, 1,2,3,4-[1H]-tetrazol-5-yl, 1,2,3,4-[2H]-tetrazol-5-yl, 2-pyridinyl, 3-pyridinyl, 4 pyridinyl, 1-oxopyridin-2-yl, 1-oxopyridin-3-yl, 1-oxopyridin-4-yl, 3-pyridazinyl, 4-pyridazinyl, 2-pyrimidinyl, 4 pyrimidinyl, 5-pyrimidinyl, 2-pyrazinyl, 1,3,5-triazin-2-yl, 1,2,4-triazin-3-yl, 1,2,4-triazin-5-yl, 1,2,3,4-tetrazin-1-yl, 1,2,3,4-tetrazin-2-yl, 1,2,3,4-tetrazin-5-yl.

Examples for 5- to 8-membered bicyclic spirocyclic saturated carbocyclic rings comprise spiro[2.2]pentyl, spiro[2.3]hexyl, spiro[2.4]heptyl, spiro[3.3]heptyl, spiro[4.4]nonyl, spiro[5.5]undecyl.

Examples of 5- to 8-membered bicyclic condensed saturated carbocyclic rings comprise bicyclo[3.1.0]hexyl, bicyclo[3.2.0]heptyl, bicyclo[3.3.0]octyl, 1,2,3,3a,4,5,6,6a-octahydropentalenyl, bicyclo[4.2.0]octyl.

Examples of 5- to 8-membered bicyclic bridged saturated carbocyclic rings comprise bicyclo[1.1.1]pentyl, bicyclo[2.1.1]hexyl, bicyclo[2.2.1]heptyl, bicyclo[3.1.1]heptyl, bicyclo[2.2.2]octyl, bicyclo[3.2.1]octyl.

An example for a 5- to 8-membered polycyclic saturated carbocyclic is cubyl.

An example for a 5- to 8-membered partly unsaturated bicyclic bridged carbocyclic ring is bicyclo[2.2.2]oct-2-enyl. Examples for saturated 5- to 8-membered bicyclic condensed heterocyclic rings are:

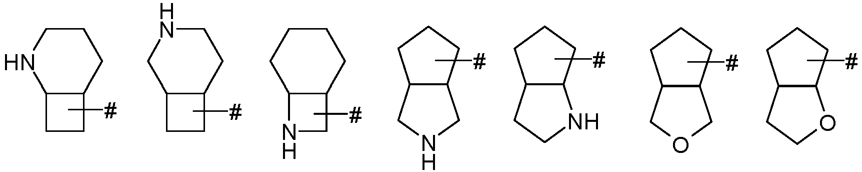

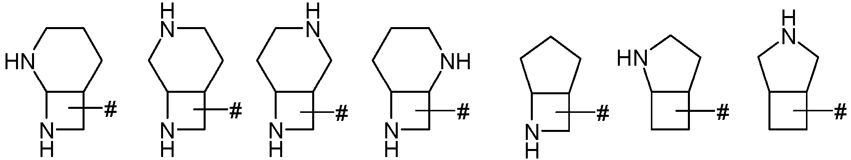

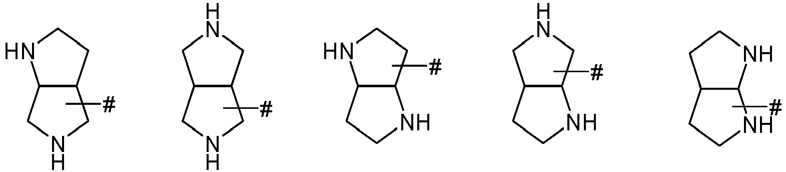

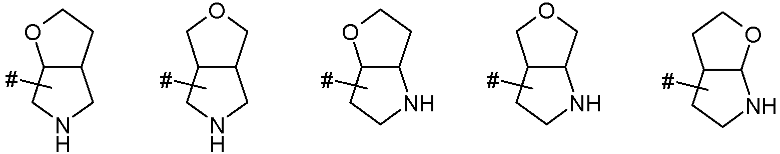

Examples for saturated 5- to 8-membered bicyclic spirocyclic heterocyclic rings are:

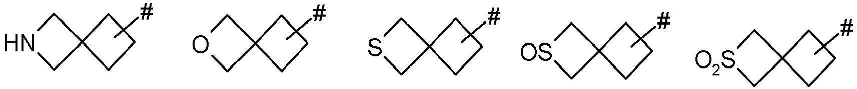

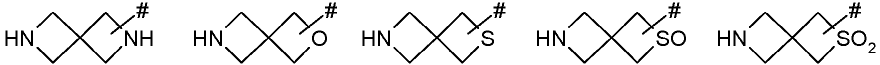

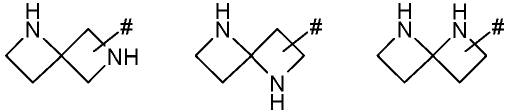

Examples for saturated 5- to 8-membered bicyclic bridged heterocyclic rings are:

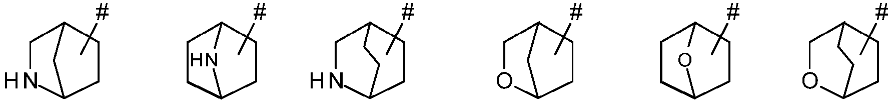

Examples for partly unsaturated 5- to 8-membered bicyclic bridged heterocyclic rings are:

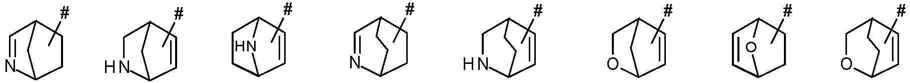

In the above structures # denotes the attachment point to the remainder of the molecule. The attachment point is not restricted to the ring on which this is shown, but can be on either of the two rings, and may be on a carbon or on a nitrogen ring atom. If the rings carry one or more substituents, these may be bound to carbon and/or to nitrogen ring atoms.

Variable Ar is a bicyclic or tricyclic saturated, partially unsaturated, partially aromatic condensed, or aromatic ring system. The ring system can be carbocyclic, containing only carbon atoms as ring members, or heterocyclic, containing at least one N, O and/or S atom as ring member. The carbon or sulfur ring atoms of Ar bear 0, 1, or 2 oxo groups. A carbon ring atom can of course bear only 0 or 1 oxo group froming a ring member -(C=O)-, a sulfur ring atom can however bear 0, 1 and 2 oxo group and thus form a ring member -S-, -S(=O)-, or -S(=O)₂-.

Aromatic ring systems are throughout aromatic. In partially aromatic condensed ring systems, an aromatic ring is condensed to a nonaromatic ring.

Examples for bicyclic or tricyclic carbocyclic aromatic ring systems are naphthyl, anthracenyl and phenanthrenyl. Examples for bicyclic or tricyclic carbocyclic partially aromatic ring systems are indanyl, indenyl, tetralinyl, 1,2-dihydrotetralinyl, 1,4-dihydrotetralinyl, 9H-fluorenyl and 9,10-dihydroanthracenyl.

Examples for bicyclic or tricyclic heterocyclic aromatic ring systems are indolyl, 2H-isoindolyl, benzofuranyl, benzothiophenyl, benzimidazolyl, 1H-indazolyl, 1,3-benzoxazolyl, 1,2-benzoxazolyl, 1,3-benzothiazolyl, 1,2-benzothiazolyl, quinolinyl, isoquinolinyl, quinoxalinyl, cinnolinyl, quinazolinyl, 1,8-naphthyridinyl, 1,5-naphthyridinyl, 1H-pyrrolo[3,2-b]pyridinyl, 1H-pyrrolo[3,2-c]pyridinyl, dibenzofurayl, dibenzothiophenyl, 9H-carbazolyl, acridinyl, phenazinyl.

Examples for bicyclic or tricyclic heterocyclic partially aromatic ring systems are 2,3-dihydrobenzofuranyl, 1,3-dihydroisobenzofuranyl, 2,3-dihydrobenzothiophenyl, 1-oxo-2,3-dihydrobenzothiophenyl, 1,1-dioxo-2,3-dihydrobenzothiophenyl, 1,3-dihydro-2-benzothiophenyl, 2-oxo-1,3-dihydro-2-benzothiophenyl, 2,2-dioxo-1,3-dihydro-2-benzothiophenyl, indolinyl, isoindolinyl, 1,3-benzodioxolyl, 1,3-benzodithiolyl, 1-oxo-1,3-benzodithiolyl, 1,1-dioxo-1,3-benzodithiolyl, 1,3-dioxo-1,3-benzodithiolyl, 1,1,3,3-tetraoxo-1,3-benzoxathiolyl, 2,3-dihydro-1H-benzimidazolyl, 2,3-dihydro-1H-indazolyl, 2,3-dihydro-1,3-benzoxazolyl, 2,3-dihydro-1,2-benzoxazolyl, 2,3-dihydro-1,3-benzothiazolyl, 2,3-dihydro-1,2-benzothiazolyl, chromanyl, isochromanyl, 4H-1,3-benzodioxinyl, 2,3-dihydro-1,4-benzodioxinyl, 4H-chromenyl, 2H-chromenyl, 1,2,3,4-tetrahydroquinolinyl, 1,2,3,4-tetrahydroisoquinolinyl.

The remarks made below as to further embodiments of the variables (i.e. substituents or other place holders in generic formulae) of the compounds of formula (I) are valid on their own as well as in combination with each other, as well as in combination with the stereoisomers, tautomers or salts thereof. The remarks made below concerning specific embodiments of the variables further are valid on their own as well as preferably in combination with each other concerning the compounds of formula (I), where applicable, as well as concerning the uses and methods according to the invention.

In one embodiment (Ar.1) the variable Ar in compounds of formula (I) is furan-2-yl, furan-3-yl, thien-2-yl, thien-3-yl, pyrrol-2-yl, pyrrol-3-yl, pyrazol-3-yl, pyrazol-4-yl, pyrazol-5-yl, imidazol-2-yl, imidazol-4-yl, imidazol-5-yl, oxazol-2-yl, oxazol-4-yl, oxazol-5-yl, isoxazol-3-yl, isoxazol-4-yl, isoxazol-5-yl, thiazol-2-yl, thiazol-4-yl, thiazol-5-yl, isothiazol-3-yl, isothiazol-4-yl, isothiazol-5-yl, pyridin-2-yl, pyridin-3-yl, pyridin-4-yl, pyridazin-3-yl, pyridazin-4-yl, pyrimidin-2-yl, pyrimidin-4-yl, pyrimidin-5-yl and pyrazin-2-yl; whereas the heteroaromatic ring Ar is substituted with k substituents R^{A1} and q substituents R^{A2} as defined or preferably defined herein.

In one embodiment (Ar.2) variable Ar in compounds of formula (I) is thien-2-yl, thien-3-yl, isoxazol-3-yl, isoxazol-4-yl, isoxazol-5-yl, pyridin-2-yl, pyridin-3-yl, pyridin-4-yl, pyrimidin-2-yl, pyrimidin-4-yl, or pyrimidin-5-yl; whereas the heteroaromatic ring Ar is substituted with k substituents R^{A1} and q substituents R^{A2} as defined or preferably defined herein.

In one embodiment (Ar.3) variable Ar in compounds of formula (I) is pyridin-2-yl, pyridin-3-yl, pyridin-4-yl, pyrimidin-2-yl, pyrimidin-4-yl, or pyrimidin-5-yl; whereas the heteroaromatic ring Ar is substituted with k substituents R^{A1} and q substituents R^{A2} as defined or preferably defined herein.

In one embodiment (Ar.4) variable Ar in compounds of formula (I) is pyridin-2-yl, pyridin-3-yl, pyridin-4-yl; whereas the heteroaromatic ring Ar is substituted with k substituents R^{A1} and q substituents R^{A2} as defined or preferably defined herein.

In one embodiment the variable Ar in compounds of formula (I) is a 5-membered heteroaromatic ring containing 1, 2 or 3 heteroatoms selected from the group consisting of N, O, and S as ring members; or a 6-membered heteroaromatic ring containing 1 or 2 nitrogen atoms as ring members; with the provision that the 5-membered heteroaromatic ring cannot contain 2 or more atoms selected from O and S; whereas the heteroaromatic ring Ar is substituted with k substituents R^{A1} and q substituents R^{A2}; whereas R^{A1} is each independently halogen, cyano, hydroxyl, oxo, (C₁-C₃)-alkyl, (C₁-C₃)-haloalkyl, (C₁-C₃)-alkoxy, or (C₁-C₃)-haloalkoxy; and R^{A2} is each independently phenyl-(C₁-C₃)-alkyl, (C₁-C₄)-alkylcarbonyl, (C₁-C₄)-alkylaminocarbonyl, di-(C₁-C₄-alkyl)aminocarbonyl, (C₁-C₄)-alkoxycarbonyl, benzyloxycarbonyl, fluorenyloxycarbonyl, allyloxycarbonyl, or (C₁-C₃)-alkoxy-(C₁-C₃)-alkyl; whereas k is 0, 1, 2 , or 3 and q is 0 or 1; with the provision that the total number of substituents R^{A1} and R^{A2} on each heteroaromatic ring does not exceed the number of substituents that are theoretically possible; and excluding heteroaromatic rings that bear an electronic charge.

In another embodiment the variable Ar in compounds of formula (I) is a 5-membered heteroaromatic ring containing 1 or 2 heteroatoms selected from the group consisting of N, O, and S as ring members; or a 6-membered heteroaromatic ring containing 1 or 2 nitrogen atoms as ring members; with the provision that the 5-membered heteroaromatic ring cannot contain 2 or more atoms selected from O and S; whereas the heteroaromatic ring Ar is substituted with k substituents R^{A1} and q substituents R^{A2}; whereas R^{A1} is each independently halogen, cyano, hydroxyl, (C₁-C₃)-alkyl, or (C₁-C₃)-haloalkyl; and R^{A2} is each independently (C₁-C₄)-alkoxycarbonyl; whereas k is 0, 1, or 2 and q is 0 or 1; with the provision that the total number of substituents R^{A1} and R^{A2} on each heteroaromatic ring does not exceed the number of substituents that are theoretically possible; and excluding heteroaromatic rings that bear an electronic charge.

In another embodiment the variable Ar in compounds of formula (I) is a 6-membered heteroaromatic ring containing 1 or 2 nitrogen atoms as ring members; whereas the heteroaromatic ring Ar is substituted with k substituents R^{A1}; whereas R^{A1} is each independently halogen, hydroxyl, or (C₁-C₃)-alkyl; k is 0, 1, or 2; and excluding heteroaromatic rings that bear an electronic charge.

In one embodiment of the present invention variable Ar is a bicyclic or tricyclic saturated, partially unsaturated, partially aromatic condensed, or aromatic ring system, which is 8- to 14-membered or 9- to 14-membered; whereas Ar is substituted with k substituents R^{A1} and q substituents R^{A2} as defined or preferably defined herein.

In one embodiment variable Ar is a bicyclic or tricyclic aromatic or partially aromatic condensed ring system, where at least one of the condensed rings is a phenyl ring. Examples for such rings are naphthyl, anthracenyl, phenanthrenyl, indanyl, indenyl, tetralinyl, 1,2-dihydrotetralinyl, 1,4-dihydrotetralinyl, 9H-fluorenyl, 9,10-dihydroanthracenyl, indolyl, 2H-isoindolyl, benzofuranyl, benzothiophenyl, benzimidazolyl, 1H-indazolyl, 1,3-benzoxazolyl, 1,2-benzoxazolyl, 1,3-benzothiazolyl, 1,2-benzothiazolyl, quinolinyl, isoquinolinyl, quinoxalinyl, cinnolinyl, quinazolinyl, dibenzofurayl, dibenzothiophenyl, 9H-carbazolyl, acridinyl, phenazinyl, 2,3-dihydrobenzofuranyl, 1,3-dihydroisobenzofuranyl, 2,3-dihydrobenzothiophenyl, 1-oxo-2,3-dihydrobenzothiophenyl, 1,1-dioxo-2,3-dihydrobenzothiophenyl, 1,3-dihydro-2-benzothiophenyl, 2-oxo-1,3-dihydro-2-benzothiophenyl, 2,2-dioxo-1,3-dihydro-2-benzothiophenyl, indolinyl, isoindolinyl, 1,3-benzodioxolyl, 1,3-benzodithiolyl, 1-oxo-1,3-benzodithiolyl, 1,1-dioxo-1,3-benzodithiolyl, 1,3-dioxo-1,3-benzodithiolyl, 1,1,3,3-tetraoxo-1,3-benzoxathiolyl, 2,3-dihydro-1H-benzimidazolyl, 2,3-dihydro-1H-indazolyl, 2,3-dihydro-1,3-benzoxazolyl, 2,3-dihydro-1,2-benzoxazolyl, 2,3-dihydro-1,3-benzothiazolyl, 2,3-dihydro-1,2-benzothiazolyl, chromanyl, isochromanyl, 4H-1,3-benzodioxinyl, 2,3-dihydro-1,4-benzodioxinyl, 4H-chromenyl, 2H-chromenyl, 1,2,3,4-tetrahydroquinolinyl and 1,2,3,4-tetrahydroisoquinolinyl; whereas Ar is substituted with k substituents R^{A1} and q substituents R^{A2} as defined or preferably defined herein.

In one embodiment (Ar.5) variable Ar in compounds of formula (I) is selected from bicyclic or tricyclic ring systems of formulae P1 to P16: 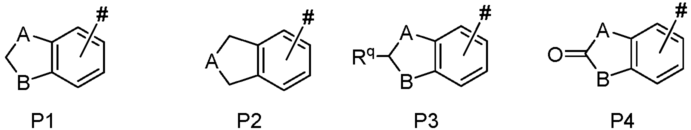 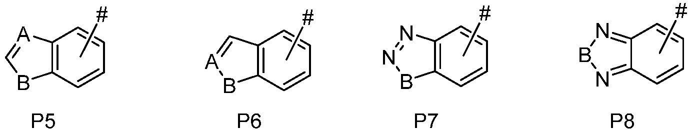 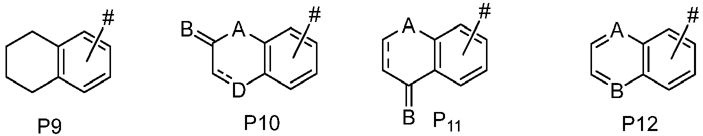 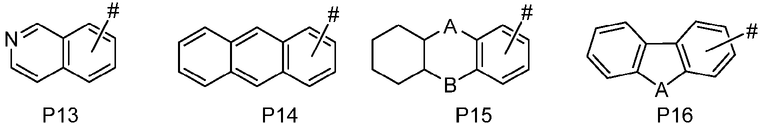 wherein
in P1: A is CH₂, NR^{q}, O, S, S(=O), or S(=O)₂; and B is CH₂, NR^{q}, O, S, S(=O), or S(=O)₂;
in P2: A is NR^{q}, O, S, S(=O), or S(=O)₂;
in P3: A is CH₂, C(=O), NR^{q}, O, S, S(=O), or S(=O)₂; and B is CH₂, C(=O), NR^{q}, O, S, S(=O), or S(=O)₂;
in P4: A is CH₂, NR^{q}, O, S, S(=O), or S(=O)₂; and B is CH₂, NR^{q}, O, S, S(=O), or S(=O)₂;
in P5: A is CH or N; and B is CH₂, NR^{q}, O, S, S(=O), or S(=O)₂;
in P6: A is CH or N; and B is CH₂, NR^{q}, O, S, S(=O), or S(=O)₂;
in P7: B is CH₂, NR^{q}, O, S, S(=O), or S(=O)₂;
in P8: B is CH₂, NR^{q}, O, S, S(=O), or S(=O)₂.
in P10: the dashed line indicates a single bond or a double bond; A is NR^{q} or O; B is O; and D is CH₂, NR^{q} or O if the dashed line indicates a single bond; and is CH or N the dashed line indicates a double bond;
in P11: the dashed line indicates a single bond or a double bond; A is NR^{q} or O; and B is O;
in P12: A is CH or N; and B is CH or N;
in P15: A is CH₂, C(=O), NR^{q}, O, S, S(=O), or S(=O)₂; and B is CH₂, C(=O), NR^{q}, O, S, S(=O), or S(=O)₂;
in P16: A is CH₂, C(=O), NR^{q}, O, S, S(=O), or S(=O)₂; wherein
R^{q} is hydrogen or (C₁-C₃)-alkyl; # is the attachment point to the group -C(=X)NR¹-; and whereas ring systems P1 to P16 are substituted with k substituents R^{A1} and q substituents R^{A2} as defined or preferably defined herein.

In one embodiment (Ar.6) variable Ar in compounds of formula (I) is a bicyclic condensed ring system of formula P1 to P13 as defined in embodiment (Ar.5).

In one embodiment (Ar.7) variable Ar in compounds of formula (I) is a bicyclic condensed ring system of formula P1, P2, P5 and P12 as defined in embodiment (Ar.5).

In one embodiment (Ar.8) variable Ar in compounds of formula (I) is a bicyclic condensed ring system of formula of formula P1, P2, P5 or P12 as defined in embodiment (Ar.5), wherein
in P1: A and B are O;
in P2: A is S, S(=O), or S(O)₂; preferably S(=O)₂;
in P5: A is CH or N; and B is preferably NR^{q}, O, or S;
in P12: A and B are CH; wherein
R^{q} is hydrogen or (C₁-C₃)-alkyl; # is the attachment point to the group -C(=X)NR¹-; and whereas ring systems P1, P2, P5 or P12 are substituted with k substituents R^{A1} and q substituents R^{A2} as defined or preferably defined herein.

Variable k is preferably 0, 1, 2, or 3, more preferably 0, 1 or 2; q is preferably 0, 1, or 2; more preferably 0 or 1; with the provision that the total number of substituents R^{A1} and R^{A2} on each ring or ring system does not exceed the number of substituents that are theoretically possible; and excluding heteroaromatic rings that bear an electronic charge.

In one embodiment (RA1.1) variables R^{A1} are independently of each other halogen, nitro, hydroxyl, oxo, cyano, (C₁-C₃)-alkyl, (C₁-C₃)-haloalkyl, hydroxy-(C₁-C₃)-alkyl, (C₃-C₅)-cycloalkyl, (C₁-C₃)-alkoxy, (C₁-C₃)-haloalkoxy, (C₂-C₃)-alkenyl, (C₂-C₃)-haloalkenyl, (C₂-C₃)-alkynyl, or (C₂-C₃)-haloalkynyl; and whereas the sulfur ring atoms bear 0, 1, or 2 oxo groups; and whereas k is 0, 1, 2, 3, 4, or 5.

In one embodiment (RA1.2) variables R^{A1} are independently of each other halogen, cyano, hydroxyl, oxo, (C₁-C₃)-alkyl, (C₁-C₃)-haloalkyl, (C₁-C₃)-alkoxy, or (C₁-C₃)-haloalkoxy; and whereas k is 0, 1, 2, or 3.

In one embodiment (RA1.3) variables R^{A1} are independently of each other halogen, cyano, hydroxyl, (C₁-C₃)-alkyl, (C₁-C₃)-haloalkyl, (C₁-C₃)-alkoxy, or (C₁-C₃)-haloalkoxy; and whereas k is 0, 1, or 2.

In one embodiment (RA1.4) variables R^{A1} are independently of each other halogen, cyano, hydroxyl, (C₁-C₃)-alkyl, or (C₁-C₃)-haloalkyl; and whereas k is 0, 1, or 2.

In one embodiment variables R^{A2} are independently of each other phenyl-(C₁-C₃)-alkyl, (C₁-C₄)-alkylcarbonyl, aminocarbonyl, (C₁-C₄)-alkylaminocarbonyl, di-(C₁-C₄-alkyl)aminocarbonyl, (C₁-C₄)-alkoxycarbonyl, benzyloxycarbonyl, fluorenyloxycarbonyl, allyloxycarbonyl, (C₁-C₃)-alkoxy-(C₁-C₃)-alkyl, (C₁-C₃)-alkylthio, (C₁-C₃)-alkylsulfinyl, (C₁-C₃)-alkylsulfonyl, or phenylsulfonyl, where the aliphatic or aromatic moieties in the 14 last-mentioned radicals are unsubstituted or substituted by 1, 2, or 3 radicals selected from the group consisting of fluorine, chlorine, bromine, cyano and (C₁-C₂)-alkoxy; and whereas q is 0, 1 or 2.

In one embodiment variables R^{A2} are independently of each other phenyl-(C₁-C₃)-alkyl, (C₁-C₄)-alkylcarbonyl, (C₁-C₄)-alkylaminocarbonyl, di-(C₁-C₄-alkyl)aminocarbonyl, (C₁-C₄)-alkoxycarbonyl, benzyloxycarbonyl, fluorenyloxycarbonyl, allyloxycarbonyl, (C₁-C₃)-alkoxy-(C₁-C₃)-alkyl; and whereas q is 0 or 1.

In one embodiment (RA2.1) variables R^{A2} are independently of each other benzyl, acetyl, methylaminocarbonyl, dimethylaminocarbonyl, (C₁-C₄)-alkoxycarbonyl, methoxymethyl; and whereas q is 0 or 1.

In one embodiment (RA2.2) variables R^{A2} are independently of each other (C₁-C₄)-alkoxycarbonyl; and whereas q is 0 or 1.

If the meaning of R⁷ and R⁹ in compounds of formula (I) is not identical they exhibit a stereocenter at the carbon atom substituted with radicals R⁷, R⁹. Accordingly, they can exist as (S)- or (R)-configured stereoisomers. In the context of the present invention, compounds of formula (I), which are in the (Reconfiguration, i.e. compounds of formula (I.R) as depicted below, are preferred (stereocenter marked with "§").

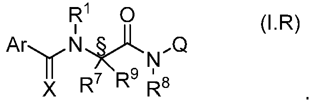

In one embodiment in compounds of formula (I) R¹ is hydrogen or (C₁-C₃)-alkyl, and is preferably hydrogen.

In one embodiment in compounds of formula (I) R⁸ is hydrogen or (C₁-C₃)-alkyl, and is preferably hydrogen.

In one embodiment in compounds of formula (I) R¹ and R⁸ are both hydrogen.

In one embodiment in compounds of formula (I) R⁷ is methyl or ethyl; preferably methyl.

In one embodiment in compounds of formula (I) R⁹ is hydrogen or methyl; preferably hydrogen.

In one embodiment in compounds of formula (I) R⁷ and R⁹ together with the carbon atom to which they are attached form a cyclopropyl ring.

In one embodiment in compounds of formula (I), R⁷ is methyl or ethyl; and R⁹ is hydrogen. In another embodiment R⁷ is methyl and R⁹ is hydrogen.

In one embodiment in compounds of formula (I) X is oxygen.

In one embodiment in compounds of formula (I) R¹⁰ is methyl or ethyl. In particular, R¹⁰ is methyl.

In one embodiment (S.1) in compounds of formula (I), X is O; R¹ and R⁸ are hydrogen; R⁷ is methyl or ethyl; R⁹ is hydrogen or methyl.

In one embodiment (S.2) in compounds of formula (I), X is O, R¹ and R⁸ are hydrogen; R⁷ is methyl; R⁹ is hydrogen; whereas the stereocenter at the carbon atom substituted with radicals R⁷, R⁹ is in the R-configuration.

### Embodiment Q.1: Q represents the radical of formula (Z-Y),

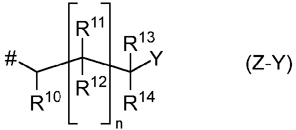

wherein # indicates the bond to the adjacent nitrogen atom, and wherein the substituents have the following meanings, wherein
- R¹⁰: is methyl;
- R¹¹, R¹², R¹³, R¹⁴: are hydrogen; or
- R¹⁰ and R¹⁴: form, together with the carbon atoms to which they are bound, a four- to six-membered saturated or partially unsaturated carbocycle or a five-membered saturated or partially unsaturated heterocycle containing 1 oxygen, nitrogen or sulfur atom as ring members;
- Y: is CO₂R^{e}, CONR^{b}R^{h}, CONR^{e}S(=O)R^{a}, CONR^{e}SO₂R^{a}, or CONR^{b1}SO₂NR^{b2}R^{b3};
- R^{a}: is each independently (C₁-C₆)-alkyl, (C₁-C₆)-haloalkyl, (C₃-C₄)-alkynyl, or (C₃-C₆)-cycloalkyl, each of which is unsubstituted or substituted with m radicals selected from the group consisting of fluorine, chlorine, bromine, iodine, cyano, hydroxy, and (C₁-C₃)-alkoxy;
- R^{b}: is hydrogen, (C₁-C₆)-alkyl, (C₂-C₄)-alkenyl, (C₃-C₄)-alkynyl, (C₃-C₆)-cycloalkyl, (C₃-C₆)-cycloalkyl-(C₁-C₃)-alkyl, phenyl-(C₁-C₃)-alkyl, or furanyl-(C₁-C₃)-alkyl, where each of the seven last-mentioned radicals is unsubstituted or substituted with m radicals selected from the group consisting of fluorine, chlorine, bromine, cyano, CO₂R^{a}, (C₁-C₂)-alkoxy, (C₁-C₃)-alkylthio, (C₁-C₃)-alkylsulfinyl, (C₁-C₃)-alkylsulfonyl, phenylthio, phenylsulfinyl, and phenylsulfonyl;
- R^{b1} and R^{b2}: independently of each other are hydrogen or have independently of each other one of the meanings given for R^{a};
- R^{b3}: is hydrogen, (C₁-C₆)-alkyl, (C₂-C₄)-alkenyl, (C₃-C₄)-alkynyl, (C₃-C₆)-cycloalkyl, (C₃-C₆)-cycloalkyl-(C₁-C₃)-alkyl, phenyl-(C₁-C₃)-alkyl, or furanyl-(C₁-C₃)-alkyl, where each of the seven last-mentioned radicals is unsubstituted or substituted with m radicals selected from the group consisting of fluorine, chlorine, bromine, cyano, CO₂R^{a}, (C₁-C₂)-alkoxy, (C₁-C₃)-alkylthio, (C₁-C₃)-alkylsulfinyl, (C₁-C₃)-alkylsulfonyl, phenylthio, phenylsulfinyl, and phenylsulfonyl; or
- R^{b2} and R^{b3}: form, together with the nitrogen atom to which they are bound, a saturated 3-, 4-, 5-, 6-, or 7-membered heterocycle which may contain one further heteroatom or heteroatom group selected from the group consisting of N, O, S, S(=O) and S(=O)₂ as ring member;
- R^{e}: is hydrogen, (C₁-C₆)-alkyl, (C₂-C₄)-alkenyl, (C₃-C₄)-alkynyl, (C₃-C₆)-cycloalkyl, (C₃-C₆)-cycloalkyl-(C₁-C₃)-alkyl, phenyl-(C₁-C₃)-alkyl, or furanyl-(C₁-C₃)-alkyl, where each of the seven last-mentioned radicals is unsubstituted or substituted with m radicals selected from the group consisting of fluorine, chlorine, bromine, cyano, CO₂R^{a}, (C₁-C₂)-alkoxy, (C₁-C₂)-haloalkoxy, (C₁-C₃)-alkylthio, (C₁-C₃)-haloalkylthio, (C₁-C₃)-alkylsulfinyl, (C₁-C₃)-haloalkylsulfinyl, (C₁-C₃)-alkylsulfonyl, (C₁-C₃)-haloalkylsulfonyl, phenylthio, phenylsulfinyl, and phenylsulfonyl;
- R^{h}: is hydrogen, hydroxyl, (C₁-C₆)-alkyl, (C₁-C₂)-alkoxy, (C₃-C₆)-cycloalkyl, (C₂-C₄)-alkenyl, (C₁-C₆)-alkoxycarbonyl-(C₁-C₆)-alkyl, or (C₃-C₄)-alkynyl, where each of the six last-mentioned radicals is unsubstituted or substituted with m radicals selected from the group consisting of fluorine, chlorine, bromine, cyano, CO₂R^{a}, (C₁-C₂)-alkyl, (C₁-C₂)-alkoxy and (C₁-C₂)-alkoxy-(C₁-C₂)-alkoxy; or
- R^{b} and R^{h}: form, together with the nitrogen atom to which they are bound, a saturated or partially unsaturated 5-, 6-, or 7-membered heterocycle which may contain one further heteroatom or heteroatom group selected from the group consisting of N, O, S, S(=O) and S(=O)₂ as ring member;
- m: is independently of each occurrence 1, 2, or 3;
- n: is 1 or 2.

### Embodiment Q.2: Q represents the radical of formula (Z-Y),

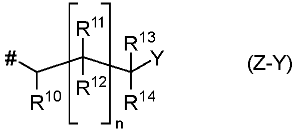

wherein # indicates the bond to the adjacent nitrogen atom, and wherein the substituents have the following meanings, wherein
- R¹⁰: is methyl;
- R¹¹, R¹², R¹³, R¹⁴: are hydrogen; or
- R¹⁰ and R¹⁴: form, together with the carbon atoms to which they are bound, a four- to six-membered saturated or partially unsaturated carbocycle or a five-membered saturated or partially unsaturated heterocycle containing 1 oxygen atom as ring member;
- Y: is CO₂R^{e}, CONR^{b}R^{h}, or CONR^{e}SO₂R^{a};
- R^{a}: is each independently (C₁-C₆)-alkyl, (C₁-C₆)-haloalkyl, (C₃-C₄)-alkynyl, or (C₃-C₆)-cycloalkyl, each of which is unsubstituted or substituted with m radicals selected from the group consisting of fluorine, chlorine, bromine, iodine, cyano, hydroxy, and (C₁-C₃)-alkoxy;
- R^{b}: is hydrogen;
- R^{e}: is hydrogen, (C₁-C₆)-alkyl, or (C₃-C₆)-cycloalkyl, where each of the two last-mentioned radicals is unsubstituted or substituted with m radicals selected from the group consisting of fluorine, chlorine, bromine, cyano, CO₂R^{a}, (C₁-C₂)-alkoxy, (C₁-C₂)-haloalkoxy, (C₁-C₃)-alkylthio, (C₁-C₃)-haloalkylthio, (C₁-C₃)-alkylsulfinyl, (C₁-C₃)-haloalkylsulfinyl, (C₁-C₃)-alkylsulfonyl, (C₁-C₃)-haloalkylsulfonyl, phenylthio, phenylsulfinyl, and phenylsulfonyl;
- R^{h}: is hydrogen, hydroxyl, (C₁-C₆)-alkyl, (C₁-C₂)-alkoxy, (C₃-C₆)-cycloalkyl, (C₂-C₄)-alkenyl, (C₁-C₆)-alkoxycarbonyl-(C₁-C₆)-alkyl, or (C₃-C₄)-alkynyl, where each of the six last-mentioned radicals is unsubstituted or substituted with m radicals selected from the group consisting of fluorine, chlorine, bromine, cyano, CO₂R^{a}, (C₁-C₂)-alkyl, (C₁-C₂)-alkoxy and (C₁-C₂)-alkoxy-(C₁-C₂)-alkoxy; or
- R^{b} and R^{h}: form, together with the nitrogen atom to which they are bound, a saturated or partially unsaturated 6-membered heterocycle which contains 0 or 1 oxygen atom as ring member;
- m: is independently of each occurrence 1 or 2;
- n: is 1 or 2.

### Embodiment Q.3: Q represents the radical of formula Z-Y,

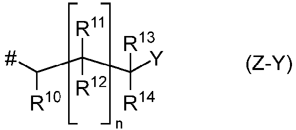

wherein # indicates the bond to the adjacent nitrogen atom, and wherein the substituents have the following meanings, wherein
- R¹⁰: is methyl;
- R¹¹, R¹², R¹³, R¹⁴: are hydrogen; or
- R¹⁰ and R¹⁴: form, together with the carbon atoms to which they are bound, a four- to six-membered saturated or partially unsaturated carbocycle or a five-membered saturated or partially unsaturated heterocycle containing 1 oxygen atom as ring member;
- Y: is CO₂R^{e}, CONR^{b}R^{h}, or CONR^{e}SO₂R^{a};
- R^{a}: is (C₁-C₆)-alkyl or (C₁-C₆)-haloalkyl;
- R^{b}: is hydrogen;
- R^{e}: is hydrogen, (C₁-C₆)-alkyl, or (C₃-C₆)-cycloalkyl, where each of the two last-mentioned radicals is unsubstituted or substituted with m radicals selected from the group consisting of (C₁-C₂)-alkoxy, (C₁-C₃)-alkylthio, (C₁-C₃)-alkylsulfinyl, (C₁-C₃)-alkylsulfonyl, phenylthio, phenylsulfinyl, and phenylsulfonyl;
- R^{h}: is hydrogen, hydroxyl, (C₁-C₆)-alkyl, (C₁-C₂)-alkoxy, (C₃-C₆)-cycloalkyl, (C₂-C₄)-alkenyl, (C₁-C₆)-alkoxycarbonyl-(C₁-C₆)-alkyl, or (C₃-C₄)-alkynyl, where each of the six last-mentioned radicals is unsubstituted or substituted with m radicals selected from the group consisting of (C₁-C₂)-alkyl, (C₁-C₂)-alkoxy and (C₁-C₂)-alkoxy-(C₁-C₂)-alkoxy; or
- R^{b} and R^{h}: form, together with the nitrogen atom to which they are bound, a saturated or partially unsaturated 6-membered heterocycle which contains 0 or 1 oxygen atom as ring member;
- m: is independently of each occurrence 1 or 2;
- n: is 1 or 2.

### Embodiment Q.4: Q represents the radical of formula Z-Y,

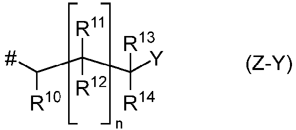

wherein # indicates the bond to the adjacent nitrogen atom, and wherein the substituents have the following meanings, wherein
R¹⁰ is methyl;
R¹¹, R¹², R¹³, R¹⁴ are hydrogen; or
R¹⁰ and R¹⁴ together with the carbon atoms to which they are bound form a four- to six-membered saturated or partially unsaturated carbocycle or a five-membered saturated or partially unsaturated heterocycle containing 1 oxygen atom as ring member;
Y is CO₂R^{e};
R^{e} is hydrogen, (C₁-C₆)-alkyl, or (C₃-C₆)-cycloalkyl, where each of the two last-mentioned radicals is unsubstituted or substituted with m radicals selected from the group consisting of (C₁-C₂)-alkoxy, (C₁-C₃)-alkylthio, (C₁-C₃)-alkylsulfinyl, (C₁-C₃)-alkylsulfonyl, phenylthio, phenylsulfinyl, and phenylsulfonyl;
m is 1 or 2;
n is 1 or 2.

### Embodiment Q.5: Q are the following radicals Z¹-Y and Z²-Y

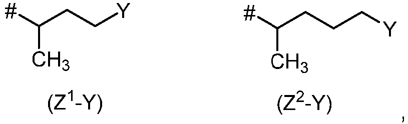

wherein # indicates the bond to the adjacent nitrogen atom, and wherein the substituents have the following meanings, wherein
- Y: is CO₂R^{e}, CONR^{b}R^{h}, or CONR^{e}SO₂R^{a};
- R^{a}: is (C₁-C₆)-alkyl or (C₁-C₆)-haloalkyl;
- R^{b}: is hydrogen;
- R^{e}: is hydrogen, (C₁-C₆)-alkyl, or (C₃-C₆)-cycloalkyl, where each of the two last-mentioned radicals is unsubstituted or substituted with m radicals selected from the group consisting of (C₁-C₂)-alkoxy, (C₁-C₃)-alkylthio, (C₁-C₃)-alkylsulfinyl, (C₁-C₃)-alkylsulfonyl, phenylthio, phenylsulfinyl, and phenylsulfonyl;
- R^{h}: is hydrogen, hydroxyl, (C₁-C₆)-alkyl, (C₁-C₂)-alkoxy, (C₃-C₆)-cycloalkyl, (C₂-C₄)-alkenyl, (C₁-C₆)-alkoxycarbonyl-(C₁-C₆)-alkyl, or (C₃-C₄)-alkynyl, where each of the six last-mentioned radicals is unsubstituted or substituted with m radicals selected from the group consisting of (C₁-C₂)-alkyl, (C₁-C₂)-alkoxy and (C₁-C₂)-alkoxy-(C₁-C₂)-alkoxy; or
- R^{b} and R^{h}: form, together with the nitrogen atom to which they are bound, a saturated 6-membered heterocycle which contains 0 or 1 oxygen atom as ring member;
- m: is independently of each occurrence 1 or 2.

### Embodiment Q.5a: Q is as defined in Q.5, whereas

- Y: is CO₂R^{e};
- R^{e}: is hydrogen, (C₁-C₆)-alkyl, or (C₃-C₆)-cycloalkyl, where each of the two last-mentioned radicals is unsubstituted or substituted with m radicals selected from the group consisting of (C₁-C₂)-alkoxy, (C₁-C₃)-alkylthio, (C₁-C₃)-alkylsulfinyl, (C₁-C₃)-alkylsulfonyl, phenylthio, phenylsulfinyl, and phenylsulfonyl;
- m: is 1 or 2.

### Embodiment Q.5b: Q is as defined in Q.5, whereas

- Y: is CO₂R^{e};
- R^{e}: is hydrogen, (C₁-C₆)-alkyl, or (C₃-C₆)-cycloalkyl.

### Embodiment Q.6: Q representing the radicals Z³-Y to Z⁸-Y:

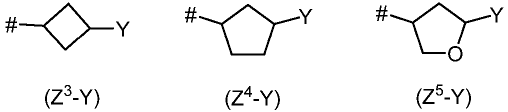

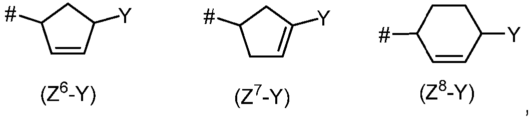

wherein # indicates the bond to the adjacent nitrogen atom, and wherein the substituents have the following meanings
- Y: is CO₂R^{e}, CONR^{b}R^{h}, or CONR^{e}SO₂R^{a};
- R^{a}: is (C₁-C₆)-alkyl or (C₁-C₆)-haloalkyl;
- R^{b}: is hydrogen;
- R^{e}: is hydrogen, (C₁-C₆)-alkyl, or (C₃-C₆)-cycloalkyl, where each of the two last-mentioned radicals is unsubstituted or substituted with m radicals selected from the group consisting of (C₁-C₂)-alkoxy, (C₁-C₃)-alkylthio, (C₁-C₃)-alkylsulfinyl, (C₁-C₃)-alkylsulfonyl, phenylthio, phenylsulfinyl, and phenylsulfonyl;
- R^{h}: is hydrogen, hydroxyl, (C₁-C₆)-alkyl, (C₁-C₂)-alkoxy, (C₃-C₆)-cycloalkyl, (C₂-C₄)-alkenyl, (C₁-C₆)-alkoxycarbonyl-(C₁-C₆)-alkyl, or (C₃-C₄)-alkynyl, where each of the six last-mentioned radicals is unsubstituted or substituted with m radicals selected from the group consisting of (C₁-C₂)-alkyl, (C₁-C₂)-alkoxy and (C₁-C₂)-alkoxy-(C₁-C₂)-alkoxy; or
- R^{b} and R^{h}: form, together with the nitrogen atom to which they are bound, a saturated 6-membered heterocycle which contains 0 or 1 oxygen atom as ring member;
- m: is independently of each occurrence 1 or 2.

### Embodiment Q.6a: Q is as defined in Q.6, whereas

- Y: is CO₂R^{e};
- R^{e}: is hydrogen, (C₁-C₆)-alkyl, or (C₃-C₆)-cycloalkyl, where each of the two last-mentioned radicals is unsubstituted or substituted with m radicals selected from the group consisting of (C₁-C₂)-alkoxy, (C₁-C₃)-alkylthio, (C₁-C₃)-alkylsulfinyl, (C₁-C₃)-alkylsulfonyl, phenylthio, phenylsulfinyl, and phenylsulfonyl;
- m: is 1 or 2.

### Embodiment Q.6b: Q is as defined in Q.6, whereas

- Y: is CO₂R^{e};
- R^{e}: is hydrogen, (C₁-C₆)-alkyl, or (C₃-C₆)-cycloalkyl.

In a further embodiment Q has the meaning represented by the group of radicals Z¹-Y to Z⁸-Y, whereas the other variables are as defined in embodiment Q.5.

In a further embodiment Q has the meaning represented by the group of radicals Z¹-Y to Z⁸-Y, whereas the other variables are as defined in embodiment Q.5a.

In a further embodiment Q has the meaning represented by the group of radicals Z¹-Y to Z⁸-Y, whereas the other variables are as defined in embodiment Q.5b.

Further embodiments H1.1 to H1.128, each defined in one line of Table H1 below, relate to the meaning of the variables of compounds of formula (I). They are defined as combinations of embodiments as defined herein; with the provision that the total number of substituents R^{A1} and R^{A2} on each ring or ring system does not exceed the number of substituents that are theoretically possible; and excluding heteroaromatic rings that bear an electronic charge.

**Table H1**

| | **Ar** | **R^{A1}** | **R^{A2}** | **R¹, R⁷, R⁸, R⁹, X** | **Q** |
|---|---|---|---|---|---|
| H1.1 | Ar.1 | RA1.1 | RA2.1 | S.1 | Q.3 |
| H1.2 | Ar.1 | RA1.2 | RA2.1 | S.1 | Q.3 |
| H1.3 | Ar.1 | RA1.3 | RA2.1 | S.1 | Q.3 |
| H1.4 | Ar.1 | RA1.4 | RA2.1 | S.1 | Q.3 |
| H1.5 | Ar.1 | RA1.1 | RA2.2 | S.1 | Q.3 |
| H1.6 | Ar.1 | RA1.2 | RA2.2 | S.1 | Q.3 |
| H1.7 | Ar.1 | RA1.3 | RA2.2 | S.1 | Q.3 |
| H1.8 | Ar.1 | RA1.4 | RA2.2 | S.1 | Q.3 |
| H1.9 | Ar.1 | RA1.1 | RA2.1 | S.2 | Q.3 |
| H1.10 | Ar.1 | RA1.2 | RA2.1 | S.2 | Q.3 |
| H1.11 | Ar.1 | RA1.3 | RA2.1 | S.2 | Q.3 |
| H1.12 | Ar.1 | RA1.4 | RA2.1 | S.2 | Q.3 |
| H1.13 | Ar.1 | RA1.1 | RA2.2 | S.2 | Q.3 |
| H1.14 | Ar.1 | RA1.2 | RA2.2 | S.2 | Q.3 |
| H1.15 | Ar.1 | RA1.3 | RA2.2 | S.2 | Q.3 |
| H1.16 | Ar.1 | RA1.4 | RA2.2 | S.2 | Q.3 |
| H1.17 | Ar.1 | RA1.1 | RA2.1 | S.1 | Q.4 |
| H1.18 | Ar.1 | RA1.2 | RA2.1 | S.1 | Q.4 |
| H1.19 | Ar.1 | RA1.3 | RA2.1 | S.1 | Q.4 |
| H1.20 | Ar.1 | RA1.4 | RA2.1 | S.1 | Q.4 |
| H1.21 | Ar.1 | RA1.1 | RA2.2 | S.1 | Q.4 |
| H1.22 | Ar.1 | RA1.2 | RA2.2 | S.1 | Q.4 |
| H1.23 | Ar.1 | RA1.3 | RA2.2 | S.1 | Q.4 |
| H1.24 | Ar.1 | RA1.4 | RA2.2 | S.1 | Q.4 |
| H1.25 | Ar.1 | RA1.1 | RA2.1 | S.2 | Q.4 |
| H1.26 | Ar.1 | RA1.2 | RA2.1 | S.2 | Q.4 |
| H1.27 | Ar.1 | RA1.3 | RA2.1 | S.2 | Q.4 |
| H1.28 | Ar.1 | RA1.4 | RA2.1 | S.2 | Q.4 |
| H1.29 | Ar.1 | RA1.1 | RA2.2 | S.2 | Q.4 |
| H1.30 | Ar.1 | RA1.2 | RA2.2 | S.2 | Q.4 |
| H1.31 | Ar.1 | RA1.3 | RA2.2 | S.2 | Q.4 |
| H1.32 | Ar.1 | RA1.4 | RA2.2 | S.2 | Q.4 |
| H1.33 | Ar.1 | RA1.1 | RA2.1 | S.1 | Q.5 |
| H1.34 | Ar.1 | RA1.2 | RA2.1 | S.1 | Q.5 |
| H1.35 | Ar.1 | RA1.3 | RA2.1 | S.1 | Q.5 |
| H1.36 | Ar.1 | RA1.4 | RA2.1 | S.1 | Q.5 |
| H1.37 | Ar.1 | RA1.1 | RA2.2 | S.1 | Q.5 |
| H1.38 | Ar.1 | RA1.2 | RA2.2 | S.1 | Q.5 |
| H1.39 | Ar.1 | RA1.3 | RA2.2 | S.1 | Q.5 |
| H1.40 | Ar.1 | RA1.4 | RA2.2 | S.1 | Q.5 |
| H1.41 | Ar.1 | RA1.1 | RA2.1 | S.2 | Q.5 |
| H1.42 | Ar.1 | RA1.2 | RA2.1 | S.2 | Q.5 |
| H1.43 | Ar.1 | RA1.3 | RA2.1 | S.2 | Q.5 |
| H1.44 | Ar.1 | RA1.4 | RA2.1 | S.2 | Q.5 |
| H1.45 | Ar.1 | RA1.1 | RA2.2 | S.2 | Q.5 |
| H1.46 | Ar.1 | RA1.2 | RA2.2 | S.2 | Q.5 |
| H1.47 | Ar.1 | RA1.3 | RA2.2 | S.2 | Q.5 |
| H1.48 | Ar.1 | RA1.4 | RA2.2 | S.2 | Q.5 |
| H1.49 | Ar.1 | RA1.1 | RA2.1 | S.1 | Q.5a |
| H1.50 | Ar.1 | RA1.2 | RA2.1 | S.1 | Q.5a |
| H1.51 | Ar.1 | RA1.3 | RA2.1 | S.1 | Q.5a |
| H1.52 | Ar.1 | RA1.4 | RA2.1 | S.1 | Q.5a |
| H1.53 | Ar.1 | RA1.1 | RA2.2 | S.1 | Q.5a |
| H1.54 | Ar.1 | RA1.2 | RA2.2 | S.1 | Q.5a |
| H1.55 | Ar.1 | RA1.3 | RA2.2 | S.1 | Q.5a |
| H1.56 | Ar.1 | RA1.4 | RA2.2 | S.1 | Q.5a |
| H1.57 | Ar.1 | RA1.1 | RA2.1 | S.2 | Q.5a |
| H1.58 | Ar.1 | RA1.2 | RA2.1 | S.2 | Q.5a |
| H1.59 | Ar.1 | RA1.3 | RA2.1 | S.2 | Q.5a |
| H1.60 | Ar.1 | RA1.4 | RA2.1 | S.2 | Q.5a |
| H1.61 | Ar.1 | RA1.1 | RA2.2 | S.2 | Q.5a |
| H1.62 | Ar.1 | RA1.2 | RA2.2 | S.2 | Q.5a |
| H1.63 | Ar.1 | RA1.3 | RA2.2 | S.2 | Q.5a |
| H1.64 | Ar.1 | RA1.4 | RA2.2 | S.2 | Q.5a |
| H1.65 | Ar.1 | RA1.1 | RA2.1 | S.1 | Q.5b |
| H1.66 | Ar.1 | RA1.2 | RA2.1 | S.1 | Q.5b |
| H1.67 | Ar.1 | RA1.3 | RA2.1 | S.1 | Q.5b |
| H1.68 | Ar.1 | RA1.4 | RA2.1 | S.1 | Q.5b |
| H1.69 | Ar.1 | RA1.1 | RA2.2 | S.1 | Q.5b |
| H1.70 | Ar.1 | RA1.2 | RA2.2 | S.1 | Q.5b |
| H1.71 | Ar.1 | RA1.3 | RA2.2 | S.1 | Q.5b |
| H1.72 | Ar.1 | RA1.4 | RA2.2 | S.1 | Q.5b |
| H1.73 | Ar.1 | RA1.1 | RA2.1 | S.2 | Q.5b |
| H1.74 | Ar.1 | RA1.2 | RA2.1 | S.2 | Q.5b |
| H1.75 | Ar.1 | RA1.3 | RA2.1 | S.2 | Q.5b |
| H1.76 | Ar.1 | RA1.4 | RA2.1 | S.2 | Q.5b |
| H1.77 | Ar.1 | RA1.1 | RA2.2 | S.2 | Q.5b |
| H1.78 | Ar.1 | RA1.2 | RA2.2 | S.2 | Q.5b |
| H1.79 | Ar.1 | RA1.3 | RA2.2 | S.2 | Q.5b |
| H1.80 | Ar.1 | RA1.4 | RA2.2 | S.2 | Q.5b |
| H1.81 | Ar.1 | RA1.1 | RA2.1 | S.1 | Q.6 |
| H1.82 | Ar.1 | RA1.2 | RA2.1 | S.1 | Q.6 |
| H1.83 | Ar.1 | RA1.3 | RA2.1 | S.1 | Q.6 |
| H1.84 | Ar.1 | RA1.4 | RA2.1 | S.1 | Q.6 |
| H1.85 | Ar.1 | RA1.1 | RA2.2 | S.1 | Q.6 |
| H1.86 | Ar.1 | RA1.2 | RA2.2 | S.1 | Q.6 |
| H1.87 | Ar.1 | RA1.3 | RA2.2 | S.1 | Q.6 |
| H1.88 | Ar.1 | RA1.4 | RA2.2 | S.1 | Q.6 |
| H1.89 | Ar.1 | RA1.1 | RA2.1 | S.2 | Q.6 |
| H1.90 | Ar.1 | RA1.2 | RA2.1 | S.2 | Q.6 |
| H1.91 | Ar.1 | RA1.3 | RA2.1 | S.2 | Q.6 |
| H1.92 | Ar.1 | RA1.4 | RA2.1 | S.2 | Q.6 |
| H1.93 | Ar.1 | RA1.1 | RA2.2 | S.2 | Q.6 |
| H1.94 | Ar.1 | RA1.2 | RA2.2 | S.2 | Q.6 |
| H1.95 | Ar.1 | RA1.3 | RA2.2 | S.2 | Q.6 |
| H1.96 | Ar.1 | RA1.4 | RA2.2 | S.2 | Q.6 |
| H1.97 | Ar.1 | RA1.1 | RA2.1 | S.1 | Q.6a |
| H1.98 | Ar.1 | RA1.2 | RA2.1 | S.1 | Q.6a |
| H1.99 | Ar.1 | RA1.3 | RA2.1 | S.1 | Q.6a |
| H1.100 | Ar.1 | RA1.4 | RA2.1 | S.1 | Q.6a |
| H1.101 | Ar.1 | RA1.1 | RA2.2 | S.1 | Q.6a |
| H1.102 | Ar.1 | RA1.2 | RA2.2 | S.1 | Q.6a |
| H1.103 | Ar.1 | RA1.3 | RA2.2 | S.1 | Q.6a |
| H1.104 | Ar.1 | RA1.4 | RA2.2 | S.1 | Q.6a |
| H1.105 | Ar.1 | RA1.1 | RA2.1 | S.2 | Q.6a |
| H1.106 | Ar.1 | RA1.2 | RA2.1 | S.2 | Q.6a |
| H1.107 | Ar.1 | RA1.3 | RA2.1 | S.2 | Q.6a |
| H1.108 | Ar.1 | RA1.4 | RA2.1 | S.2 | Q.6a |
| H1.109 | Ar.1 | RA1.1 | RA2.2 | S.2 | Q.6a |
| H1.110 | Ar.1 | RA1.2 | RA2.2 | S.2 | Q.6a |
| H1.111 | Ar.1 | RA1.3 | RA2.2 | S.2 | Q.6a |
| H1.112 | Ar.1 | RA1.4 | RA2.2 | S.2 | Q.6a |
| H1.113 | Ar.1 | RA1.1 | RA2.1 | S.1 | Q.6b |
| H1.114 | Ar.1 | RA1.2 | RA2.1 | S.1 | Q.6b |
| H1.115 | Ar.1 | RA1.3 | RA2.1 | S.1 | Q.6b |
| H1.116 | Ar.1 | RA1.4 | RA2.1 | S.1 | Q.6b |
| H1.117 | Ar.1 | RA1.1 | RA2.2 | S.1 | Q.6b |
| H1.118 | Ar.1 | RA1.2 | RA2.2 | S.1 | Q.6b |
| H1.119 | Ar.1 | RA1.3 | RA2.2 | S.1 | Q.6b |
| H1.120 | Ar.1 | RA1.4 | RA2.2 | S.1 | Q.6b |
| H1.121 | Ar.1 | RA1.1 | RA2.1 | S.2 | Q.6b |
| H1.122 | Ar.1 | RA1.2 | RA2.1 | S.2 | Q.6b |
| H1.123 | Ar.1 | RA1.3 | RA2.1 | S.2 | Q.6b |
| H1.124 | Ar.1 | RA1.4 | RA2.1 | S.2 | Q.6b |
| H1.125 | Ar.1 | RA1.1 | RA2.2 | S.2 | Q.6b |
| H1.126 | Ar.1 | RA1.2 | RA2.2 | S.2 | Q.6b |
| H1.127 | Ar.1 | RA1.3 | RA2.2 | S.2 | Q.6b |
| H1.128 | Ar.1 | RA1.4 | RA2.2 | S.2 | Q.6b |

Further embodiments H2.1-H2.128 of the invention differ from embodiments H1.1-H1.128 of Table H1 only in that the meaning of variable Ar is each defined as in embodiment Ar.2.

Accordingly, for example, embodiment H2.80 discloses the combination of embodiment Ar.2 with embodiments RA1.1, RA2.2, S.2 and B.5b, i.e. the combination of embodiments other than embodiment Ar as defined for H1.80.

Likewise, further embodiments H3.1-H3.128 of the invention differ from embodiments H1.1-H1.128 of Table H1 only in that the meaning of variable Ar is each defined as in embodiment Ar.3.

Further embodiments H4.1-H4.128 of the invention differ from embodiments H1.1-H1.128 of Table H1 only in that the meaning of variable Ar is each defined as in embodiment Ar.4.

Further embodiments H5.1-H5.128 of the invention differ from embodiments H1.1-H1.128 of Table H1 only in that the meaning of variable Ar is each defined as in embodiment Ar.5.

Further embodiments H6.1-H6.128 of the invention differ from embodiments H1.1-H1.128 of Table H1 only in that the meaning of variable Ar is each defined as in embodiment Ar.6.

Further embodiments H7.1-H7.128 of the invention differ from embodiments H1.1-H1.128 of Table H1 only in that the meaning of variable Ar is each defined as in embodiment Ar.7 .

Further embodiments H8.1-H8.128 of the invention differ from embodiments H1.1-H1.128 of Table H1 only in that the meaning of variable Ar is each defined as in embodiment Ar.8.

Further embodiments H9.1-H9.128 of the invention differ from embodiments H1.1-H1.128 of Table H1 only in that the meaning of variable Ar is each defined as a combination of embodiments Ar.1 and Ar.5.

Accordingly, for example, embodiment H9.80 discloses the combination of embodiments Ar.1 and Ar.5 with embodiments RA1.1, RA2.2, S.2 and B.5b, i.e. the combination of embodiments other than embodiment Ar as defined for H1.80.

Likewise, further embodiments H10.1-H10.128 of the invention differ from embodiments H1.1-H1.128 of Table H1 only in that the meaning of variable Ar is each defined as a combination of embodiments Ar.1 and Ar.6.

Further embodiments H11.1-H11.128 of the invention differ from embodiments H1.1-H1.128 of Table H1 only in that the meaning of variable Ar is each defined as a combination of embodiments Ar.1 and Ar.7 .

Further embodiments H12.1-H12.128 of the invention differ from embodiments H1.1-H1.128 of Table H1 only in that the meaning of variable Ar is each defined as a combination of embodiments Ar.1 and Ar.8.

Further embodiments H13.1-H13.128 of the invention differ from embodiments H1.1-H1.128 of Table H1 only in that the meaning of variable Ar is each defined as a combination of embodiments Ar.2 and Ar.5.

Further embodiments H14.1-H14.128 of the invention differ from embodiments H1.1-H1.128 of Table H1 only in that the meaning of variable Ar is each defined as a combination of embodiments Ar.2 and Ar.6.

Further embodiments H15.1-H15.128 of the invention differ from embodiments H1.1-H1.128 of Table H1 only in that the meaning of variable Ar is each defined as a combination of embodiments Ar.2 and Ar.7.

Further embodiments H16.1-H16.128 of the invention differ from embodiments H1.1-H1.128 of Table H1 only in that the meaning of variable Ar is each defined as a combination of embodiments Ar.2 and Ar.8.

Further embodiments H17.1-H17.128 of the invention differ from embodiments H1.1-H1.128 of Table H1 only in that the meaning of variable Ar is each defined as a combination of embodiments Ar.3 and Ar.5.

Further embodiments H18.1-H18.128 of the invention differ from embodiments H1.1-H1.128 of Table H1 only in that the meaning of variable Ar is each defined as a combination of embodiments Ar.3 and Ar.6.

Further embodiments H19.1-H 19.128 of the invention differ from embodiments H1.1-H1.128 of Table H1 only in that the meaning of variable Ar is each defined as a combination of embodiments Ar.3 and Ar.7.

Further embodiments H20.1-H20.128 of the invention differ from embodiments H1.1-H1.128 of Table H1 only in that the meaning of variable Ar is each defined as a combination of embodiments Ar.3 and Ar.8.

Further embodiments H21.1-H21.128 of the invention differ from embodiments H1.1-H1.128 of Table H1 only in that the meaning of variable Ar is each defined as a combination of embodiments Ar.4 and Ar.5.

Further embodiments H22.1-H22.128 of the invention differ from embodiments H1.1-H1.128 of Table H1 only in that the meaning of variable Ar is each defined as a combination of embodiments Ar.4 and Ar.6.

Further embodiments H23.1-H23.128 of the invention differ from embodiments H1.1-H1.128 of Table H1 only in that the meaning of variable Ar is each defined as a combination of embodiments Ar.4 and Ar.7.

Further embodiments H24.1-H24.128 of the invention differ from embodiments H1.1-H1.128 of Table H1 only in that the meaning of variable Ar is each defined as a combination of embodiments Ar.4 and Ar.8.

The invention also relates to agrochemical formulations comprising a compound of formula (I) and one or more auxiliaries, which are customary for formulating crop protection compounds. An agrochemical formulation comprises a pesticidally effective amount of a compound of formula (I). The term "effective amount" denotes an amount of the combination or of the compound of formula (I), which is sufficient for controlling undesired vegetation, especially for controlling undesired vegetation in crops (i.e. cultivated plants) and which does not result in a substantial damage to the treated crop plants. Such amount can vary in a broad range and is dependent on various factors, such as the undesired vegetation to be controlled, the treated crop plants or material, the climatic conditions and the specific compound of formula (I) used.

The compounds of formula (I) and their salts can be converted into customary types of agrochemical formulations, e. g. solutions, emulsions, suspensions, dusts, powders, pastes, granules, pressings, capsules, and mixtures thereof. Examples for agrochemical formulation types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials such as seeds (e.g. GF). These and further agrochemical formulation types are defined in the "Catalogue of pesticide formulation types and international coding system", Technical Monograph No. 2, 6th Ed. May 2008, CropLife International.

The agrochemical formulations are prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005.

Suitable auxiliaries are solvents, liquid carriers, solid carriers or fillers, surfactants, dispersants, emulsifiers, wetting agents, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, such as mineral oil fractions of medium to high boiling point, e.g. kerosene, diesel oil; oils of vegetable or animal origin; aliphatic, cyclic and aromatic hydrocarbons, e. g. toluene, paraffin, tetrahydronaphthalene, alkylated naphthalenes; alcohols, e.g. ethanol, propanol, butanol, benzylalcohol, cyclohexanol; glycols; DMSO; ketones, e.g. cyclohexanone; esters, e.g. lactates, carbonates, fatty acid esters, gamma-butyrolactone; fatty acids; phosphonates; amines; amides, e.g. N-methylpyrrolidone, fatty acid dimethylamides; and mixtures thereof.

Suitable solid carriers or fillers are mineral earths, e.g. silicates, silica gels, talc, kaolins, limestone, lime, chalk, clays, dolomite, diatomaceous earth, bentonite, calcium sulfate, magnesium sulfate, magnesium oxide; polysaccharides, e.g. cellulose, starch; fertilizers, e.g. ammonium sulfate, ammonium phosphate, ammonium nitrate, ureas; products of vegetable origin, e.g. cereal meal, tree bark meal, wood meal, nutshell meal, and mixtures thereof.

Suitable surfactants are surface-active compounds, such as anionic, cationic, nonionic and amphoteric surfactants, block polymers, polyelectrolytes, and mixtures thereof. Such surfactants can be used as emulsifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).

Suitable anionic surfactants are alkali, alkaline earth or ammonium salts of sulfonates, sulfates, phosphates, carboxylates, and mixtures thereof. Examples of sulfonates are alkylarylsulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-substituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-substituted fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are home- or copolymers of vinylpyrrolidone, vinylalcohols, or vinylacetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinylamines or polyethyleneamines.

Suitable adjuvants are compounds, which have a neglectable or even no pesticidal activity themselves, and which improve the biological performance of the compounds of formula (I) on the target. Examples are surfactants, mineral or vegetable oils, and other auxiliaries. Further examples are listed by Knowles, Adjuvants and additives, Agrow Reports DS256, T&F Informa UK, 2006, chapter 5.

Suitable thickeners are polysaccharides (e.g. xanthan gum, carboxymethylcellulose), inorganic clays (organically modified or unmodified), polycarboxylates, and silicates.

Suitable bactericides are bronopol and isothiazolinone derivatives such as alkylisothiazolinones and benzisothiazolinones.

Suitable anti-freezing agents are ethylene glycol, propylene glycol, urea and glycerin.

Suitable anti-foaming agents are silicones, long chain alcohols, and salts of fatty acids.

Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo- and phthalocyanine colorants).

Suitable tackifiers or binders are polyvinylpyrrolidons, polyvinyl acetates, polyvinyl alcohols, polyacrylates, biological or synthetic waxes, and cellulose ethers.

Examples for agrochemical formulation types and their preparation are:
i) Water-soluble concentrates (SL, LS)
   10-60 wt% of a compound of formula (I) or a combination comprising at least one compound of formula (I) (component A) according to the invention and 5-15 wt% wetting agent (e.g. alcohol alkoxylates) are dissolved in water and/or in a water-soluble solvent (e.g. alcohols) ad 100 wt%. The active substance dissolves upon dilution with water.
ii) Dispersible concentrates (DC)
   5-25 wt% of a compound of formula (I) or a combination comprising at least one compound of formula (I) (component A) according to the invention and 1-10 wt% dispersant (e. g. polyvinylpyrrolidone) are dissolved in organic solvent (e.g. cyclohexanone) ad 100 wt%. Dilution with water gives a dispersion.
iii) Emulsifiable concentrates (EC) 15-70 wt% of compound of formula (I) or a combination comprising at least one compound of formula (I) (component A) according to the invention and 5-10 wt% emulsifiers (e.g. calcium dodecylbenzenesulfonate and castor oil ethoxylate) are dissolved in water-insoluble organic solvent (e.g. aromatic hydrocarbon) ad 100 wt%. Dilution with water gives an emulsion.
iv) Emulsions (EW, EC, ES)
   5-40 wt% of compound of formula (I) or a combination comprising at least one compound of formula (I) (component A) according to the invention and 1-10 wt% emulsifiers (e.g. calcium dodecylbenzenesulfonate and castor oil ethoxylate) are dissolved in 20-40 wt% water-insoluble organic solvent (e.g. aromatic hydrocarbon). This mixture is introduced into water ad 100 wt% by means of an emulsifying machine and made into a homogeneous emulsion. Dilution with water gives an emulsion.
v) Suspensions (SC, CD, FS)
   In an agitated ball mill, 20-60 wt% of a compound of formula (I) or a combination comprising at least one compound of formula (I) (component A) according to the invention are comminuted with addition of 2-10 wt% dispersants and wetting agents (e.g. sodium lignosulfonate and alcohol ethoxylate), 0,1-2 wt% thickener (e.g. xanthan gum) and water ad 100 wt% to give a fine active substance suspension. Dilution with water gives a stable suspension of the active substance. For FS type formulation up to 40 wt% binder (e.g. polyvinylalcohol) is added.
vi) Water-dispersible granules and water-soluble granules (WG, SG)
   50-80 wt% of a compound of formula (I) or a combination comprising at least one compound of formula (I) (component A) according to the invention are ground finely with addition of dispersants and wetting agents (e.g. sodium lignosulfonate and alcohol ethoxylate) ad 100 wt% and prepared as water-dispersible or water-soluble granules by means of technical appliances (e. g. extrusion, spray tower, fluidized bed). Dilution with water gives a stable dispersion or solution of the active substance.
vii) Water-dispersible powders and water-soluble powders (WP, SP, WS)
   50-80 wt% of a compound of formula (I) or a combination comprising at least one compound of formula (I) (component A) according to the invention are ground in a rotor-stator mill with addition of 1-5 wt% dispersants (e.g. sodium lignosulfonate), 1-3 wt% wetting agents (e.g. alcohol ethoxylate) and solid carrier (e.g. silica gel) ad 100 wt%. Dilution with water gives a stable dispersion or solution of the active substance.
viii) Gel (GW, GF)
   In an agitated ball mill, 5-25 wt% of a compound of formula (I) or a combination comprising at least one compound of formula (I) (component A) according to the invention are comminuted with addition of 3-10 wt% dispersants (e.g. sodium lignosulfonate), 1-5 wt% thickener (e.g. carboxymethylcellulose) and water ad 100 wt% to give a fine suspension of the active substance. Dilution with water gives a stable suspension of the active substance.
iv) Microemulsion (ME)
   5-20 wt% of a compound of formula (I) or a combination comprising at least one compound of formula (I) (component A) according to the invention are added to 5-30 wt% organic solvent blend (e.g. fatty acid dimethylamide and cyclohexanone), 10-25 wt% surfactant blend (e.g. alcohol ethoxylate and arylphenol ethoxylate), and water ad 100 %. This mixture is stirred for 1 h to produce spontaneously a thermodynamically stable microemulsion.
iv) Microcapsules (CS)
   An oil phase comprising 5-50 wt% of a compound of formula (I) or a combination comprising at least one compound of formula (I) (component A) according to the invention, 0-40 wt% water insoluble organic solvent (e.g. aromatic hydrocarbon), 2-15 wt% acrylic monomers (e.g. methylmethacrylate, methacrylic acid and a di- or triacrylate) are dispersed into an aqueous solution of a protective colloid (e.g. polyvinyl alcohol). Radical polymerization initiated by a radical initiator results in the formation of poly(meth)acrylate microcapsules. Alternatively, an oil phase comprising 5-50 wt% of a compound of formula (I) according to the invention, 0-40 wt% water insoluble organic solvent (e.g. aromatic hydrocarbon), and an isocyanate monomer (e.g. diphenylmethene-4,4'-diisocyanate) are dispersed into an aqueous solution of a protective colloid (e.g. polyvinyl alcohol). The addition of a polyamine (e.g. hexamethylenediamine) results in the formation of polyurea microcapsules. The monomers amount to 1-10 wt%. The wt% relate to the total CS formulation.
ix) Dustable powders (DP, DS)
   1-10 wt% of a compound of formula (I) or a combination comprising at least one compound of formula (I) (component A) according to the invention are ground finely and mixed intimately with solid carrier (e.g. finely divided kaolin) ad 100 wt%.
x) Granules (GR, FG)
   0.5-30 wt% of a compound of formula (I) or a combination comprising at least one compound of formula (I) (component A) according to the invention is ground finely and associated with solid carrier (e.g. silicate) ad 100 wt%. Granulation is achieved by extrusion, spray-drying or the fluidized bed.
xi) Ultra-low volume liquids (UL)
   1-50 wt% of a compound of formula (I) or a combination comprising at least one compound of formula (I) (component A) according to the invention are dissolved in organic solvent (e.g. aromatic hydrocarbon) ad 100 wt%.

The agrochemical formulation types i) to xi) may optionally comprise further auxiliaries, such as 0,1-1 wt% bactericides, 5-15 wt% anti-freezing agents, 0,1-1 wt% anti-foaming agents, and 0,1-1 wt% colorants.

The agrochemical formulations and/or combinations generally comprise between 0.01 and 95%, preferably between 0.1 and 90%, and in particular between 0.5 and 75%, by weight of the compounds of formula (I).

The compounds of formula (I) are employed in a purity of from 90% to 100%, preferably from 95% to 100% (according to NMR spectrum).

Solutions for seed treatment (LS), suspoemulsions (SE), flowable concentrates (FS), powders for dry treatment (DS), water-dispersible powders for slurry treatment (WS), water-soluble powders (SS), emulsions (ES), emulsifiable concentrates (EC) and gels (GF) are usually employed for the purposes of treatment of plant propagation materials, particularly seeds. The agrochemical formulations in question give, after two-to-tenfold dilution, active substance concentrations of from 0.01 to 60% by weight, preferably from 0.1 to 40% by weight, in the ready-to-use preparations.

Methods for applying compounds of formula (I), agrochemical formulations and /or combinations thereof, on to plant propagation material, especially seeds, include dressing, coating, pelleting, dusting, soaking and in-furrow application methods of the propagation material. Preferably, compounds of formula (I), agrochemical formulations and /or combinations thereof, respectively, are applied on to the plant propagation material by a method such that germination is not induced, e. g. by seed dressing, pelleting, coating and dusting.

Various types of oils, wetting agents, adjuvants, fertilizer, or micronutrients may be added to the compounds of formula (I), the agrochemical formulations and/or the combinations comprising them as premix or, if appropriate not until immediately prior to use (tank mix). These agents can be admixed with the agrochemical formulations according to the invention in a weight ratio of 1:100 to 100:1, preferably 1:10 to 10:1.

The user applies the compounds of formula (I) according to the invention, the agrochemical formulations and/or the combinations comprising them usually from a pre-dosage device, a knapsack sprayer, a spray tank, a spray plane, or an irrigation system. Usually, the agrochemical formulation is made up with water, buffer, and/or further auxiliaries to the desired application concentration and the ready-to-use spray liquor or the agrochemical formulation according to the invention is thus obtained. Usually, 20 to 2000 liters, preferably 50 to 400 liters, of the ready-to-use spray liquor are applied per hectare of agricultural useful area.

The compounds of formula (I) are suitable as herbicides. The compounds of formula (I), or the agrochemical formulations comprising the compounds of formula (I), control undesired vegetation on non-crop areas very efficiently, especially at high rates of application. They act against broad-leaved weeds and grass weeds, particularly in crops such as wheat, rice, maize, soya and cotton without causing any significant damage to the crop plants. This effect is mainly observed at low rates of application.

The compounds of formula (I) have an outstanding herbicidal activity against undesired vegetation, i.e. against a broad spectrum of economically important harmful monocotyledonous and dicotyledonous weeds.

According to one embodiment, the compounds of formula (I) are used to control monocotyledonous weeds, such as weeds from the genera *Alopecurus spp., Hordeum* spp., *Echinochloa* spp., Poa spp., *Bromus* spp., *Digitaria* spp., *Eriochloa* spp., *Setaria* spp., *Pennisetum* spp., *Eleusine* spp., *Eragrostís* spp., *Panicum* spp., *Lolium* spp., *Brachiaria* spp., *Leptochloa* spp., *Avena* spp., *Cyperus* spp., *Axonopris* spp., *Sorghum* spp., and *Melinis* spp.

Preferred examples of monocotyledonous weeds on which the compounds of formula (I) act efficiently are selected from the species *Alopecurus myosuroides, Hordeum murinum, Echinochloa crusgalli, Poa annua, Bromus rubens L., Bromus rigidus, Bromus secalinus L., Digitaria sanguinalis, Digitaria insularis, Eriochloa gracilis, Setaria faberi, Setaria viridis, Pennisetum glaucum, Eleusine indica, Eragrostis pectinacea, Panicum miliaceum, Lolium multiflorum, Brachiaria platyphylla, Leptochloa fusca, Avena fatua, Cyperus compressus, Cyperus esculentes, Cyperus rotundus, Axonopris offinis, Sorghum halapense,* and *Melinis repens.*

Especially preferred examples of monocotyledonous weeds on which compounds I or compositions comprising compounds I act efficiently are selected from the species *Alopecurus spp., Echinochloa* spp., *Digitaria* spp., *Setaria* spp., *Eleusine* spp. and *Brachiarium* spp.

According to another embodiment, the the compounds of formula (I) are used to control dicotyledonous weeds, such as weeds selected from the genera *Amaranthus* spp., *Erigeron* spp., *Conyza* spp., *Polygonum* spp., *Medicago* spp., *Mollugo* spp., *Cyclospermum* spp., *Stellaria* spp., *Gnaphalium* spp., *Taraxacum* spp., *Oenothera* spp., *Amsinckia* spp., *Erodium* spp., *Erigeron* spp., *Senecio* spp., *Lamium* spp., *Kochia* spp., *Chenopodium* spp., *Lactuca* spp., *Malva* spp., *Ipomoea* spp., *Brassica* spp., *Sinapis* spp., *Urtica* spp., *Sida spp, Portulaca* spp., *Richardia* spp., *Ambrosia* spp., *Calandrinia* spp., *Sisymbrium* spp., *Sesbania* spp., *Capsella* spp., *Sonchus* spp., *Euphorbia* spp., *Helianthus* spp., *Coronopus* spp., *Salsola* spp., *Abutilon* spp., *Vicia* spp., *Epilobium* spp., *Cardamine* spp., *Picris* spp., *Trifoli-um* spp., *Galinsoga* spp., *Epimedium* spp., *Marchantia* spp., *Solanum* spp., *Oxalis* spp., *Metricar-ia* spp., *Plantago* spp., *Tribulus* spp., *Cenchrus spp. Bidens* spp., *Veronica* spp., *and Hypochaeris* spp.

Preferred examples of dicotyledonous weeds on which the compounds of formula (I) act efficiently are selected from the species *Amaranthus spinosus, A. retroflexus, A. palmeri, A. hybridus, A. viridis, Polygonum convolvulus, Medicago polymorpha, Mollugo verticillata, Cyclospermum leptophyllum, Stellaria media, Gnaphalium purpureum, Taraxacum officinale, Oenothera laciniata, Amsinckia intermedia, Erodium cicutarium, Erodium moschatum, Erigeron bonariensis (Conyza bonariensis),* C. *canadensis,* C. *sumafrensis, Senecio vulgaris, Lamium amplexicaule, Erigeron canadensis, Polygonum aviculare, Kochia scoparia, Chenopodium album, Lactuca serriola, Malva parviflora, Malva neglecta, Ipomoea hederacea, Ipomoea lacunose, Brassica nigra, Sinapis arvensis, Urtica dioica, Amaranthus blitoides, Amaranthus retroflexus, Amaranthus hybridus, Amaranthus lividus, Sida spinosa, Portulaca oleracea, Richardia scabra, Ambrosia artemisiifolia, Calandrinia caulescens, Sisymbrium irio, Sesbania exaltata, Capsella bursapastoris, Sonchus oleraceus, Euphorbia maculate,* Euphorbia heterophylla, *Bidens pilosa, Spermacoce verticillata, Helianthus annuus, Coronopus didymus, Salsola tragus, Abutilon theophrasti, Vicia benghalensis L., Epilobium paniculatum, Cardamine spp, Picris echioides, Trifolium* spp., *Ga-linsoga* spp., *Epimedium* spp., *Marchantia* spp., *Solanum* spp., *Oxalis* spp., *Metricaria matriccari-oides, Plantago* spp., *Tribulus terrestris, Salsola kali, Cenchrus* spp., *Bidens bipinnata, Veronica* spp., and *Hypochaeris radicata.*

Especially preferred examples of dicotyledonous weeds on which the compounds I or compositions comprising compounds I act efficiently are selected from the species *Amaranthus* spp., *Erigeron* spp., *Conyza* spp., *Kochia* spp. and *Abutilon* spp.

Especially preferred examples of dicotyledonous weeds on which the compounds of formula (I) act efficiently are selected from the species *Amaranthus* spp., *Erigeron* spp., *Conyza* spp., *Kochia* spp. and *Abutilon* spp.

The compounds of the invention are particularly useful for controlling for example following weeds:
*Alopercurus myosuroides* (ALOMY), *Amaranthus retroflexus* (AMARE), *Apera spica-venti* (APESV), *Avena fatua* (AVEFA), *Chenopodium album* (CHEAL), *Digitaria sanguinalis* (CHEAL), *Echinocloa crus-galli* (ECHCG), *Lolium multiflorum* (LOLMU), *Setaria faberi* (SETFA), *Setaria viridis* (SETVI).

The compounds of formula (I), or the agrochemical formulations comprising them, are applied to the plants mainly by spraying the leaves. Here, the application can be carried out using, for example, water as carrier by customary spraying techniques using spray liquor amounts of from about 100 to 1000 l/ha (for example from 300 to 400 l/ha). The compounds of formula (I), or the agrochemical formulations and/or the combinations comprising them, may also be applied by the low-volume or the ultra-low-volume method, or in the form of microgranules.

Application of the compounds of formula (I), or the agrochemical formulations comprising them, can be done before, during and/or after, preferably during and/or after, the emergence of the undesired vegetation.

Application of the compounds of formula (I), or the agrochemical formulations can be carried out before or during sowing.

The compounds of formula (I), or the agrochemical formulations comprising them, can be applied pre-, post-emergence or pre-plant, or together with the seed of a crop plant. It is also possible to apply the compounds of formula (I), or the agrochemical formulations, by applying seed, pretreated with the compounds of formula (I), or the agrochemical formulations, of a crop plant. If the active ingredients are less well tolerated by certain crop plants, application techniques may be used in which the agrochemical formulations are sprayed, with the aid of the spraying equipment, in such a way that as far as possible they do not come into contact with the leaves of the sensitive crop plants, while the active ingredients reach the leaves of undesired vegetation growing underneath, or the bare soil surface (post-directed, lay-by).

In a further embodiment, the compounds of formula (I), or the agrochemical formulations comprising them, can be applied by treating seed. The treatment of seeds comprises essentially all procedures familiar to the person skilled in the art (seed dressing, seed coating, seed dusting, seed soaking, seed film coating, seed multilayer coating, seed encrusting, seed dripping and seed pelleting) based on the compounds of formula (I), or the agrochemical formulations prepared therefrom.

The term "seed" comprises seed of all types, such as, for example, corns, seeds, fruits, tubers, seedlings and similar forms. Here, preferably, the term seed describes corns and seeds. The seed used can be seed of the crop plants mentioned above, but also the seed of transgenic plants or plants obtained by customary breeding methods.

When employed in plant protection, the amounts of active substances applied, i.e. the compounds of formula (I), and, if appropriate, component C without formulation auxiliaries, are, depending on the kind of effect desired, from 0.001 to 2 kg per ha, preferably from 0.005 to 2 kg per ha, more preferably from 0.05 to 0.9 kg per ha and in particular from 0.1 to 0.75 kg per ha.

In another embodiment of the invention, the application rate of the compounds of formula (I), and, if appropriate, component C, is from 0.001 to 3 kg/ha, preferably from 0.005 to 2.5 kg/ha and in particular from 0.01 to 2 kg/ha of active substance (a.s.).

In another embodiment of the invention, the rates of application of the compounds of formula (I) according to the present invention (total amount of compounds of formula (I)) are from 0.1 g/ha to 3000 g/ha, preferably 10 g/ha to 1000 g/ha, depending on the control target, the season, the target plants and the growth stage.

In another embodiment of the invention, the application rates of the compounds of formula (I) are in the range from 0.1 g/ha to 5000 g/ha and preferably in the range from 1 g/ha to 2500 g/ha or from 5 g/ha to 2000 g/ha.

In another embodiment of the invention, the application rate of the compounds of formula (I) is 0.1 to 1000 g/ha, preferably1 to 750 g/ha, more preferably 5 to 500 g/ha.

In treatment of plant propagation materials such as seeds, e. g. by dusting, coating or drenching seed, amounts of active substance of from 0.1 to 1000 g, preferably from 1 to 1000 g, more preferably from 1 to 100 g and most preferably from 5 to 100 g, per 100 kilogram of plant propagation material (preferably seeds) are generally required.

In another embodiment of the invention, to treat the seed, the amounts of active substances applied, i.e. the compounds of formula (I), and, if appropriate, component C are generally employed in amounts of from 0.001 to 10 kg per 100 kg of seed.

When used in the protection of materials or stored products, the amount of active substance applied depends on the kind of application area and on the desired effect. Amounts customarily applied in the protection of materials are 0.001 g to 2 kg, preferably 0.005 g to 1 kg of active substance per cubic meter of treated material.

In case of combinations according to the present invention it is immaterial whether the compounds of formula (I), and/or the component C are formulated and applied jointly or separately.

In the case of separate application, it is of minor importance, in which order the application takes place. It is only necessary, that the compounds of formula (I), and/or the component C are applied in a time frame that allows simultaneous action of the active ingredients on the plants, preferably within a time-frame of at most 14 days, in particular at most 7 days.

The compounds (I) and the agrochemical formulations containing them may be applied in combination with, or by utilizing smart agricultural technologies, such as precision agriculture, remote and proximate imaging and image recognition, or smart agricultural site management programs. Such technologies typically include models, e.g. computer programs, that support the user by considering information from a variety of sources to increase quality and yield of harvested material, reduce damage by pests including the prediction of pest pressure and smart application of crop protection products, secure environmental protection, support quick and reliable agronomic decision making, reduce usage of fertilizers and crop protection products, reduce product residues in consumables, increase spatial and temporal precision of agronomical measures, automate processes, and enable traceability of measures. Commercially available systems which include agronomic models are e.g. FieldScripts^{™} (The Climate Corporation), Xarvio^{™} (BASF) and AGLogic^{™} (John Deere).

Information input for these models includes, but is not limited to, soil data, information on: the plants that are currently growing or that may grow at the area of interest including crops and/or unwanted vegetation, weather, the location of the area and directly derivable information thereof, pest pressure, beneficial organisms; comprising forecast, present and / or historic information of any of the aforementioned.

The information usable for precision agriculture may be based on input by at least one user, be accessible from external data sources and databases, or be based on sensor data. Data sources typically include proximate-detection systems like soil-borne sensors and remote sensing as may be achieved by imaging with unmanned airborne vehicles like drones, or satellites. Sensors may be included in an Internet-of-Things system and may be directly or indirectly connected to the processing unit, e.g. via a wireless network and/or cloud applications. The information is typically taken into account by at least one processing unit and used to provide recommendations, and to generate control signals.

Typical technologies that are used in smart agricultural technologies include self-steering ro-bots (such as tractors, harvesters, drones), artificial intelligence (e.g. machine learning), imaging technologies (e.g. image segmentation technologies), big data analysis, model generation, cloud computing, and machine-to-machine communication. Precision agriculture or farming is characterized by spatially and/or temporally resolved, targeted application of active ingredients like pesticides, plant-growth-regulators, fertilizers, and/or water including the variation of application rates over the agronomic site, zone or spot application, and of the spatially and/or temporally resolved, targeted planting or seeding of desired plant propagation material to an agronomic site. Precision farming typically includes the use of geo-positioning technologies like GPS for gaining information on the location and boundaries of the area of interest, the utilized application equipment, sensing equipment and recorded data, and to control the actions of farm vehicles such as spraying. By combining geo-positioning data with (digital) maps, it is possible to (semi)-automate agricultural measures at the site of interest, e.g. by using (semi)-autonomous spraying or seeding equipment. Precision farming typically includes the application of smart spraying equipment, e.g. spot spraying, and precision spraying, e.g. by irrigation systems, tractors, robots, helicopters, airplanes, unmanned aerial vehicles, such as drones. Such equipment usually includes input sensors (e.g. camera) and a processing unit configured to analyze the input data and configured to provide a recommendation or decision based on the analysis of input data to apply the compounds I or compositions comprising them to the agronomic site, e.g. the soil, the crop plants, or to control pests in a specific and precise manner. Pests may be detected, identified, and/or classified from imagery acquired by a camera. Such identification and classification can make use of image processing algorithms, which may utilize artificial intelligence (e.g. machine learning algorithms), or decision trees. In this manner, the compounds I or compositions described herein can be applied only at the required location, point in time and dose rate.

Depending on the application method in question, the compounds according to the invention can additionally be employed in a further number of crop plants for eliminating undesirable plants.

According to the invention all of the above cultivated plants are understood to comprise all species, subspecies, variants, varieties and/or hybrids which belong to the respective cultivated plants, including but not limited to winter and spring varieties, in particular in cereals such as wheat and barley, as well as oilseed rape, e.g. winter wheat, spring wheat, winter barley etc, further including dwarf, semi-dwarf and full-dwarf varieties and/or hybrids with reduced height and thicker and shorter stems, e.g. short stature corn (also called 'smart corn'), semi-dwarf wheat and dwarf rice.

For example, corn is also known as Indian corn or maize (Zea mays) which comprises all kinds of corn such as field corn and sweet corn. According to the invention all maize or corn subspecies and/or varieties are comprised, in particular flour corn (Zea mays var. amylacea), popcorn (Zea mays var. everta), dent corn (Zea mays var. indentata), flint corn (Zea mays var. indurata), sweet corn (Zea mays var. saccharata and var. rugosa), waxy corn (Zea mays var. ceratina), amylomaize (high amylose Zea mays varieties), pod corn or wild maize (Zea mays var. tunicata) and striped maize (Zea mays var. japonica).

Further, most soybean cultivars are classifiable into indeterminate and determinate growth habit, whereas Glycine soja, the wild progenitor of soybean, is indeterminate (PNAS 2010, 107 (19) 8563-856). The indeterminate growth habit (Maturity Group, MG 00 to MG 4.9) is characterized by a continuation of vegetative growth after flowering begins whereas determinate soybean varieties (Maturity Group, (MG) 5 to MG 8) characteristically have finished most of their vegetative growth when flowering begins. According to the invention all soybean cultivars or varieties are comprised, in particular indeterminate and determinate cultivars or varieties.

Examples of suitable crops are the following:
Allium cepa, Ananas comosus, Arachis hypogaea, Asparagus officinalis, Avena sativa, Beta vulgaris spec. altissima, Beta vulgaris spec. rapa, Brassica napus var. napus, Brassica napus var. napobrassica, Brassica rapa var. silvestris, Brassica oleracea, Brassica nigra, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pistacia vera, Pisum sativum, Prunus avium, Prunus persica, Pyrus communis, Prunus armeniaca, Prunus cerasus, Prunus dulcis and prunus domestica, Ribes sylvestre, Ricinus communis, Saccharum officinarum, Secale cereale, Sinapis alba, Solanum tuberosum, Sorghum bicolor (s. vulgare), Theobroma cacao, Trifolium pratense, Triticum aestivum, Triticale, Triticum durum, Vicia faba, Vitis vinifera, Zea mays.

Preferred crops are Arachis hypogaea, Beta vulgaris spec. altissima, Brassica napus var. napus, Brassica oleracea, Citrus limon, Citrus sinensis, Coffea arabica (Coffea canephora, Coffea liberica), Cynodon dactylon, Glycine max, Gossypium hirsutum, (Gossypium arboreum, Gossypium herbaceum, Gossypium vitifolium), Helianthus annuus, Hordeum vulgare, Juglans regia, Lens culinaris, Linum usitatissimum, Lycopersicon lycopersicum, Malus spec., Medicago sativa, Nicotiana tabacum (N.rustica), Olea europaea, Oryza sativa, Phaseolus lunatus, Phaseolus vulgaris, Pistacia vera, Pisum sativum, Prunus dulcis, Saccharum officinarum, Secale cereale, Solanum tuberosum, Sorghum bicolor (s. vulgare), Triticale, Triticum aestivum, Triticum durum, Vicia faba, Vitis vinifera and Zea mays

Especially preferred crops are crops of cereals, corn, soybeans, rice, millets, oilseed rape, cotton, sugarcane, potatoes, legumes, turf or permanent crops.

The compositions according to the invention can also be used in crops which have been modified by mutagenesis or genetic engineering to provide a new trait to a plant or to modify an already present trait.

The term "crops" as used herein includes also (crop) plants which have been modified by mutagenesis or genetic engineering to provide a new trait to a plant or to modify an already present trait.

Mutagenesis includes techniques of random mutagenesis using X-rays or mutagenic chemicals, but also techniques of targeted mutagenesis, to create mutations at a specific locus of a plant genome. Targeted mutagenesis techniques frequently use oligonucleotides or proteins like CRISPR/Cas, zinc-finger nucleases, TALENs or meganucleases to achieve the targeting effect.

Genetic engineering usually uses recombinant DNA techniques to create modifications in a plant genome which under natural circumstances cannot readily be obtained by cross breeding, mutagenesis or natural recombination. Typically, one or more genes are integrated into the genome of a plant to add a trait or improve a trait. These integrated genes are also referred to as transgenes in the art, while plant comprising such transgenes are referred to as transgenic plants. The process of plant transformation usually produces several transformation events, wich differ in the genomic locus in which a transgene has been integrated. Plants comprising a specific transgene on a specific genomic locus are usually described as comprising a specific "event", which is referred to by a specific event name. Traits which have been introduced in plants or have been modified include in particular herbicide tolerance, insect resistance, increased yield and tolerance to abiotic conditions, like drought.

Herbicide tolerance has been created by using mutagenesis as well as using genetic engineering. Plants which have been rendered tolerant to acetolactate synthase (ALS) inhibitor herbicides by conventional methods of mutagenesis and breeding comprise plant varieties commercially available under the name Clearfield^{®}. However, most of the herbicide tolerance traits have been created via the use of transgenes.

Herbicide tolerance has been created to glyphosate, glufosinate, 2,4-D, dicamba, oxynil herbicides, like bromoxynil and ioxynil, sulfonylurea herbicides, ALS inhibitor herbicides and 4-hydroxyphenylpyruvate dioxygenase (HPPD) inhibitors, like isoxaflutole and mesotrione.

Transgenes wich have been used to provide herbicide tolerance traits comprise: for tolerance to glyphosate: cp4 epsps, epsps grg23ace5, mepsps, 2mepsps, gat4601, gat4621 and goxv247, for tolerance to glufosinate: pat and bar, for tolerance to 2,4-D: aad-1 and aad-12, for tolerance to dicamba: dmo, for tolerance to oxynil herbicies: bxn, for tolerance to sulfonylurea herbicides: zm-hra, csr1-2, gm-hra, S4-HrA, for tolerance to ALS inhibitor herbicides: csr1-2, for tolerance to HPPD inhibitor herbicides: hppdPF, W336 and avhppd-03.

Transgenic corn events comprising herbicide tolerance genes are for example, but not excluding others, DAS40278, MON801, MON802, MON809, MON810, MON832, MON87411, MON87419, MON87427, MON88017, MON89034, NK603, GA21, MZHG0JG, HCEM485, VCO-Ø1981-5, 676, 678, 680, 33121, 4114, 59122, 98140, Bt10, Bt176, CBH-351, DBT418, DLL25, MS3, MS6, MZIR098, T25, TC1507 and TC6275.

Transgenic soybean events comprising herbicide tolerance genes are for example, but not excluding others, GTS 40-3-2, MON87705, MON87708, MON87712, MON87769, MON89788, A2704-12, A2704-21, A5547-127, A5547-35, DP356043, DAS44406-6, DAS68416-4, DAS-81419-2, GU262, SYHTØH2, W62, W98, FG72 and CV127. Transgenic cotton events comprising herbicide tolerance genes are for example, but not excluding others, 19-51a, 31707, 42317, 81910, 281-24-236, 3006-210-23, BXN10211, BXN10215, BXN10222, BXN10224, MON1445, MON1698, MON88701, MON88913, GHB119, GHB614, LLCotton25, T303-3 and T304-40.

Transgenic canola events comprising herbicide tolerance genes are for example, but not excluding others, MON88302, HCR-1, HCN10, HCN28, HCN92, MS1, MS8, PHY14, PHY23, PHY35, PHY36, RF1, RF2 and RF3. Insect resistance has mainly been created by transferring bacterial genes for insecticidal proteins to plants. Transgenes which have most frequently been used are toxin genes of *Bacillus spec.* and synthetic variants thereof, like cry1A, cry1Ab, cry1Ab-Ac, cry1Ac, cry1A.105, cry 1F, cry 1 Fa2, cry2Ab2, cry2Ae, mcry3A, ecry3.1Ab, cry3Bb1, cry34Ab1, cry35Ab1, cry9C, vip3A(a), vip3Aa20. However, also genes of plant origin have been transferred to other plants. In particular genes coding for protease inhibitors, like CpTI and pinll. A further approach uses transgenes to produce double stranded RNA in plants to target and downregulate insect genes. An example for such a transgene is dvsnf7.

Transgenic corn events comprising genes for insecticidal proteins or double stranded RNA are for example, but not excluding others, Bt10, Bt11, Bt176, MON801, MON802, MON809, MON810, MON863, MON87411, MON88017, MON89034, 33121, 4114, 5307, 59122, TC1507, TC6275, CBH-351, MIR162, DBT418 and MZIR098.

Transgenic soybean events comprising genes for insecticidal proteins are for example, but not excluding others, MON87701, MON87751 and DAS-81419.

Transgenic cotton events comprising genes for insecticidal proteins are for example, but not excluding others, SGK321, MON531, MON757, MON1076, MON15985, 31707, 31803, 31807, 31808, 42317, BNLA-601, Event1, COT67B, COT102, T303-3, T304-40, GFM Cry1A, GK12, MLS 9124, 281-24-236, 3006-210-23, GHB119 and SGK321.

Increased yield has been created by increasing ear biomass using the transgene athb17, being present in corn event MON87403, or by enhancing photosynthesis using the transgene bbx32, being present in the soybean event MON87712.

Crops comprising a modified oil content have been created by using the transgenes: gm-fad2-1, Pj.D6D, Nc.Fad3, fad2-1A and fatb1-A. Soybean events comprising at least one of these genes are: 260-05, MON87705 and MON87769.

Tolerance to abiotic conditions, in particular to tolerance to drought, has been created by using the transgene cspB, comprised by the corn event MON87460 and by using the transgene Hahb-4, comprised by soybean event IND-QlQl41 Ql-5.

Traits are frequently combined by combining genes in a transformation event or by combining different events during the breeding process. Preferred combinations of traits are herbicide tolerance to different groups of herbicides, insect tolerance to different kind of insects, in particular tolerance to lepidopteran and coleopteran insects, herbicide tolerance with one or several types of insect resistance, herbicide tolerance with increased yield as well as a combination of herbicide tolerance and tolerance to abiotic conditions.

Plants comprising singular or stacked traits as well as the genes and events providing these traits are well known in the art. For example, detailed information as to the mutagenized or integrated genes and the respective events are available from websites of the organizations "International Service for the Acquisition of Agri-biotech Applications (ISAAA)" (http://www.isaaa.org/gmapprovaldatabase) and the "Center for Environmental Risk Assessment (CERA)" (http://cera-qmc.orq/GMCropDatabase), as well as in patent applications, like EP3028573 and WO2017/011288. The use of compositions according to the invention on crops may result in effects which are specific to a crop comprising a certain gene or event. These effects might involve changes in growth behavior or changed resistance to biotic or abiotic stress factors. Such effects may in particular comprise enhanced yield, enhanced resistance or tolerance to insects, nematodes, fungal, bacterial, mycoplasma, viral or viroid pathogens as well as early vigour, early or delayed ripening, cold or heat tolerance as well as changed amino acid or fatty acid spectrum or content. Furthermore, plants are also covered that contain using recombinant DNA techniques a modified amount of ingredients or new ingredients, specifically to improve raw material production, e.g., potatoes that produce increased amounts of amylopectin (e.g. Amflora^{®} potato, BASF SE, Germany).

Furthermore, it has been found that the the compositions according to the invention are also suitable for the defoliation and/or desiccation of plant parts, for which crop plants such as cotton, potato, oilseed rape, sunflower, soybean or field beans, in particular cotton, are suitable. In this regard compositions have been found for the desiccation and/or defoliation of plants, processes for preparing these compositions, and methods for desiccating and/or defoliating plants using the compositions according to the invention.

As desiccants, the compositions according to the invention are suitable in particular for desiccating the above-ground parts of crop plants such as potato, oilseed rape, sunflower and soybean, but also cereals. This makes possible the fully mechanical harvesting of these important crop plants.

Also of economic interest is the facilitation of harvesting, which is made possible by concentrating within a certain period of time the dehiscence, or reduction of adhesion to the tree, in citrus fruit, olives and other species and varieties of pomaceous fruit, stone fruit and nuts. The same mechanism, i.e. the promotion of the development of abscission tissue between fruit part or leaf part and shoot part of the plants is also essential for the controlled defoliation of useful plants, in particular cotton.

Moreover, a shortening of the time interval in which the individual cotton plants mature leads to an increased fiber quality after harvesting.

The compounds of formula (I) can be prepared according to methods or in analogy to methods that are described in the prior art and are very well known and apply common amid and peptide coupling technics. The synthesis takes advantage of starting materials that are commercially available or may be prepared according to conventional procedures starting from readily available compounds.

The compounds of formula (I) according to the invention can be prepared by standard processes of organic chemistry, for example by the following processes:

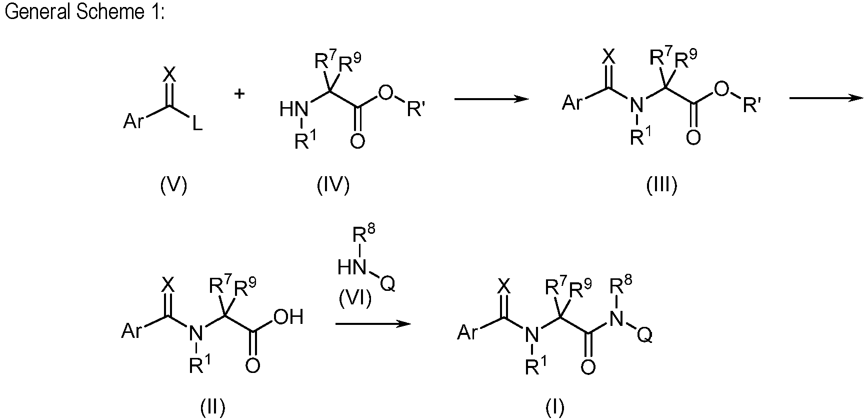

Compounds of the formula (I) can, for example, be prepared according to General Scheme 1 from the compounds of formula (V) (when X = O, L = OH, Cl or F; when X = S, L = CI or F) and commercially available amino acids and esters (IV) to form hetaroylamino esters (III). Such coupling reactions using acid chlorides and a base are for example described in analogy for benzoyl derivatives in J. Heterocyclic Chem., 41, 57 (2004*)* or in Can. J. Chem. 1987, 65, 1224-1227 with common coupling reagents such as EDC/HOBt, which is also described in WO 2010/084979, or such coupling can be achieved by using other coupling reagents, for example HATU (O-(7-azabenzotriazole-1-yl)-N,N,N',N'-tetramethyluronium-hexafluorphosphate), and an organic base.

To obtain the compounds of formula (I) the intermediates (III) can for example be converted to amino acids (II), e.g. by applying common saponification techniques such as treatment with a base, followed by a second coupling step with amines (VI) using similar methods as described above and which are common to a person skilled in the art.

Compounds of formula (I) might also, for example, be prepared according to General Scheme 2 from the compounds of formula (V) (when X = O, L = OH, Cl or F; when X = S, L = Cl or F) and appropriate amino precursors (VII) using similar coupling reactions as described above, and as exemplified below in the experimental part.

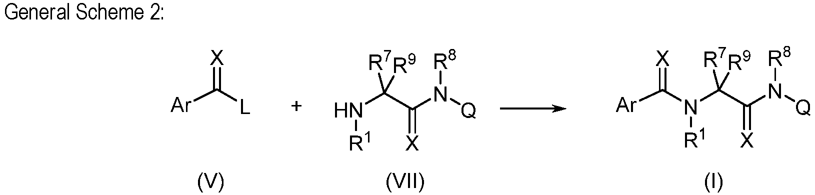

Alternatively, compounds of formula (I) might also be prepared according to General Scheme 3 by modification of compounds of formula (I), for example using an additional saponification/coupling step (Y = CO₂R^{e}), as exemplified below in the experimental part below.

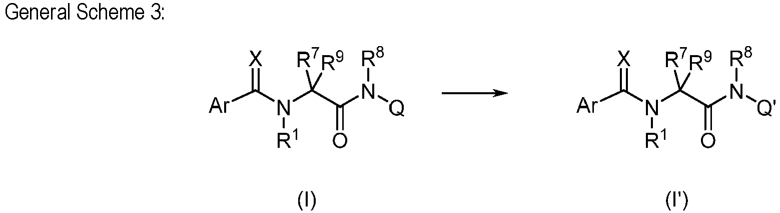

A thioamide of formula (I) (X = S) can be prepared from the corresponding amide of formula (I) (X = O), according to General Scheme 4 using a thionation reagent. Preferably elemental sulfur, phosphorus pentasulfide, ammonium phosphorodithioate or Lawesson's reagent (CAS: 19172-47-5) are employed. Most preferably, Lawesson's reagent is employed. The reaction is typically carried out in an organic solvent. Preferably an aprotic organic solvent is used. Most preferably tetrahydrofuran, 1,4-dioxane and toluene are used. The reaction is carried out at temperatures between room temperature to refluxing temperatures. Preferably, the reaction is carried out at refluxing temperatures.

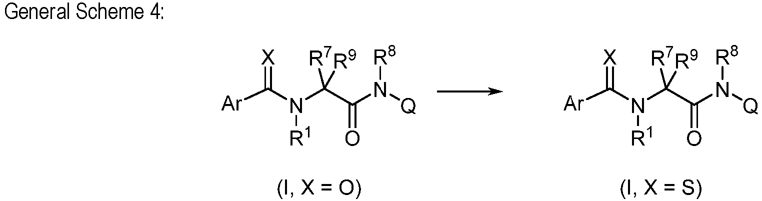

### A Synthesis Examples

### Example 1: Methyl (1S,4R)-4-[[(2R)-2-[(5-chloropyridine-3-carbonyl)amino]propanoyl]amino]cyclopent-2-ene-1-carboxylate (Ex-7)

### Step 1:

### Methyl (1S,4R)-4-[[(2R)-2-(tert-butoxycarbonylamino)propanoyl]amino] cyclopent-2-ene-1-carboxylate

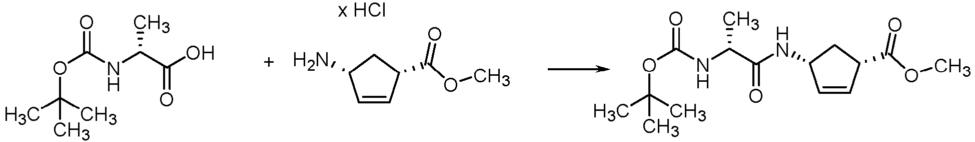

To a mixture of (2*R*)-2-(*tert*-butoxycarbonylamino)propanoic acid (30.0 g,159 mmol), methyl (1*S*,4*R*)-4-aminocyclopent-2-ene-1-carboxylate hydrochloride (29.6, 166 mmol) and N,N-diisopropylethylamine (61.5 g, 476 mmol) in 200 ml dichloromethane [dimethylamino(triazolo[4,5-b]pyridin-3-yloxy)methylene]-dimethyl-ammonium hexafluorophosphate (HATU, 63.3 g, 166 mmol) was added at room temperature. The initially formed suspension was heated to reflux and became clear after a couple of minutes and stirring was continued for 2 hours at the same temperature. After cooling down to room temperature the organic phase was washed with water (3 x 500 ml) and then concentrated *in vacuo* to yield a crude product which was purified by column chromatography to give the title compound in 90% yield. 1H NMR: (400MHz, DMSO-d6) δ 7.8 (d, 1H), 6.8 (d, 1H) 5.9 (m, 1H), 5.7 (m, 1H), 4.8 (m, 1H), 3.95 (m, 1H), 3.65 (s, 3H), 3.55 (m, 1H), 2.5 (m, 1H), 1.75 (m, 1 H), 1.35 (s, 9H), 1.15 (d, 3H).

### Step 2:

### Methyl (1S,4R)-4-[[(2R)-2-aminopropanoyl]amino]cyclopent-2-ene-1-carboxylate

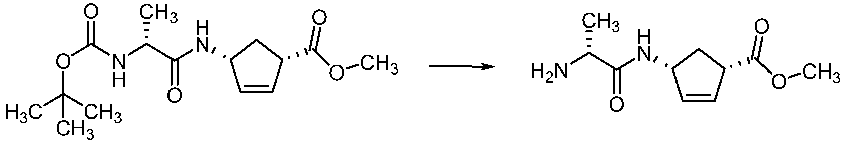

To a solution of methyl (1*S*,4*R*)-4-[[(2*R*)-2-(*tert*-butoxycarbonylamino)propanoyl] amino]cyclopent-2-ene-1-carboxylate (90.2 g, 289 mmol) in dichloromethane (250 ml) 2,2,2-trifluoroacetic acid (230 g, 2 mol) was added at room temperature. The reaction mixture was heated under reflux for 1 day. The reaction mixture was carefully quenched with aqueous sodium hydroxide solution (4 mol/l, 400 ml) and adjusted to pH 8.5 and extracted with dichloromethane (6 x 200 ml). The combined organic phases were dried *in vacuo* to yield the title compound (37.4 g, 61%). (400MHz, DMSO-d6) δ 7.85 (d, 1H), 5.9 (m, 1H), 5.75 (m, 1H), 4.8 (m, 1H), 3.65 (s, 3H), 3.55 (m, 1H), 3.2 (m. 1H), 2.45 (m, 1H), 1.8 (br s, 2 H), 1.7 (m, 1H), 1.1 (d, 3H).

### Step 3: Methyl (1S,4R)-4-[[(2R)-2-[(5-chloropyridine-3-carbonyl)amino]propanoyl]amino]cyclopent-2-ene-1-carboxylate (Ex-7)

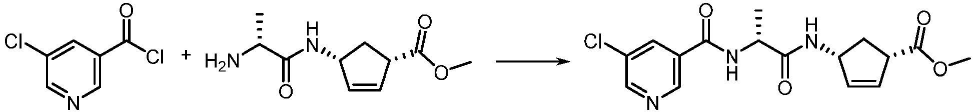

To a solution of methyl (1*S*,4*R*)-4-[[(2*R*)-2-aminopropanoyl]amino]cyclopent-2-ene-1-carboxylate
(316 mg) and N-ethyl-N-isopropyl-propan-2-amine (Hünig base, 385 mg) in dichloromethane (20 ml) a solution of 5-chloropyridine-3-carbonyl chloride (275 mg) in dichloromethane (5 ml) was added at room temperature and stirring continued for 2 hours. Then the reaction mixture was treated with an excess of aqueous hydrochloric acid solution (1 mol/l) and washed with water (2x) and an aqueous saturated solution of sodium chloride (1x). The organic phase was concentrated in vacuo. The crude product was purified by chromatography to yield the title compound (250 mg). 1H NMR: (400MHz, CDCl₃) δ 8.9 (s, 1H), 8.7 (s, 1H), 8.15 (s, 1H), 7.2 (d, 1H), 6.7 (d, 1H), 5.9 (m, 2H), 5.1 (m, 1H), 4.65 (m, 1H), 3.7 (s, 3H), 3.5 (m, 1H), 2.45 (m, 1H), 1.8 (m, 1 H), 1.5 (d, 3H)

### Example 2: Isopropyl (1S,4R)-4-[[(2S)-2-[(5-fluoropyridine-3-carbonyl)amino]propanoyl]amino]cyclopent-2-ene-1-carboxylate (Ex-3)

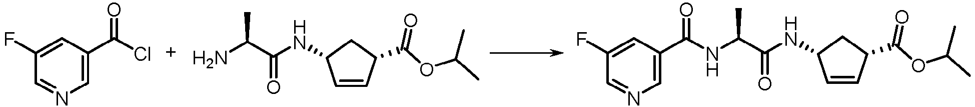

To a solution of isopropyl (1*S*,4*R*)-4-[[(2*S*)-2-aminopropanoyl]amino]cyclopent-2-ene-1-carboxylate (276 mg, prepared in analogy to the procedure described above for the synthesis of Ex-7 (Example 1), starting from commercially available isopropy (1*S*,4*R*)-4-aminocyclopent-2-ene-1-carboxylate hydrochloride) and N-ethyl-N-isopropyl-propan-2-amine (Hünig base, 426 mg) in dichloromethane (20 ml) a solution of 5-fluoropyridine-3-carbonyl chloride (276) in dichloromethane (5 ml) was added at room temperature and stirring continued for 2 hours. Afterwards the reaction mixture was treated with an excess of aqueous hydrochloric acid solution (1 mol/l) and washed with water (2x) and an aqueous saturated solution of sodium chloride (1x). The organic phase was concentrated *in vacuo.* The crude product was purified by chromatography to yield the title compound (250 mg). 1H NMR: (400MHz, CDCl₃) δ 8.85 (s, 1H), 8.6 (s, 1H), 7.9 (m, 1H), 7.35 (d, 1H), 6.8 (d, 1H), 5.9 (m, 2H), 5.05 (m, 1H), 4.95 (m, 1H), 4.65 (m, 1H), 3.45 (m, 1H), 2.4 (m, 1H), 1.9 (m, 1 H), 1.5 (d, 3H), 1.25 (d, 6H).

### Example 3: (1S,4R)-4-[[(2R)-2-[(5-chlorothiophene-2-carbonyl)amino]propanoyl]amino]cyclopent-2-ene-1-carboxylic acid (Ex-28)

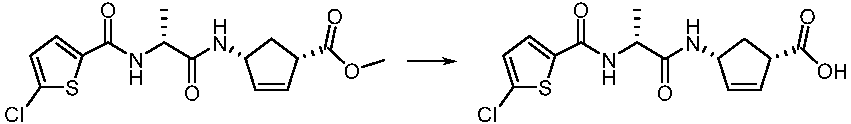

To a solution of methyl (1S,4R)-4-[[(2R)-2-[(5-chlorothiophene-2-carbonyl)amino]propanoyl]amino]cyclopent-2-ene-1-carboxylate (470 mg) in 20 ml dichloromethane hydroxy(trimethyl)stannane was added (595 mg) and the reaction stirred at reflux for 3 d. Aqueous hydrochloric acid solution (1 mol/l, 30 ml) was added. The resulting precipitate was separated by filtration and washed with dichloromethane and plenty of water. The remainder was dried at 45°C in vacuo overnight to yield the title compound (200 mg). 1H NMR: (400MHz, DMSO-d6) δ ca. 12.4 (br s, 1H), 8.6 (d, 1H), 8.1 (d, 1H) 7.9 (s, 1H), 7.8 (s, 1H), 5.85 (m, 1H), 5.7 (m, 1H), 4.75 (m, 1H), 4.4 (m, 1H), 3.45 (m, 1H), 2.5 (m, 1H), 1.75 (m, 1 H), 1.3 (d, 3H).

In analogy to the examples described above, the following compounds Ex-1 to Ex-45 in Table I, which are of formula (I.Ex), wherein some of the variable defined for compounds of formula (I) are specified, *i*.*e*. R¹, R⁸ and R⁹ are hydrogen, R⁷ is methyl, were prepared using commercially available amines. The absolute configuration (R or S) of the chiral carbon atom labeled with "$" is provided in column $.

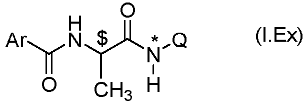

**Table I: MZ: is LC-MS-MZ, (MZ: mass charge ratio); # indicates the bond to the adjacent group -C(=O)NH-.**

| **Cpd.** | **Ar** | **$** | **N*-Q** | **LCMS MZ*** |
|---|---|---|---|---|
| Ex-1 | 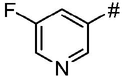 | R | 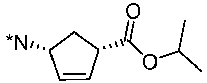 | 364 |
| Ex-2 | 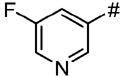 | S | 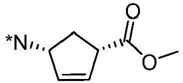 | 335,9 |
| Ex-3 | 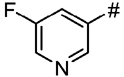 | S | 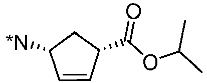 | 364 |
| Ex-4 | 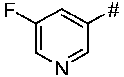 | R | 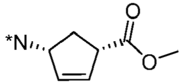 | 336 |
| Ex-5 | 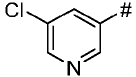 | R | 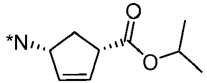 | 9,654 |
| Ex-6 | 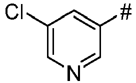 | S | 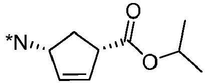 | 379,9 |
| Ex-7 | 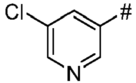 | R | 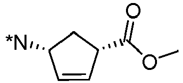 | 351,9 |
| Ex-8 | 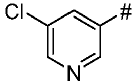 | S | 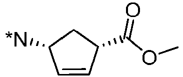 | 352 |
| Ex-9 | 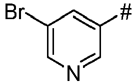 | R | 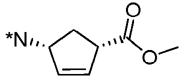 | 397,8 |
| Ex-10 | 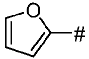 | R | 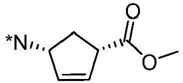 | 306,8 |
| Ex-11 | 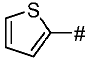 | R | 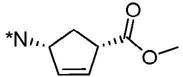 | 322,8 |
| Ex-12 | 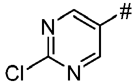 | R | 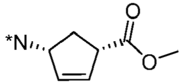 | 352,9 |
| Ex-13 | 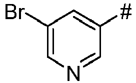 | R | 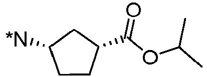 | 423,9 |
| Ex-14 | 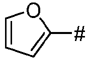 | R | 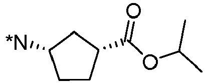 | 335,1 |
| Ex-15 | 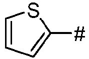 | R | 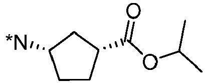 | 351,1 |
| Ex-16 | 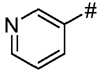 | R | 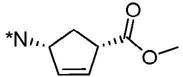 | 318 |
| Ex-17 | 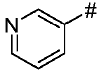 | R | 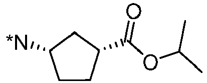 | 346 |
| Ex-18 | 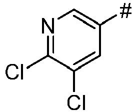 | R | 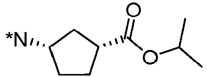 | 413,9 |
| Ex-19 | 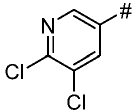 | R | 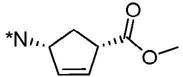 | 385,9 |
| Ex-20 | 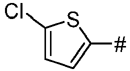 | R | 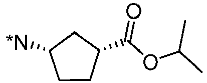 | 384,9 |
| Ex-21 | 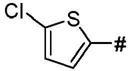 | R | 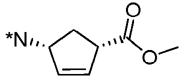 | 356,9 |
| Ex-22 | 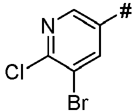 | R | 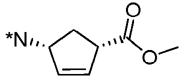 | 431,8 |
| Ex-23 | 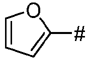 | R | 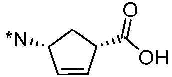 | 293 |
| Ex-24 | 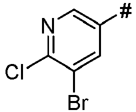 | R | 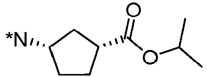 | 459,8 |
| Ex-25 | 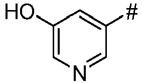 | R | 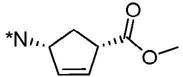 | 334 |
| Ex-26 | 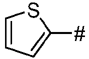 | R | 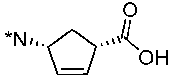 | 308,9 |
| Ex-27 | 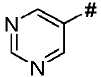 | R | 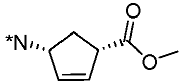 | 319 |
| Ex-28 | 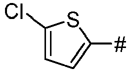 | R | 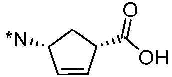 | 342,9 |
| Ex-29 | 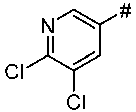 | R | 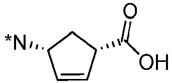 | 371,9 |
| Ex-30 | 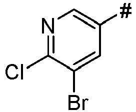 | R | 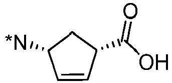 | 417,8 |
| Ex-31 | 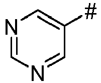 | R | 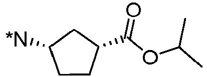 | 347 |
| Ex-32 | 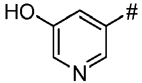 | R | 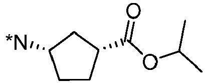 | 362 |
| Ex-33 | 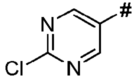 | R | 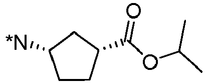 | 380,9 |
| Ex-34 | 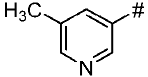 | R | 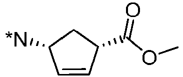 | 332 |
| Ex-35 | 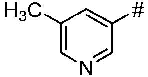 | R | 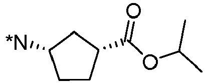 | 360,3 |
| Ex-36 | 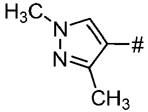 | R | 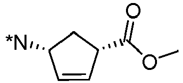 | 335 |
| Ex-37 | 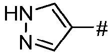 | R | 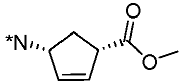 | 306,9 |
| Ex-38 | 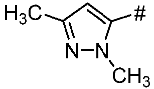 | R | 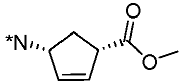 | 335 |
| Ex-39 | 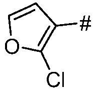 | R | 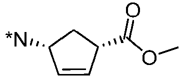 | 340,9 |
| Ex-40 | 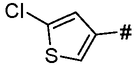 | R | 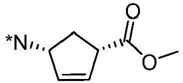 | 356,9 |
| Ex-41 | 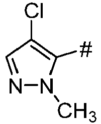 | R | 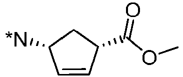 | 355 |
| Ex-42 | 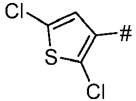 | R | 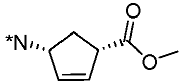 | 390,9 |
| Ex-43 | 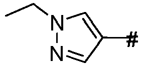 | R | 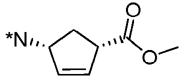 | 335 |
| Ex-44 | 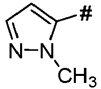 | R | 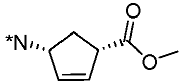 | 321 |
| Ex-45 | 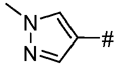 | R | 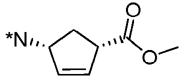 | 321 |

| | | | | |
|---|---|---|---|---|
| * LC-MS Method: Shimadzu Nexera LC-30 LCMS-2020; HPLC-column Kinetex XB C18 1,7µ (50 x 2,1 mm); eluent: acetonitrile / water + 0.1% trifluoroacetic acid (gradient acetonitrile:water from 5:95 to pure acetonitrile in 1.5 minutes at 60 °C, flow gradient from 0.8 to 1.0 ml/min in 1.5 minutes). | | | | |

### B Biological examples

The herbicidal activity of the compounds of formula (I) was demonstrated by the following greenhouse experiments:
The culture containers used were plastic flowerpots containing loamy sand with approximately 3.0% of humus as the substrate. The seeds of the test plants were sown separately for each species.

For the pre-emergence treatment, the active ingredients, which had been suspended or emulsified in water, were applied directly after sowing by means of finely distributing nozzles. The containers were irrigated gently to promote germination and growth and subsequently covered with transparent plastic hoods until the test plants had rooted. This cover caused uniform germination of the test plants, unless this had been impaired by the active ingredients. For the post-emergence treatment, the test plants were first grown to a height of 3 to 15 cm, depending on the plant habit, and only then treated with the active ingredients which had been suspended or emulsified in water. For this purpose, the test plants were either sown directly and grown in the same containers, or they were first grown separately as seedlings and transplanted into the test containers a few days prior to treatment.

Depending on the species, the test plants were kept at 10-25 °C or 20-35 °C, respectively.

The test period extended over 2 to 4 weeks. During this time, the test plants were tended, and their response to the individual treatments was evaluated.

Evaluation was carried out using a scale from 0 to 100. 100 means no emergence of the test plants, or complete destruction of at least the aerial moieties, and 0 means no damage, or normal course of growth. A moderate herbicidal activity is given at values of 50 to 70, good herbicidal activity is given at values of 70 to < 90 and a very good herbicidal activity is given at values of 90 to 100.

The test plants used in the greenhouse experiments were of the following species:

| Bayer code | Scientific name |
|---|---|
| ALOMY | *Alopercurus myosuroides* |
| AMARE | *Amaranthus retroflexus* |
| AVEFA | *Avena fatua* |
| DIGSA | *Digitaria sanguinalis* |
| ECHCG | *Echinocloa crus-galli* |
| LOLMU | *Lolium multiflorum* |
| SETFA | *Setaria faberi* |

At an application rate of 0.125 kg/ha, applied by the post-emergence method:
- compound Ex-5 showed very good herbicidal activity against AMARE
- compounds Ex-5, Ex-20 showed very good herbicidal activity against ALOMY
- compound Ex-42 showed moderate herbicidal activity against ALOMY
- compound Ex-42 showed moderate herbicidal activity against ECHCG
- compounds Ex-5, Ex-20 showed very good herbicidal activity against AVEFA
- compound Ex-42 showed good herbicidal activity against AVEFA

At an application rate of 0.250 kg/ha, applied by the pre-emergence method:
- compounds Ex-28, Ex-40 showed very good herbicidal activity against LOLMU
- compound Ex-7 showed good herbicidal activity against LOLMU
- compounds Ex-7, Ex-28 showed good herbicidal activity against DIGSA
- compound Ex-40 showed moderate good herbicidal activity against DIGSA
- compound Ex-40 showed good herbicidal activity against SETFA

At an application rate of 0.250 kg/ha, applied by the post-emergence method:
- compounds Ex-4, Ex-7, Ex-9, Ex-13, Ex-21, Ex-28, Ex-35 showed very good herbicidal activity against AMARE
- compound Ex-34 showed good herbicidal activity against AMARE
- compounds Ex-7, Ex-9, Ex-13, Ex-28 showed very good herbicidal activity against ECHCG
- compounds Ex-4, Ex-11, Ex-26, Ex-34, Ex-35, Ex-40 showed good herbicidal activity against ECHCG
- compounds Ex-9, Ex-11, Ex-13, Ex-21, Ex-26 showed very good herbicidal activity against AVEFA
- compounds Ex-7, Ex-40 showed good herbicidal activity against AVEFA
- compound Ex-4 showed moderate herbicidal activity against AVEFA
- compound Ex-21 showed very good herbicidal activity against ALOMY
- compounds Ex-11, Ex-28, Ex-35, Ex-40 showed good herbicidal activity against ALOMY

## Claims

1. Compounds of formula (I) 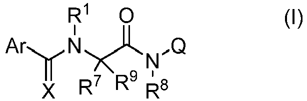 wherein the substituents have the following meanings:
Ar is a 5- or 6-membered heteroaromatic ring; or a bicyclic or tricyclic saturated, partially unsaturated, partially aromatic condensed, or aromatic ring system; wherein the 5- or 6-membered heteroaromatic ring contains 1, 2, 3, or 4 heteroatoms selected from the group consisting of N, O, and S as ring members; wherein the bicyclic or tricyclic ring system contains 0, 1, 2, 3, or 4 heteroatoms selected from the group consisting of N, O, and S as ring members; and with the provision that the 5- or 6-membered heteroaromatic ring cannot contain 2 or more atoms selected from O and S, and the bicyclic or tricyclic ring system cannot contain 2 contiguous atoms selected from O and S; and wherein the heteroaromatic ring, or the bicyclic, or tricyclic ring systems are substituted with k substituents R^{A1} and q substituents R^{A2};
R^{A1} independently of each other are halogen, nitro, hydroxyl, oxo, cyano, (C₁-C₃)-alkyl, (C₁-C₃)-haloalkyl, hydroxy-(C₁-C₃)-alkyl, (C₃-C₅)-cycloalkyl, (C₁-C₃)-alkoxy, (C₁-C₃)-haloalkoxy, (C₂-C₃)-alkenyl, (C₂-C₃)-haloalkenyl, (C₂-C₃)-alkynyl, or (C₂-C₃)-haloalkynyl; and whereas the sulfur ring atoms bear 0, 1, or 2 oxo groups;
R^{A2} independently of each other are phenyl-(C₁-C₃)-alkyl, (C₁-C₄)-alkylcarbonyl, aminocarbonyl, (C₁-C₄)-alkylaminocarbonyl, di-(C₁-C₄-alkyl)aminocarbonyl, (C₁-C₄)-alkoxycarbonyl, benzyloxycarbonyl, fluorenyloxycarbonyl, allyloxycarbonyl, (C₁-C₃)-alkoxy-(C₁-C₃)-alkyl, (C₁-C₃)-alkylthio, (C₁-C₃)-alkylsulfinyl, (C₁-C₃)-alkylsulfonyl, or phenylsulfonyl, where the aliphatic or aromatic moieties in the 14 last-mentioned radicals are unsubstituted or substituted by m radicals selected from the group consisting of fluorine, chlorine, bromine, cyano and (C₁-C₂)-alkoxy;
k is 0, 1, 2, 3, 4, or 5;
q is 0, 1, 2, 3, 4, or 5;
with the provision that the total number of substituents R^{A1} and R^{A2} on each ring or ring system does not exceed the number of substituents that are theoretically possible; and excluding heteroaromatic rings that bear an electronic charge;
R¹, R⁸ independently of each other are hydrogen, (C₁-C₃)-alkyl, (C₁-C₃)-haloalkyl, (C₃-C₄)-cycloalkyl, (C₂-C₃)-alkenyl, (C₂-C₃)-haloalkenyl, (C₃-C₄)-alkynyl, (C₃-C₄)-haloalkynyl, (C₁-C₃)-alkoxy-(C₁-C₃)-alkyl, aryl, aryl-(C₁-C₃)-alkyl, (C₁-C₃)-alkoxy, (C₁-C₃)-haloalkoxy, (C₁-C₃)-alkoxy-(C₁-C₃)-alkoxy, (C₁-C₃)-alkylcarbonyl, arylcarbonyl, (C₁-C₃)-alkoxycarbonyl, or aryloxycarbonyl;
R⁷ is methyl or ethyl;
R⁹ is hydrogen or methyl; or
R⁷ and R⁹ together with the carbon atom to which they are attached form a 3- or 4-membered, saturated carbocyclic ring;
X is oxygen or sulfur;
Q represents the radical of formula (Z-Y), 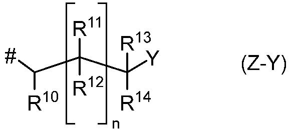
wherein # indicates the bond to the adjacent nitrogen atom, and wherein the substituents have the following meanings
R¹⁰ is (C₁-C₄)-alkyl;
R¹¹, R¹², R¹³, R¹⁴ independently of each other are hydrogen or (C₁-C₄)-alkyl; or
R¹⁰ and R¹⁴ together with the carbon atoms to which they are bound form a three- to six-membered saturated or partially unsaturated carbocycle or a three- to six-membered saturated or partially unsaturated heterocycle containing 1 or 2 oxygen, nitrogen or sulfur atoms as ring members; with the provision that the heterocycle cannot contain 2 contiguous atoms selected from O and S;
Y is CO₂R^{e}, CONR^{b}R^{h}, CON(R^{x})-OR^{y}, C(=N-OR^{y})-OR^{x}, CONR^{e}S(=O)R^{a}, CONR^{e}SO₂R^{a}, or CONR^{b1}SO₂NR^{b2}R^{b3};
R^{a} is each independently (C₁-C₆)-alkyl, (C₁-C₆)-haloalkyl, (C₃-C₄)-alkynyl, or (C₃-C₆)-cycloalkyl, each of which is unsubstituted or substituted with m radicals selected from the group consisting of fluorine, chlorine, bromine, iodine, cyano, hydroxyl, and (C₁-C₃)-alkoxy;
R^{b} is hydrogen, (C₁-C₆)-alkyl, (C₂-C₄)-alkenyl, (C₃-C₄)-alkynyl, (C₃-C₆)-cycloalkyl, (C₃-C₆)-cycloalkyl-(C₁-C₃)-alkyl, phenyl-(C₁-C₃)-alkyl, or furanyl-(C₁-C₃)-alkyl, where each of the seven last-mentioned radicals is unsubstituted or substituted with m radicals selected from the group consisting of fluorine, chlorine, bromine, cyano, CO₂R^{a}, (C₁-C₂)-alkoxy, (C₁-C₃)-alkylthio, (C₁-C₃)-alkylsulfinyl, (C₁-C₃)-alkylsulfonyl, phenylthio, phenylsulfinyl, and phenylsulfonyl;
R^{b1} and R^{b2} independently of each other and independently of each occurrence, are hydrogen or have independently of each other one of the meanings given for R^{a};
R^{b3} is hydrogen, (C₁-C₆)-alkyl, (C₂-C₄)-alkenyl, (C₃-C₄)-alkynyl, (C₃-C₆)-cycloalkyl, (C₃-C₆)-cycloalkyl-(C₁-C₃)-alkyl, phenyl-(C₁-C₃)-alkyl, or furanyl-(C₁-C₃)-alkyl, where each of the seven last-mentioned radicals is unsubstituted or substituted with m radicals selected from the group consisting of fluorine, chlorine, bromine, cyano, CO₂R^{a}, , (C₁-C₂)-alkoxy, (C₁-C₃)-alkylthio, (C₁-C₃)-alkylsulfinyl, (C₁-C₃)-alkylsulfonyl, phenylthio, phenylsulfinyl, and phenylsulfonyl; or
R^{b2} and R^{b3} form, together with the nitrogen atom to which they are bound, a saturated 3-, 4-, 5-, 6-, or 7-membered heterocycle which may contain one further heteroatom or heteroatom group selected from the group consisting of N, O, S, S(=O) and S(=O)₂ as ring member;
R^{e} is hydrogen, (C₁-C₆)-alkyl, (C₂-C₄)-alkenyl, (C₃-C₄)-alkynyl, (C₃-C₆)-cycloalkyl, (C₃-C₆)-cycloalkyl-(C₁-C₃)-alkyl, phenyl-(C₁-C₃)-alkyl, or furanyl-(C₁-C₃)-alkyl, where each of the seven last-mentioned radicals is unsubstituted or substituted with m radicals selected from the group consisting of fluorine, chlorine, bromine, cyano, CO₂R^{a}, (C₁-C₂)-alkoxy, (C₁-C₂)-haloalkoxy, (C₁-C₃)-alkylthio, (C₁-C₃)-haloalkylthio, (C₁-C₃)-alkylsulfinyl, (C₁-C₃)-haloalkylsulfinyl, (C₁-C₃)-alkylsulfonyl, (C₁-C₃)-haloalkylsulfonyl, phenylthio, phenylsulfinyl, and phenylsulfonyl;
R^{h} is hydrogen, hydroxyl, (C₁-C₆)-alkyl, (C₁-C₂)-alkoxy, (C₃-C₆)-cycloalkyl, (C₂-C₄)-alkenyl, (C₁-C₆)-alkoxycarbonyl-(C₁-C₆)-alkyl, or (C₃-C₄)-alkynyl, where each of the six last-mentioned radicals is unsubstituted or substituted with m radicals selected from the group consisting of fluorine, chlorine, bromine, cyano, CO₂R^{a}, (C₁-C₂)-alkyl, (C₁-C₂)-alkoxy and (C₁-C₂)-alkoxy-(C₁-C₂)-alkoxy; or
R^{b} and R^{h} form, together with the nitrogen atom to which they are bound, a saturated 3-, 4-, 5-, 6-, or 7-membered heterocycle which may contain one further heteroatom or heteroatom group selected from the group consisting of N, O, S, S(=O), and S(=O)₂ as ring member;
R^{x} and R^{y} together with the atoms to which they are each bound, form a saturated 5- or 6-membered heterocycle;
m is each independently 1, 2, or 3;
n is 0, 1, or 2;
including their agriculturally acceptable salts, stereoisomers and tautomers.

2. The compounds as claimed in claim 1 or 2, wherein the variable Ar in compounds of formula (I) is a 5-membered heteroaromatic ring containing 1, 2 or 3 heteroatoms selected from the group consisting of N, O, and S as ring members; or a 6-membered heteroaromatic ring containing 1 or 2 nitrogen atoms as ring members; with the provision that the 5-membered heteroaromatic ring cannot contain 2 or more atoms selected from O and S; whereas the heteroaromatic ring Ar is substituted with k substituents R^{A1} and q substituents R^{A2}; whereas R^{A1} is each independently halogen, cyano, hydroxyl, oxo, (C₁-C₃)-alkyl, (C₁-C₃)-haloalkyl, (C₁-C₃)-alkoxy, or (C₁-C₃)-haloalkoxy; and R^{A2} is each independently phenyl-(C₁-C₃)-alkyl, (C₁-C₄)-alkylcarbonyl, (C₁-C₄)-alkylaminocarbonyl, di-(C₁-C₄-alkyl)aminocarbonyl, (C₁-C₄)-alkoxycarbonyl, benzyloxycarbonyl, fluorenyloxycarbonyl, allyloxycarbonyl, or (C₁-C₃)-alkoxy-(C₁-C₃)-alkyl; whereas k is 0, 1, 2 , or 3 and q is 0 or 1; with the provision that the total number of substituents R^{A1} and R^{A2} on each heteroaromatic ring does not exceed the number of substituents that are theoretically possible; and excluding heteroaromatic rings that bear an electronic charge.

3. The compounds as claimed in any one of claims 1 to 3, wherein the variable Ar in compounds of formula (I) is a 5-membered heteroaromatic ring containing 1 or 2 heteroatoms selected from the group consisting of N, O, and S as ring members; or a 6-membered heteroaromatic ring containing 1 or 2 nitrogen atoms as ring members; with the provision that the 5-membered heteroaromatic ring cannot contain 2 or more atoms selected from O and S; whereas the heteroaromatic ring Ar is substituted with k substituents R^{A1} and q substituents R^{A2}; whereas R^{A1} is each independently halogen, cyano, hydroxyl, (C₁-C₃)-alkyl, or (C₁-C₃)-haloalkyl; and R^{A2} is each independently (C₁-C₄)-alkoxycarbonyl; whereas k is 0, 1, or 2 and q is 0 or 1; with the provision that the total number of substituents R^{A1} and R^{A2} on each heteroaromatic ring does not exceed the number of substituents that are theoretically possible; and excluding heteroaromatic rings that bear an electronic charge.

4. The compounds as claimed in any one of claims 1 to 3, wherein the variable Ar in compounds of formula (I) is a 6-membered heteroaromatic ring containing 1 or 2 nitrogen atoms as ring members; whereas the heteroaromatic ring Ar is substituted with k substituents R^{A1}; whereas R^{A1} is each independently halogen, hydroxyl, or (C₁-C₃)-alkyl; k is 0, 1, or 2; and excluding heteroaromatic rings that bear an electronic charge.

5. The compounds as claimed in any one of claims 1 to 4, wherein
R¹ is hydrogen;
R⁸ is hydrogen.

6. The compounds as claimed in any one of claims 1 to 5, wherein
R⁷ is methyl;
R⁹ is hydrogen.

7. The compounds as claimed in any one of claims 1 to 6, wherein
R¹⁰ is methyl.

8. The compounds as claimed in any one of claims 1 to 7, wherein
X is oxygen.

9. The compounds as claimed in any one of claims 1 to 8, wherein
Q is Z-Y selected from the group Z¹-Y to Z⁸-Y: 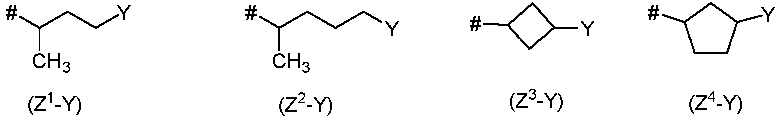 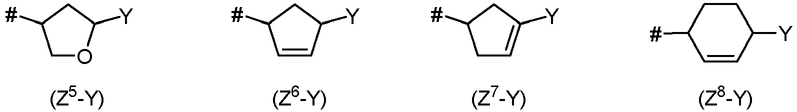
wherein # indicates the bond to the adjacent nitrogen atom, and wherein the substituents have the following meanings, wherein
Y is CO₂R^{e};
R^{e} is hydrogen, (C₁-C₆)-alkyl, or (C₃-C₆)-cycloalkyl, where each of the two last-mentioned radicals is unsubstituted or substituted with m radicals selected from the group consisting of (C₁-C₂)-alkoxy, (C₁-C₃)-alkylthio, (C₁-C₃)-alkylsulfinyl, (C₁-C₃)-alkylsulfonyl, phenylthio, phenylsulfinyl, and phenylsulfonyl;
m is 1 or 2.

10. The compounds as claimed in any one of claims 1 to 8, wherein
Q is Z-Y selected from the group Z¹-Y to Z⁸-Y: 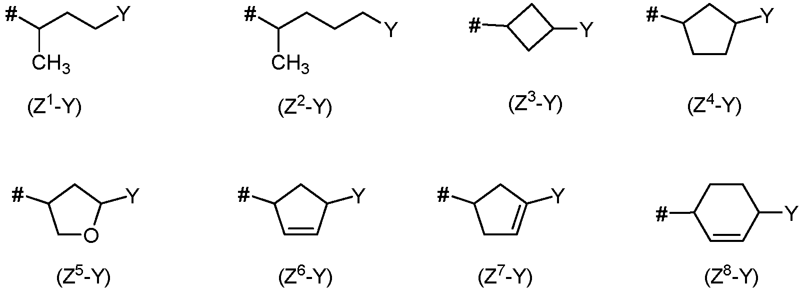
wherein # indicates the bond to the adjacent nitrogen atom, and wherein the substituents have the following meanings, wherein
Y is CO₂R^{e}
R^{e} is hydrogen, (C₁-C₆)-alkyl, or (C₃-C₆)-cycloalkyl.

11. Compounds of formula (I.R), wherein Ar, R¹, R⁷, R⁸, R⁹, X, and Q are defined as in any one of claims 1 to 11, and wherein the stereocenter (§) is in the (R)-configuration

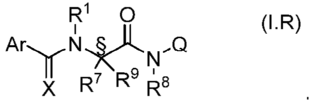

12. An agrochemical formulation comprising a compound of formula (I) as defined in any one of claims 1 to 10, or a compound of formula (I.R) as defined in claim 11, and one or more auxiliaries, which are customary for formulating crop protection compounds.

13. The use of a compound of formula (I) as defined in any one of claims 1 to 10, or a compound of formula (I.R) as defined in claim 11, or an agrochemical formulation as claimed in claim 12, for controlling unwanted vegetation.

14. A method for controlling unwanted vegetation which comprises allowing a herbicidally effective amount of a compound of formula (I) as defined in any one of claims 1 to 10, or a compound of formula (I.R) as defined in claim 11, or an agrochemical formulation as claimed in claim 12, to act on plants, their seed and/or their habitat.
